# EUROPEAN PATENT APPLICATION

(11) **EP 4 428 125 A1**
(43) Date of publication of application: **11.09.2024**
(21) Application number: 22889390.5
(22) Date of filing: 03.11.2022
(51) Int. Cl.: C07D 243/36, A61P 35/00, A61K 31/403

(54) **BICYCLIC COMPOUND AND APPLICATION THEREOF**

(30) Priority: 02.11.2021 WO PCT/CN2021/128239; 16.12.2021 WO PCT/CN2021/138668
(71) Applicant: Betta Pharmaceuticals Co., Ltd, Yuhang Hangzhou Zhejiang 311100 (CN)
(72) Inventor: YANG, Rongwen, Beijing 100176 (CN); SUN, Yun, Beijing 100176 (CN); ZHANG, Jian, Beijing 100176 (CN); YI, Xuegang, Beijing 100176 (CN); MA, Teng, Beijing 100176 (CN); WANG, Yu, Beijing 100176 (CN); WANG, Pingping, Beijing 100176 (CN); DING, Lieming, Hangzhou, Zhejiang 311100 (CN); LAN, Hong, Beijing 100176 (CN); WANG, Jiabing, Beijing 100176 (CN)
(74) Representative: Pfenning, Meinig & Partner mbB
(86) International application number: PCT/CN2022/129643
(87) International publication number: WO 2023/078369

(57) **Abstract**

The present invention relates to a compound as shown in formula (I). The present invention also provides a composition and preparation containing the compound, and a method for using and preparing the compound.

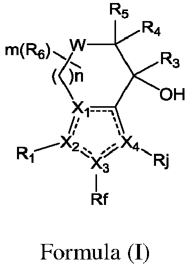

## Description

### Technical Field

The present invention relates to a bicyclic compound, a composition and preparation containing the compound, and a method for using and preparing the compound.

### Background Art

HIFs (hypoxia inducible factors) are members of the transcription factor family and act as a pathway for the body to sense oxygen changes. They are also known as oxygen-deficiency inducible factors and mediate cellular hypoxia responses by controlling more than 40 hypoxia-adaptation genes downstream. They are mainly composed of two parts: HIFα (HIF-1α, HIF-2α, and HIF-3α) and HIF-1β. HIF-1β is always located in the cell nucleus, while HIFα is located in the cytoplasm. Under conditions of sufficient oxygen, HIFα will finally be degraded by a proteasome via pathways including hydroxylation by prolyl hydroxylase (PHD), and ubiquitination by VHL (von Hippel-Lindau syndrome) ubiquitinase. However, when there is hypoxia or an abnormal metabolic pathway, HIFα cannot be degraded and thus accumulates in the nucleus, where it combines with HIF-1β to form a heterodimer, activating the hypoxia response elements (HREs) in the promoter of downstream genes, thereby regulating the transcription of related genes and allowing cells to survive under hypoxic conditions. These genes are involved in tumor angiogenesis, cell proliferation, survival, metabolism, invasion and metastasis, drug resistance, inflammation and immunity. HIF-2α mediates chronic hypoxia and can be continuously activated under physiological hypoxia conditions, playing a more critical role in the occurrence and development of tumors.

Current studies have shown that the mechanisms by which HIF-2α mediates the occurrence and development of tumors mainly include: 1. under conditions such as hypoxia or VHL mutation, the HIF-2α metabolic pathway is blocked, and it accumulates in the cell nucleus and forms a heterodimer with HIF-1β, thereby activating the hypoxia response elements (HREs), upregulating downstream cancer-related genes such as VEGFA, CXCR4, and Cyclin D1, and promoting tumor angiogenesis; 2. HIF-2α also participates in the immunosuppressive signaling by upregulating CD73 expression. Therefore, targeting HIF-2α can restore or enhance the anti-tumor immune function of mature DC cells, activated B cells and NK cells.

The activation of HIF-2α pathway is closely related to the occurrence and development of renal cell carcinoma, glioma, neuroblastoma, pheochromocytoma, etc. VHL protein is an important component of E3 ubiquitin ligase, which mediates protein degradation by the proteasome. The VHL gene has a high mutation rate of 57% or a 98% heterozygous loss in renal cell carcinoma (RCC), leading to pseudohypoxia and inducing HIF-2α activation and nuclear translocation. In addition, clear cell renal cell carcinoma (ccRCC) accounts for 70%-75% of primary renal cell malignancies, and more than 90% of ccRCC patients have defects in VHL protein. In the absence of vascularization, gliomas have unstable blood supply, leading to a hypoxic microenvironment, which induces local high expression of HIF-2α and promotes tumor growth. In pheochromocytomas and paragangliomas, the AA mutation rate at positions 529-532 of HIF-2α is as high as 81%, which directly affects the hydroxylation and degradation of HIF-2α and causes HIF-2α to remain activated.

The activation of HIF-2α and the formation of heterodimers with HIF-1β are the key factors leading to downstream activation, and the PAS binding domains of the two are the binding sites for the formation of heterodimers. Based on this, Peloton's R&D team developed a small molecule inhibitor PT2977 of HIF-2α, which exerts an anti-tumor effect by inhibiting the binding of HIF-2α and HIF-1β.

Since the HIF-2α pathway has a very important close relationship with tumor occurrence and migration, it is very necessary to develop more efficient new molecular entities.

### Summary of the Invention

The present invention firstly provides a compound as shown in formula (I), or a stereoisomer, tautomer, pharmaceutically acceptable salt, prodrug, chelate, non-covalent complex or solvate thereof, 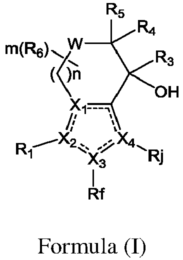 wherein,
-̅ -̅ -̅ -̅ -̅ -̅ represents a single bond or double bond;
X₁, X₂, X₃ and X₄ are each independently selected from C or N, and at least one of X₁, X₂, X₃ and X₄ is N;
W is selected from C, N, O or S;
R₁ is selected from C₁-C₁₀ alkyl, C₂-C₁₀ alkenyl, C₂-C₁₀ alkynyl, C₁-C₁₀ alkoxy, C₆-C₁₀ aryl, 5- to 18-membered heteroaryl, C₅-C₁₀ cycloalkyl, or 5- to 10-membered heterocyclyl; wherein the 5- to 18-membered heteroaryl and 5- to 10-membered heterocyclyl contain 1, 2 or 3 heteroatoms each independently selected from N, O and S; the C₁-C₁₀ alkyl, C₂-C₁₀ alkenyl, C₂-C₁₀ alkynyl, C₁-C₁₀ alkoxy, C₆-C₁₀ aryl, 5- to 18-membered heteroaryl, C₅-C₁₀ cycloalkyl and 5- to 10-membered heterocyclyl can be optionally substituted with one or more substituents selected from H, halogen, -OH, -CN, oxo, amino, C₁-C₆ alkyl, C₁-C₆ alkoxy, C₂-C₆ alkenyl, C₃-C₅ cycloalkyl, C₂-C₆ alkynyl, C₁-C₆ haloalkyl, cyano-substituted C₁-C₆ alkyl, cyano-substituted C₁-C₆ haloalkyl, C₁-C₆ haloalkoxy, -C₁-C₆ alkylene-OR_{c}, -C₁-C₆ alkylene-C=O-R_{c}, -NO₂, -OR_{c}, -SR_{c}, -NRₐR_{b}, -C(=O)R_{c}, -C(=O)OR_{c}, -C(=O)NRₐR_{b}, -NC(=O)R_{c}, - S(=O)R_{c}, -S(=O)₂R_{c}, -S(=O)₂NRₐR_{b}, -S(=O)(=NRₐ)R_{b}, -P(=O)RₐR_{b} or -P(=S)RₐR_{b};
R₃ is selected from H, deuterium, halogen, -NO₂, -CN, -OH, C₁-C₁₀ alkyl, C₂-C₁₀ alkenyl, C₁-C₅ haloalkyl, C₁-C₁₀ alkoxy, -O-C(=O)-C₁₃ alkyl, -C(=O)-O-C₁₋₃ alkyl or C₂-C₁₀ alkynyl, wherein the C₁-C₁₀ alkyl, C₂-C₁₀ alkenyl, C₁-C₅ haloalkyl, C₁-C₁₀ alkoxy, -O-C(=O)-C₁₋₃ alkyl, -C(=O)-O-C₁₋₃ alkyl, and C₂-C₁₀ alkynyl can be optionally substituted with one or more substituents selected from H, halogen, -CN, -OH, amino, C₁-C₅ alkyl, C₁-C₆ alkoxy, C₂-C₆ alkenyl or C₁-C₅ haloalkyl;
R₄ and R₅ are each independently selected from H, halogen, -NO₂, -NRₐR_{b}, -OH, C₁-C₆ alkyl, C₁-C₆ alkoxy, C₁-C₆ haloalkyl, C₂-C₁₀ alkenyl, C₂-C₁₀ alkynyl, C₃-C₅ cycloalkyl and 3- to 6-membered heterocyclyl; the C₁-C₆ alkyl, C₁-C₆ alkoxy, C₁-C₆ haloalkyl, C₂-C₁₀ alkenyl, C₂-C₁₀ alkynyl, C₃-C₅ cycloalkyl and 3- to 6-membered heterocyclyl can be optionally substituted with one or more substituents selected from H, halogen, -CN, -OH, amino, oxo, C₁-C₅ alkyl, C₁-C₆ alkoxy, C₂-C₆ alkenyl or C₁-C₅ haloalkyl; or R₄ and R₅ together form oxo; or R₄ and R₅ together with the C atom to which they are attached form substituted or unsubstituted cyclopropyl;
R₆ is selected from H, -CN, halogen, -NO₂, -OH, -NO₂, -NRₐR_{b}, oxo, C₁-C₆ alkyl, C₁-C₆ alkoxy, C₁-C₆ haloalkyl, C₂-C₁₀ alkenyl, C₂-C₁₀ alkynyl, C₃-C₅ cycloalkyl and 3- to 6-membered heterocyclyl; the C₁-C₆ alkyl, C₁-C₆ alkoxy, C₁-C₆ haloalkyl, C₂-C₁₀ alkenyl, C₂-C₁₀ alkynyl, C₃-C₅ cycloalkyl and 3- to 6-membered heterocyclyl can be optionally substituted with one or more substituents selected from H, halogen, -CN, -OH, amino, oxo, C₁-C₅ alkyl, C₁-C₆ alkoxy, C₂-C₆ alkenyl or C₁-C₅ haloalkyl; or two R₆ together with the C atom to which they are attached form substituted or unsubstituted C₃-C₅ cycloalkyl or 3- to 5-membered heterocyclyl;
or R₆ and R₅ together with the C atom to which they are attached form substituted or unsubstituted C₃-C₄ cycloalkyl;
R_{f} is absent or selected from H, -CN, halogen, C₁-C₁₀ alkyl, C₁-C₁₀ haloalkyl, C₃-C₁₀ cycloalkyl, oxo, or - NRₐR_{b}; the C₁-C₁₀ alkyl, C₁-C₁₀ haloalkyl and C₃-C₁₀ cycloalkyl can be optionally substituted with one or more substituents selected from H, halogen, -CN, -OH, amino, C₁-C₅ alkyl, C₁-C₆ alkoxy, C₂-C₆ alkenyl or C₁-C₅ haloalkyl;
Rⱼ is selected from -C₁-C₆ alkylene-C(=O)R_{c}, -C₁-C₆ alkylene-C(=O)OR_{c}, -C₂F₅, -C(=O)R_{c}, -C(=O)OR_{c}, - SR_{c}, -S(=O)R_{c}, -S(=O)₂R_{C} or -S(=O)(=NRₐ)R_{c}, wherein the C₁-C₆ alkylene can be optionally substituted with one or more substituents selected from H, halogen, cyano, C₁-C₄ alkyl, C₂-C₄ alkenyl, C₂-C₄ alkynyl, C_{¡}-C₄ haloalkyl, C_{¡}-C₄ alkoxy and C_{¡}-C₄ haloalkoxy;
Rₐ and R_{b} are each independently selected from H, CN, C₁-C₁₀ alkyl, C₁-C₁₀ haloalkyl, C₁-C₁₀ alkoxy, C₂-C₁₀ alkenyl, C₂-C₁₀ alkynyl, C₃-C₁₀ cycloalkyl, 3- to 10-membered heterocyclyl, C₆-C₁₀ aryl or 5- to 10-membered heteroaryl;
R_{c} is selected from C₁-C₁₀ alkyl, C₁-C₁₀ alkoxy, C₃-C₆ cycloalkyl and -NRₐR_{b}, wherein the C₁-C₁₀ alkyl, C₁-C₁₀ alkoxy and C₃-C₆ cycloalkyl can be optionally substituted with one or more substituents selected from H, halogen, CN, -NH₂, oxo, C₁-C₄ haloalkyl, C₁-C₄ haloalkoxy, and C₃-C₆ cycloalkyl;
n is 0 or 1;
m is 0, 1, 2, 3 or 4;
provided that the compound is not:
   3-fluoro-5-(4-hydroxyl-3-(methylsulfonyl)-4,5,6,7-tetrahydro-1H-indol-1-yl)benzonitrile;
   1-(3-chloro-5-fluorophenyl)-5,5-difluoro-3-(methylsulfonyl)-4,5,6,7-tetrahydro-1H-indol-4-ol;
   3-(5,5-difluoro-4-hydroxyl-3-(methylsulfonyl)-4,5,6,7-tetrahydro-1H-indol-1-yl)-5-fluorobenzonitrile;
   3-(3-((difluoromethyl)sulfonyl)-5,5-difluoro-4-hydroxyl-4,5,6,7-tetrahydro-1H-indol-1-yl)-5-fluorobenzonitrile;
   1-(3,5-difluorophenyl)-5,5-difluoro-3-(methylsulfonyl)-4,5,6,7-tetrahydro-1H-indol-4-ol;
   1-(3-chloro-5-fluorophenyl)-5,6-difluoro-3-(methylsulfonyl)-4,5,6,7-tetrahydro-1H-indol-4-ol;
   (S)-5-(5,5-difluoro-4-hydroxyl-3-(methylsulfonyl)-4,5,6,7-tetrahydro-1H-indol-1-yl)-2-fluorobenzonitrile;
   1-(3-chloro-5-fluorophenyl)-5,5-difluoro-3-((S)-methylsulfoxyl)-4,5,6,7-tetrahydro-1H-indol-4-ol;
   1-(3-chloro-5-fluorophenyl)-5,5-difluoro-3-((R)-methylsulfoxyl)-4,5,6,7-tetrahydro-1H-indol-4-ol;
   3-fluoro-5-(5-fluoro-4-hydroxyl-3-(methylsulfonyl)-4,5,6,7-tetrahydro-1H-indol-1-yl)benzonitrile (193);
   (S)-1-(3,5-difluorophenyl)-5,5-difluoro-3-(methylsulfonyl)-2-(trifluoromethyl)-4,5,6,7-tetrahydro-1H-indol-4-ol;
   2-chloro-5-(5,5-difluoro-4-hydroxyl-3-(methylsulfonyl)-5,6-dihydrocyclopenta[b]pyrrol-1(4H)-yl)benzonitrile; and
   1-(3-chloro-5-fluorophenyl)-3-((difluoromethyl)sulfonyl)-5,5-difluoro-1,4,5,6-tetrahydrocyclopenta[b]pyrrol-4-ol.

In some embodiments, the compound is as shown in formula (II-1) or formula (II-2):

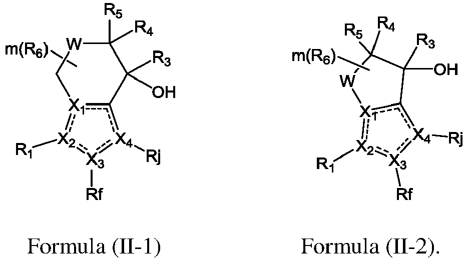

In some embodiments, R₃ is selected from H, deuterium, C₁-C₁₀ alkyl or C₂-C₁₀ alkenyl, wherein the C₁-C₁₀ alkyl and C₂-C₁₀ alkenyl can be optionally substituted with one or more substituents selected from H, halogen, -CN, -OH, amino, or C₁-C₅ haloalkyl.

In some embodiments, R₃ is selected from H, deuterium, C₃-C₃ alkyl or C₂-C₅ alkenyl, wherein the C₁-C₃ alkyl and C₂-C₅ alkenyl can be optionally substituted with one or more substituents selected from H, halogen, -CN, -OH, amino, or C₁-C₅ haloalkyl.

In some embodiments, R₃ is selected from H, deuterium or C₃-C₃ alkyl, wherein the C₁-C₃ alkyl can be optionally substituted with one or more substituents selected from H, halogen, -CN, -OH, amino, and C₁-C₅ haloalkyl.

In some embodiments, R₃ is selected from H, deuterium or C₁-C₃ alkyl.

In some embodiments, R₃ is H or deuterium.

In some embodiments, R₃ is H.

In some embodiments, R₄ and R₅ are each independently selected from H, halogen or C₁-C₆ alkyl, wherein the C₁-C₆ alkyl can be optionally substituted with one or more substituents selected from H, halogen, -CN, -OH, amino or oxo.

In some embodiments, R₄ and R₅ are each independently selected from H, halogen or C₁-C₆ alkyl.

In some embodiments, R₄ and R₅ are each independently selected from H or halogen.

In some embodiments, R₄ and R₅ are each independently selected from H or F.

In some embodiments, R_{f} is selected from H, -CN, -NH₂, halogen, -NO₂, C₁-C₃ alkyl, cyclopropyl or C₁-C₃ haloalkyl, wherein the C₁-C₃ alkyl, cyclopropyl or C₃-C₃ haloalkyl can be optionally substituted with one or more substituents selected from H, halogen, -CN, -OH, amino, C₁-C₃ alkyl, C₂-C₄ alkenyl, or C₁-C₃ haloalkyl.

In some embodiments, R_{f} is selected from H, -CN, halogen, C₁-C₃ alkyl or C₁-C₃ haloalkyl.

In some embodiments, R_{f} is H, -CN or halogen.

In some embodiments, R_{f} is H or halogen.

In some embodiments, R_{f} is H, -CN, -F, -Cl, -Br or -CF₃.

In some embodiments, R_{f} is H.

In some embodiments, W is C or O.

In some embodiments, W is C.

In some embodiments, R₆ is H, -CN, halogen, C₁-C₆ alkyl, C₁-C₆ alkoxy or C₁-C₆ haloalkyl, wherein the C₁-C₆ alkyl, C₁-C₆ alkoxy or C₁-C₆ haloalkyl can be optionally substituted with one or more substituents selected from H, halogen, -CN, -OH, oxo, or amino.

In some embodiments, R₆ is H, halogen or C₁-C₆ alkyl, wherein the C₁-C₆ alkyl can be optionally substituted with one or more substituents selected from H, halogen, -CN, -OH, oxo, or amino.

In some embodiments, R₆ is H, halogen or C₁-C₆ alkyl.

In some embodiments, R₆ is H, halogen or C₁-C₃ alkyl.

In some embodiments, R₆ is H or halogen.

In some embodiments, R₆ is H, F, Cl or Br.

In some embodiments, R₆ is H or F.

In some embodiments, R₁ is C₆-C₁₀ aryl, 5- to 10-membered heteroaryl, C₅-C₁₀ cycloalkyl or 5- to 10-membered heterocyclyl, wherein the 5- to 10-membered heteroaryl and 5- to 10-membered heterocyclyl contain 1, 2 or 3 heteroatoms each independently selected from N, O and S, and the C₆-C₁₀ aryl, 5- to 10-membered heteroaryl, C₅-C₁₀ cycloalkyl or 5- to 10-membered heterocyclyl can be optionally substituted with one or more substituents selected from halogen, -OH, -CN, oxo, amino, C₁-C₆ alkyl, C₁-C₆ alkoxy, C₁-C₆ haloalkyl, cyano-substituted C₁-C₆ alkyl, cyano-substituted C₁-C₆ haloalkyl, - C₁-C₆ alkylene-OR_{c}, -C₁-C₆ alkylene-C=O-R_{c}, -NO₂, C(=O)OR_{c} or -S(=O)₂R_{c}.

In some embodiments, R₁ is C₆-C₁₀ aryl, 5- to 10-membered heteroaryl, C₅-C₁₀ cycloalkyl or 5- to 10-membered heterocyclyl, wherein the 5- to 10-membered heteroaryl and 5- to 10-membered heterocyclyl contain 1 or 2 N heteroatoms, and the C₆-C₁₀ aryl, 5- to 10-membered heteroaryl, C₅-C₁₀ cycloalkyl or 5- to 10-membered heterocyclyl can be optionally substituted with one or more substituents selected from halogen, hydroxyl, cyano, amino, C₁-C₆ alkyl, C₃-C₅ cycloalkyl, C₁-C₆ haloalkyl, cyano-substituted C₁-C₆ alkyl, cyano-substituted C₁-C₆ haloalkyl or C₁-C₆ haloalkoxy.

In some embodiments, R₁ is C₆-C₁₀ aryl, 5- to 10-membered heteroaryl, C₅-C₁₀ cycloalkyl or 5- to 10-membered heterocyclyl, wherein the 5- to 10-membered heteroaryl and 5- to 10-membered heterocyclyl contain 1 or 2 N heteroatoms, and the C₆-C₁₀ aryl, 5- to 10-membered heteroaryl, C₅-C₁₀ cycloalkyl or 5- to 10-membered heterocyclyl can be optionally substituted with one or more substituents selected from halogen, cyano, cyano-substituted C₁-C₆ haloalkyl, cyano-substituted C₁-C₆ alkyl or C₁-C₆ haloalkyl.

In some embodiments, R₁ is phenyl, pyridyl, naphthyl, 10-membered heteroaryl, C₈-C₁₀ cycloalkyl or 8- to 10-membered heterocyclyl, wherein the 10-membered heteroaryl and 8- to 10-membered heterocyclyl contain 1 or 2 N heteroatoms, and the phenyl, pyridyl, naphthyl, 10-membered heteroaryl, C₈-C₁₀ cycloalkyl or 8- to 10-membered heterocyclyl can be optionally substituted with one or more substituents selected from halogen, cyano or C₁-C₆ haloalkyl.

In some embodiments, R₁ is phenyl, wherein the phenyl can be optionally substituted with one or more substituents selected from halogen, -CN or C₁-C₆ haloalkyl.

In some embodiments, R₁ is phenyl, wherein the phenyl can be optionally substituted with one or more substituents selected from halogen, -CN, -CH₂F, -CHF₂, -CF₃, -CH₂CHF₂ or -CH₂CF₃.

In some embodiments, Rⱼ is selected from -C₁-C₆ alkylene-C(=O)R_{c}, -C₁-C₆ haloalkylene-C(=O)R_{c}, -C₁-C₆ haloalkylene-C(=O)OR_{c}, -C₂F₅, -C₁-C₆ cyano-substituted alkylene-C(=O)R_{c}, -C(=O)R_{c}, -C(=O)OR_{c}, -SR_{c}, -S(=O)R_{c}, -S(=O)₂R_{c} or -S(=O)(=NRₐ)R_{c}.

In some embodiments, Rⱼ is selected from -C₂F₅, -C₁-C₆ haloalkylene-C(=O)R_{c}, -C₁-C₆ haloalkylene-C(=O)OR_{c}, -C(=O)OR_{c}, -C(=O)R_{c}, -SR_{c}, -S(=O)₂R_{c} or -S(=O)(=NRₐ)R_{c}.

In some embodiments, Rⱼ is -SR_{c}, -S(=O)₂R_{c} or -S(=O)(=NRₐ)R_{c}.

In some embodiments, R_{c} is selected from C₁-C₆ alkyl, C₁-C₆ alkoxy, C₃-C₆ cycloalkyl and -NH₂, wherein the C₁-C₆ alkyl, C₁-C₆ alkoxy and C₃-C₆ cycloalkyl can be optionally substituted with one or more substituents selected from H, halogen, CN, -NH₂, C₁-C₄ haloalkyl or C₃-C₆ cycloalkyl.

In some embodiments, R_{c} is selected from C₁-C₆ alkyl, C₁-C₆ alkoxy and -NH₂, wherein the C₁-C₆ alkyl and C₁-C₆ alkoxy can be optionally substituted with one or more substituents selected from H, halogen, CN, -NH₂, and C₁-C₄ haloalkyl.

In some embodiments, R_{c} is selected from C₁-C₆ alkyl, wherein the C₁-C₆ alkyl can be optionally substituted with one or more substituents selected from H, halogen and CN.

In some embodiments, Rₐ is selected from H, CN or C₁-C₄ alkyl.

In some embodiments, R_{b} is selected from H, CN or C₁-C₄ alkyl.

In some embodiments, Rⱼ is selected from

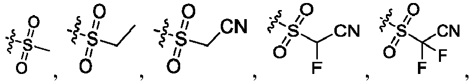

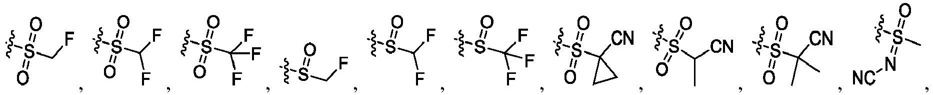

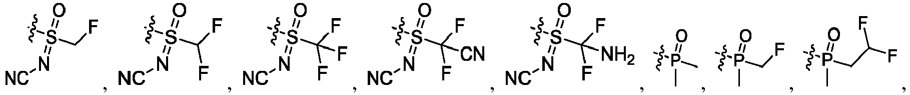

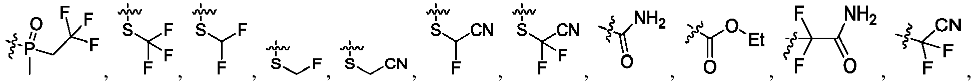

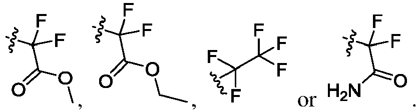

In some embodiments, Rⱼ is selected from

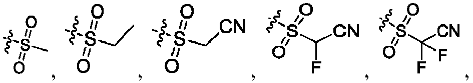

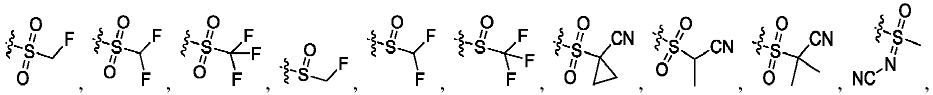

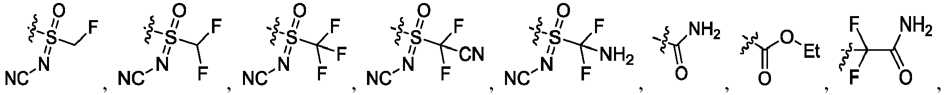

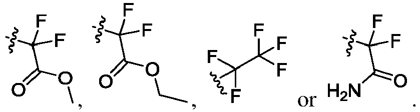

In some embodiments, the compound is as shown in formula (III-1), formula (III-2), formula (III-3) or formula (III-4): 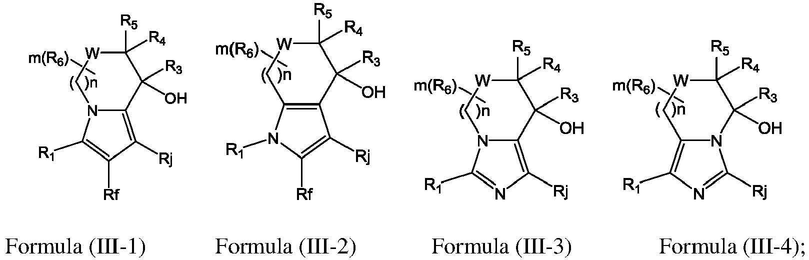 wherein R₁, R₃, R₄, R₅, R₆, R_{f}, Rⱼ, m, n and W are as defined in the above embodiments.

In some embodiments, the compound is as shown in formula (VI-1): 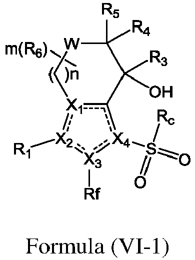 wherein R₁, R₃, R₄, R₅, R₆, R_{f}, R_{c}, X₁, X₂, X₃, X₄, m, n and W are as defined in the above embodiments.

In some embodiments, the compound is as shown in formula (VI-2): 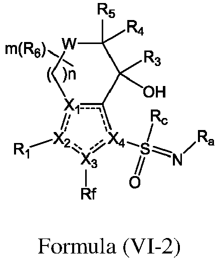 wherein R₁, R₃, R₄, R₅, R₆, R_{f}, Rₐ, R_{c}, X₁, X₂, X₃, X₄, m, n and W are as defined in the above embodiments.

In some embodiments, the compound is as shown in formula (VII-1), formula (VII-2), formula (VII-3) or formula (VII-4): 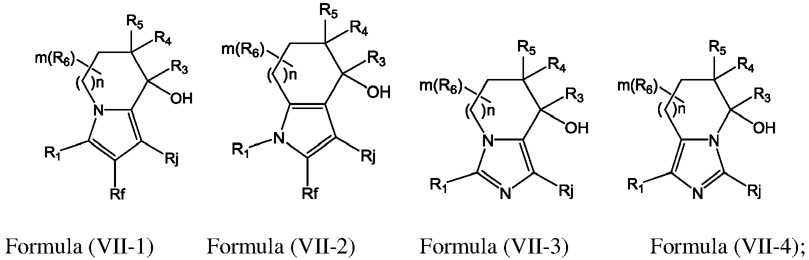 wherein R₁, R₃, R₄, R₅, R₆, R_{f}, Rⱼ, m and n are as defined in the above embodiments.

In some embodiments, the compound is as shown in formula (VIII-1), formula (VIII-2), formula (VIII-3) or formula (VIII-4): 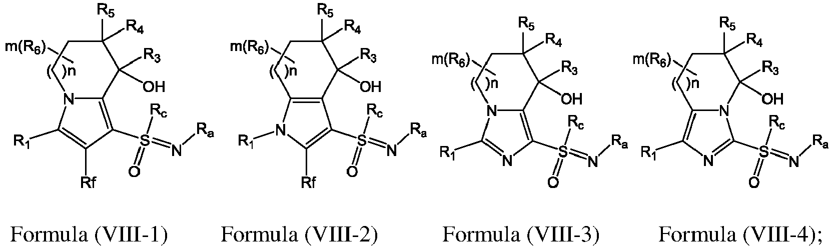 wherein R₁, R₃, R₄, R₅, R₆, R_{f}, R_{c}, Rₐ, m and n are as defined in the above embodiments.

In some embodiments, the compound is as shown in formula (VIII-5), formula (VIII-6), formula (VIII-7) or formula (VIII-8): 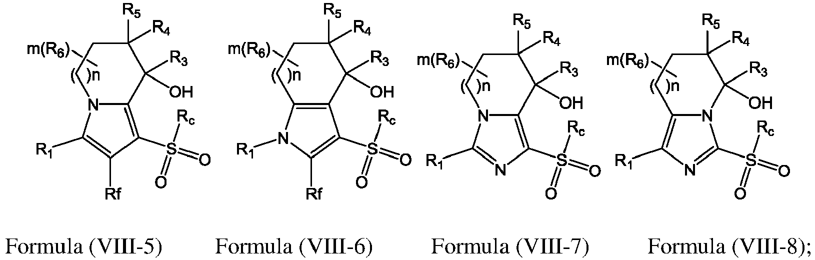 wherein R₁, R₃, R₄, R₅, R₆, R_{f}, R_{c}, m and n are as defined in the above embodiments.

In some embodiments, the compound is as shown in formula (IX-1) or formula (IX-2): 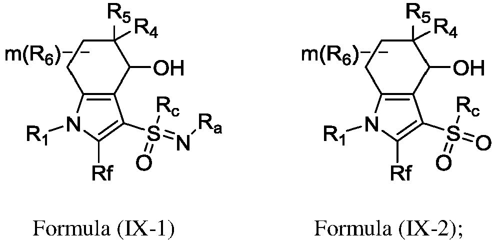 wherein R₁, R₄, R₅, R₆, R_{f}, R_{c}, Rₐ and m are as defined in the above embodiments.

In some embodiments, the compound is as shown in formula (X-1), formula (X-2), formula (X-3), formula (X-4), formula (X-5), formula (X-6), formula (X-7) or formula (X-8): 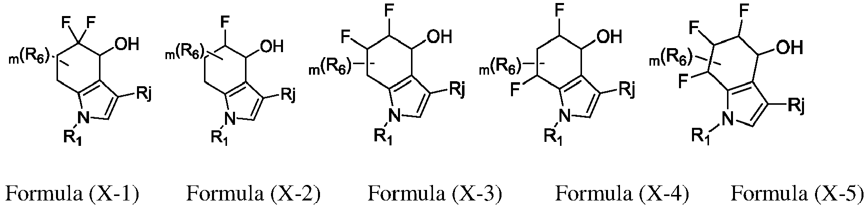 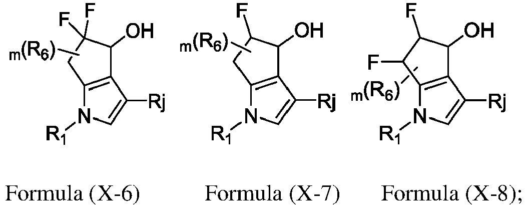 wherein R₁, R₆, Rⱼ and m are as defined in the above embodiments.

In some embodiments, the compound is as shown in formula (XI-1) or formula (XI-2), 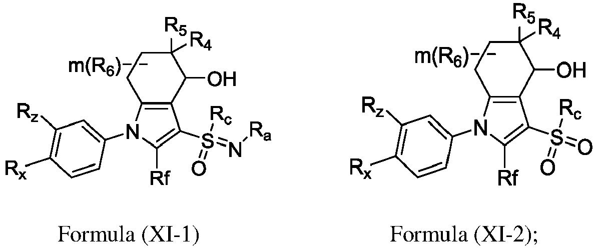
wherein R₄, R₅, R₆, R_{f}, R_{c}, Rₐ and m are as defined in the above embodiments;
Rₓ and R_{z} are each independently selected from halogen, -OH, -CN, oxo, amino, C₁-C₆ alkyl, C₁-C₆ alkoxy, C₁-C₆ haloalkyl, cyano-substituted C₁-C₆ alkyl, cyano-substituted C₁-C₆ haloalkyl, -C₁-C₆ alkylene-OR_{c}, -C₁-C₆ alkylene-C=O-R_{c}, -NO₂, -C(=O)OR_{c} and -S(=O)₂R_{c}.

The present invention further provides a compound or a pharmaceutically acceptable salt thereof, wherein the compound refers to:
1) 5-(5,5-difluoro-4-hydroxyl-3-(trifluoromethyl)-4,5,6,7-tetrahydro-1*H*-indol-1-yl)-2,3-difluorobenzonitrile;
2) 5,5-difluoro-1-(4-fluoro-3-(fluoromethyl)phenyl)-3-(trifluoromethyl)-4,5,6,7-tetrahydro-1*H-*indol-4-ol;
3) (*S*)-4-(5,5-difluoro-4-hydroxyl-3-(trifluoromethyl)-4,5,6,7-tetrahydro-1*H*-indol-1-yl)-2-(difluoromethyl)benzonitrile;
4) (*S*)-5-(3-((difluoromethyl)sulfonyl)-5,5-difluoro-4-hydroxyl-4,5,6,7-tetrahydro-1*H*-indol-1-yl)-2-fluorobenzonitrile;
5) (*S*)-1-(3-(difluoromethyl)-4-fluorophenyl)-5,5-difluoro-3-(methylsulfonyl)-4,5,6,7-tetrahydro-1*H-*indol-4-ol;
6) (*S*)-1-(3-(difluoromethyl)-4-fluorophenyl)-3-((difluoromethyl)sulfonyl)-5,5-difluoro-4,5,6,7-tetrahydro-1*H*-indol-4-ol;
7) (*S*)-3-((*S*)-(difluoromethyl)sulfinyl)-5,5-difluoro-1-(4-fluoro-3-(trifluoromethyl)phenyl)-4,5,6,7-tetrahydro-1*H*-indol-4-ol;
8) (*S*)-3-((*R*)-(difluoromethyl)sulfinyl)-5,5-difluoro-1-(4-fluoro-3-(trifluoromethyl)phenyl)-4,5,6,7-tetrahydro-1*H*-indol-4-ol;
9) 5-(5,5-difluoro-4-hydroxyl-3-(perfluoroethyl)-4,5,6,7-tetrahydro-1*H*-indol-1-yl)-2-fluorobenzonitrile;
10) (*S*)-5-(5,5-difluoro-4-hydroxyl-3-((trifluoromethyl)sulfonyl)-4,5,6,7-tetrahydro-1*H*-indol-1-yl)-2-fluorobenzonitrile;
11) (*S*)-5-(5,5-difluoro-3-((fluoromethyl)sulfonyl)-4-hydroxyl-4,5,6,7-tetrahydro-1*H*-indol-1-yl)-2-fluorobenzonitrile;
12) (*S*)-1-(3-(difluoromethyl)-4-fluorophenyl)-5,5-difluoro-3-((trifluoromethyl)sulfonyl)-4,5,6,7-tetrahydro-1*H*-indol-4-ol;
13) (*S*)-2-((5,5-difluoro-1-(4-fluoro-3-(trifluoromethyl)phenyl)-4-hydroxyl-4,5,6,7-tetrahydro-1*H-*indol-3-yl)sulfonyl)acetonitrile;
14) (*S*)-1-(3-(difluoromethyl)-4-fluorophenyl)-5,5-difluoro-3-((fluoromethyl)sulfonyl)-4,5,6,7-tetrahydro-1*H*-indol-4-ol;
15) 2-(1-(3-cyano -4-fluorophenyl)-5,5-difluoro-4-hydroxyl-4,5,6,7-tetrahydro-1*H*-indol-3-yl)-2,2-difluoroacetamide;
16) (*S*)-3-(ethylsulfonyl)-5,5-difluoro-1-(4-fluoro-3-(trifluoromethyl)phenyl)-4,5,6,7-tetrahydro-1*H-*indol-4-ol;
17) 5-(3-(cyanodifluoromethyl)-5,5-difluoro-4-hydroxyl-4,5,6,7-tetrahydro-1*H*-indol-1-yl)-2-fluorobenzonitrile;
18) *N*-(((*S*)-1-(3-cyano-4-fluorophenyl)-5,5-difluoro-4-hydroxyl-4,5,6,7-tetrahydro-1*H*-indol-3-yl)(methyl)(oxo)-16-sulfino)cyanamide;
19) (*S*)-2-((1-(3-(difluoromethyl)-4-fluorophenyl)-5,5-difluoro-4-hydroxyl-4,5,6,7-tetrahydro-1*H-*indol-3-yl)sulfonyl)acetonitrile;
20) (*S*)-5,5-difluoro-1-(4-fluoro-3-(trifluoromethyl)phenyl)-3-(methylsulfonyl)-4,5,6,7-tetrahydro-1*H*-indol-4-ol;
21) (*S*)-3-((difluoromethyl)sulfonyl)-5,5-difluoro-1-(4-fluoro-3-(trifluoromethyl)phenyl)-4,5,6,7-tetrahydro-1*H*-indol-4-ol;
22) (*S*)-5-(3-((difluoromethyl)sulfonyl)-2,5,5-trifluoroether-4-hydroxyl-4,5,6,7-tetrahydro-1*H*-indol-1-yl)-2-fluorobenzonitrile;
23) (*S*)-5,5-difluoro-1-(4-fluoro-3-(trifluoromethyl)phenyl)-3-((trifluoromethyl)sulfonyl)-4,5,6,7-tetrahydro-1*H*-indol-4-ol;
24) 1-(3-chloro-4-fluorophenyl)-5,5-difluoro-3-(methylsulfonyl)-4,5,6,7-tetrahydro-1*H*-indol-4-ol;
25) 1-(3-chloro-4-fluorophenyl)-3-((difluoromethyl)sulfonyl)-5,5-difluoro-4,5,6,7-tetrahydro-1*H-*indol-4-ol;
26) (*R*)-1-(3-(difluoromethyl)-4-fluorophenyl)-3-((difluoromethyl)sulfonyl)-5,5-difluoro-4,5,6,7-tetrahydro-1*H*-indol-4-ol;
27) (*S*)-2-chloro-5-(5,5-difluoro-4-hydroxyl-3-(methylsulfonyl)-4,5,6,7-tetrahydro-1*H*-indol-1 - yl)benzonitrile;
28) (*S*)-2-chloro-5-(3-((difluoromethyl)sulfonyl)-5,5-difluoro-4-hydroxyl-4,5,6,7-tetrahydro-1*H-*indol-1-yl)benzonitrile;
29) (*S*)-4-(5,5-difluoro-4-hydroxyl-3-(methylsulfonyl)-4,5,6,7-tetrahydro-1*H*-indol-1-yl)-2-(difluoromethyl)benzonitrile;
30) (*S*)-5,5-difluoro-1-(4-fluoro-3-(trifluoromethyl)phenyl)-3-((fluoromethyl)sulfonyl)-4,5,6,7-tetrahydro-1*H*-indol-4-ol;
31) (*S*)-4-(5,5-difluoro-4-hydroxyl-3-((trifluoromethyl)sulfonyl)-4,5,6,7-tetrahydro-1*H*-indol-1-yl)-2-(trifluoromethyl)benzonitrile;
32) (*R*)-1-(3-(difluoromethyl)-4-fluorophenyl)-3-((difluoromethyl)sulfonyl)-4,5,6,7-tetrahydro-1*H-*indol-4-ol;
33) (*S*)-1-(3-chloro-4-fluorophenyl)-5,5-difluoro-3-((trifluoromethyl)sulfonyl)-4,5,6,7-tetrahydro-1*H-*indol-4-ol;
34) (*S*)-4-(3-((difluoromethyl)sulfonyl)-5,5-difluoro-4-hydroxyl-4,5,6,7-tetrahydro-1*H*-indol-1-yl)-2-(trifluoromethyl)benzonitrile;
35) (*S*)-4-(5,5-difluoro-4-hydroxyl-3-((trifluoromethyl)sulfonyl)-4,5,6,7-tetrahydro-1*H*-indol-1-yl)-2-(difluoromethyl)benzonitrile;
36) (*S*)-4-(5,5-difluoro-3-((fluoromethyl)sulfonyl)-4-hydroxyl-4,5,6,7-tetrahydro-1*H*-indol-1-yl)-2-(trifluoromethyl)benzonitrile;
37) (*S*)-1-(3-chloro-4-fluorophenyl)-5,5-difluoro-3-((fluoromethyl)sulfonyl)-4,5,6,7-tetrahydro-1*H-*indol-4-ol;
38) (*S*)-4-(5,5-difluoro-4-hydroxyl-3-(methylsulfonyl)-4,5,6,7-tetrahydro-1H-indol-1-yl)phthalonitrile;
39) (*S*)-4-(3-((difluoromethyl)sulfonyl)-5,5-difluoro-4-hydroxyl-4,5,6,7-tetrahydro-1*H*-indol-1-yl)phthalonitrile;
40) (*S*)-2-chloro-5-(5,5-difluoro-4-hydroxyl-3-((trifluoromethyl)sulfonyl)-4,5,6,7-tetrahydro-1*H-*indol-1-yl)benzonitrile;
41) 4-(5,5-difluoro-4-hydroxyl-3-(perfluoroethyl)-4,5,6,7-tetrahydro-1*H*-indol-1-yl)-2-(difluoromethyl)benzonitrile;
42) (*S*)-4-(5,5-difluoro-4-hydroxyl-3-((trifluoromethyl)sulfonyl)-4,5,6,7-tetrahydro-1*H*-indol-1-yl)phthalonitrile;
43) (*S*)-2-(difluoromethyl)-4-(3-((difluoromethyl)sulfonyl)-5,5-difluoro-4-hydroxyl-4,5,6,7-tetrahydro-1*H*-indol-1-yl)benzonitrile;
44) (*S*)-4-(5,5-difluoro-3-((fluoromethyl)sulfonyl)-4-hydroxyl-4,5,6,7-tetrahydro-1*H*-indol-1-yl)-2-(difluoromethyl)benzonitrile;
45) (*S*)-4-(5,5-difluoro-3-((fluoromethyl)sulfonyl)-4-hydroxyl-4,5,6,7-tetrahydro-1H-indol-1-yl)phthalonitrile;
46) (*S*)-4-(5,5-difluoro-4-hydroxyl-3-(perfluoroethyl)-4,5,6,7-tetrahydro-1*H*-indol-1 -yl)phthalonitrile;
47) 2-(((*S*)-1-(3-(difluoromethyl)-4-fluorophenyl)-5,5-difluoro-4-hydroxyl-4,5,6,7-tetrahydro-1*H-*indol-3-yl)sulfonyl)-2-fluoroacetonitrile;
48) ethyl (*S*)-2-(1-(3-(difluoromethyl)-4-fluorophenyl)-5,5-difluoro-4-hydroxyl-4,5,6,7-tetrahydro-1*H*-indol-3-yl)-2,2-difluoroacetate;
49) (*S*)-2-(1-(3-(difluoromethyl)-4-fluorophenyl)-5,5-difluoro-4-hydroxyl-4,5,6,7-tetrahydro-1*H-*indol-3-yl)-2,2-difluoroacetonitrile;
50) (*S*)-2-(1-(3-(difluoromethyl)-4-fluorophenyl)-5,5-difluoro-4-hydroxyl-4,5,6,7-tetrahydro-1*H-*indol-3-yl)-2,2-difluoroacetamide;
51) (*S*)-1-(3-(difluoromethyl)-4-fluorophenyl)-5,5-difluoro-3-(thiazol-2-yl)-4,5,6,7-tetrahydro-1*H-*indol-4-ol;
52) (*R*)-1-(3-(difluoromethyl)-4-fluorophenyl)-3-((trifluoromethyl)sulfonyl)-4,5,6,7-tetrahydro-1*H-*indol-4-ol;
53) (*S*)-1-(3-(difluoromethyl)-4-fluorophenyl)-5,5-difluoro-3-(oxazol-2-yl)-4,5,6,7-tetrahydro-1*H-*indol-4-ol;
54) (*S*)-2-((1-(3-(difluoromethyl)-4-fluorophenyl)-5,5-difluoro-4-hydroxyl-4,5,6,7-tetrahydro-1*H-*indol-3-yl)sulfonyl)-2,2-difluoroacetonitrile;
55) 2-(((*S*)-5,5-difluoro-1-(4-fluoro-3-(trifluoromethyl)phenyl)-4-hydroxyl-4,5,6,7-tetrahydro-1*H-*indol-3-yl)sulfonyl)-2-fluoroacetonitrile;
56) (*S*)-(1-(3-(difluoromethyl)-4-fluorophenyl)-5,5-difluoro-4-hydroxyl-4,5,6,7-tetrahydro-1*H*-indol-3-yl) dimethylphosphine oxide methyl;
57) 2-(((*S*)-1-(3-(difluoromethyl)-4-fluorophenyl)-5,5-difluoro-4-hydroxyl-4,5,6,7-tetrahydro-1*H-*indol-3-yl)sulfonyl) propanenitrile;
58) (*S*)-1-((1-(3-(difluoromethyl)-4-fluorophenyl)-5,5-difluoro-4-hydroxyl-4,5,6,7-tetrahydro-1*H-*indol-3-yl)sulfonyl) cyclopropane-1-nitrile;
59) *N*-(((*S*)-1-(3-(difluoromethyl)-4-fluorophenyl)-5,5-difluoro-4-hydroxyl-4,5,6,7-tetrahydro-1*H-*indol-3-yl)(methyl)(oxo)-16-sulfino)cyanamide;
60) ethyl (*S*)-1-(3-(difluoromethyl)-4-fluorophenyl)-5,5-difluoro-4-hydroxyl-4,5,6,7-tetrahydro-1*H-*indole -3-carboxylate;
61) (*R*)-1-(3-(difluoromethyl)-4-fluorophenyl)-5,5-difluoro-3-((fluoromethyl)sulfonyl)-4,5,6,7-tetrahydro-1*H*-indol-4-ol;
62) (*S*)-1-(3-(difluoromethyl)-4-fluorophenyl)-5,5-difluoro-4-hydroxyl-4,5,6,7-tetrahydro-1*H*-indole-3-carboxamide;
63) (4*S*,5*R*)-1-(3-(difluoromethyl)-4-fluorophenyl)-3-((difluoromethyl)sulfonyl)-5-fluoro-4,5,6,7-tetrahydro-1*H*-indol-4-ol;
64) (4*S*,5*S*)-1-(3-(difluoromethyl)-4-fluorophenyl)-3-((difluoromethyl)sulfonyl)-5-fluoro-4,5,6,7-tetrahydro-1*H*-indol-4-ol;
65) (*R*)-2-(difluoromethyl)-4-(4-hydroxyl-3-((trifluoromethyl)sulfonyl)-4,5,6,7-tetrahydro-1*H*-indol-1-yl)benzonitrile;
66) (*S*)-4-(5,5-difluoro-4-hydroxyl-3-(trifluoromethyl)-4,5,6,7-tetrahydro-1*H*-indol-1-yl)-3-fluorobenzonitrile;
67) (4*S*)-1-(3-(difluoromethyl)-4-fluorophenyl)-5,5-difluoro-3-((fluoromethyl)sulfinyl)-4,5,6,7-tetrahydro-1*H*-indol-4-ol;
68) (*S*)-1-((*R*)-6,8-difluoro-1,2,3,4-tetrahydronaphthalen-1-yl-yl)-5,5-difluoro-3-(trifluoromethyl)-4,5,6,7-tetrahydro-1*H*-indol-4-ol;
69) (*S*)-1-((*S*)-6,8-difluoro-1,2,3,4-tetrahydronaphthalen-1-yl-yl)-5,5-difluoro-3-(trifluoromethyl)-4,5,6,7-tetrahydro-1*H*-indol-4-ol;
70) (*S*)-1-(2,4-difluorobenzyl)-5,5-difluoro-3-(trifluoromethyl)-4,5,6,7-tetrahydro-1*H*-indol-4-ol;
71) (*S*)-2-((5,5-difluoro-4-hydroxyl-3-(trifluoromethyl)-4,5,6,7-tetrahydro-1*H*-indol-1-yl)methyl)-5-fluorobenzonitrile;
72) (*S*)-1-(2,4-difluorophenyl)-5,5-difluoro-3-(trifluoromethyl)-4,5,6,7-tetrahydro-1*H*-indol-4-ol;
73) *N*-(((*S*)-1-(3-(difluoromethyl)-4-fluorophenyl)-5,5-difluoro-4-hydroxyl-4,5,6,7-tetrahydro-1*H-*indol-3-yl)(oxy)(trifluoromethyl)-16-sulfino)cyanamide;
74) *N*-((difluoromethyl)((*S*)-1-(3-(difluoromethyl)-4-fluorophenyl)-5,5-difluoro-4-hydroxyl-4,5,6,7-tetrahydro-1*H*-indol-3-yl)(oxo)-16-sulfino)cyanamide;
75) (*S*)-2-(5-(5,5-difluoro-4-hydroxyl-3-(trifluoromethyl)-4,5,6,7-tetrahydro-1*H*-indol-1-yl)-2-fluorophenyl)acetonitrile;
76) (*S*)-4-(5,5-difluoro-4-hydroxyl-3-(trifluoromethyl)-4,5,6,7-tetrahydro-1*H*-indol-1-yl)-2-(fluoromethyl)benzonitrile;
77) (*S*)-1-(3-(difluoromethyl)-4-fluorophenyl)-5,5-difluoro-3-((fluoromethyl)sulfonyl)-1,4,5,6-tetrahydrocyclopentadiene[*b*]pyrrol-4-ol;
78) (*S*)-1-(3-(difluoromethyl)-4-fluorophenyl)-5,5-difluoro-3-(methylsulfonyl)-1,4,5,6-tetrahydrocyclopentadiene[*b*] pyrrol-4-ol;
79) (*S*)-1-(3-(difluoromethyl)-4-fluorophenyl)-3-((difluoromethyl)sulfonyl)-5,5-difluoro-1,4,5,6-tetrahydrocyclopentadiene[*b*] pyrrol-4-ol;
80) (*S*)-1-(3-(difluoromethyl)-4-fluorophenyl)-5,5-difluoro-3-((trifluoromethyl)sulfonyl)-1,4,5,6-tetrahydrocyclopentadiene[*b*]pyrrol-4-ol;
81) (*S*)-5-(5,5-difluoro-4-hydroxyl-3-((trifluoromethyl)sulfonyl)-5,6-dihydrocyclopentadiene[*b*]pyrrol-1(4*H*)-yl)-yl2-fluorobenzonitrile;
82) (*S*)-3-(3-(difluoromethyl)-4-fluorophenyl)-7,7-difluoro-1-((trifluoromethyl)sulfonyl)-5,6,7,8-tetrahydroindol-8-ol;
83) (*S*)-3-(3-(difluoromethyl)-4-fluorophenyl)-7,7-difluoro-1-((trifluoromethyl)sulfonyl)-5,6,7,8-tetrahydroimidazo[1,5-*a*]pyridin-8 -ol;
84) (7*R*,8*S*)-3-(3-(difluoromethyl)-4-fluorophenyl)-7-fluoro-1-((trifluoromethyl)sulfonyl)-5,6,7,8-tetrahydroindolizin-8-ol;
85) 1-(3-(difluoromethyl)-4-fluorophenyl)-5,5-difluoro-3-((trifluoromethyl)sulfonyl)-1,4,5,7-tetrahydropyran[3,4-*b*]pyrrol-4-ol;
86) 2-(difluoromethyl)-4-((4*S*,5*R*)-3-((difluoromethyl)sulfonyl)-5-fluoro-4-hydroxyl-4,5,6,7-tetrahydro-1*H*-indol-1-yl)benzonitrile;
87) 2-(difluoromethyl)-4-((4*S*,5*R*)-5-fluoro-4-hydroxyl-3-((trifluoromethyl)sulfonyl)-4,5,6,7-tetrahydro-1*H*-indol-1-yl)benzonitrile;
88) (S)-5,5-difluoro-1-(imidazo[1,2-a]pyridin-8-yl)-3-(trifluoromethyl)-4,5,6,7-tetrahydro-1*H*-indol-4-ol;
89) (4*S*,5*R*)-1-(3-(difluoromethyl)-4-fluorophenyl)-5-fluoro-3-((trifluoromethyl)sulfonyl)-4,5,6,7-tetrahydro-1*H*-indol-4-ol;
90) (*S*)-8-(5,5-difluoro-3-((fluoromethyl)sulfonyl)-4-hydroxyl-4,5,6,7-tetrahydro-1*H*-indol-1-yl)- 5-fluoro-1-naphthonitrile;
91) (*S*)-3-((difluoromethyl)sulfonyl)-5,5-difluoro-1-(4-fluoro-5,6,7,8-tetrahydronaphthalen-1-yl)-4,5,6,7-tetrahydro-1*H*-indol-4-ol;
92) (*S*)-5,5-difluoro-1-(quinolin-8-yl)-3-((trifluoromethyl)sulfonyl)-4,5,6,7-tetrahydro-1*H*-indol-4-ol;
93) (4*S*)-1-(6,8-difluoro-1,2,3,4-tetrahydronaphthalen-1-yl)-5,5-difluoro-3-(trifluoromethyl)-4,5,6,7-tetrahydro-1*H*-indol-4-ol;
94) (4*S*)-1-(6,8-difluoro-1,2,3,4-tetrahydronaphthalen-1-yl)-5,5-difluoro-3-((trifluoromethyl)sulfonyl)- 4,5,6,7-tetrahydro-1*H*-indol-4-ol;
95) (4*S*)-1-(6,8-difluoro-1,2,3,4-tetrahydronaphthalen-1-yl)-3-((difluoromethyl)sulfonyl)-5,5-difluoro-4,5,6,7-tetrahydro-1*H*-indol-4-ol;
96) 1-(6,8-difluoro-1,2,3,4-tetrahydronaphthalen-1-yl)-5,5-difluoro-3-((fluoromethyl)sulfonyl)-4,5,6,7-tetrahydro-1*H*-indol-4-ol;
97) (4*S*)-1-(5,7difluoro-2,3-dihydro-1H-inden-1-yl)-5,5-difluoro-3-(trifluoromethyl)- 4,5,6,7-tetrahydro-1*H*-indol-4-ol;
98) (4*S*)-5,5-difluoro-1-(5,6,7,8-tetrahydroquinolin-8-yl)-3-(trifluoromethyl)- 4,5,6,7-tetrahydro-1*H-*indol-4-ol;
99) (4*S*)-5,5-difluoro-3-(methylsulfonyl)-1-(5,6,7,8-tetrahydroquinolin-8-yl)- 4,5,6,7-tetrahydro-1*H-*indol-4-ol;
100) 5,5-difluoro-3-((fluoromethyl)sulfonyl)-1-(5,6,7,8-tetrahydroquinolin-8-yl)- 4,5,6,7-tetrahydro-1*H*-indol-4-ol;
101) (4*S*)-3-((difluoromethyl)sulfonyl)-5,5-difluoro-1-(5,6,7,8-tetrahydroquinolin-8-yl)- 4,5,6,7-tetrahydro-1*H*-indol-4-ol;
102) (4*S*)-5,5-difluoro-1-(5,6,7,8-tetrahydroquinolin-8-yl)-3-((trifluoromethyl)sulfonyl)- 4,5,6,7-tetrahydro-1*H*-indol-4-ol;
103) 2-(difluoromethyl)-4-(5-fluoro-4-hydroxyl-3-((trifluoromethyl)thio)- 4,5,6,7-tetrahydro-1*H*-indol-1-yl)benzonitrile;
104) 2-(difluoromethyl)-4-(5-fluoro-4-hydroxyl-3-((trifluoromethyl)sulfonyl)- 4,5,6,7-tetrahydro-1*H-*indol-1-yl)benzonitrile;
105) 2-fluoro-5-(5-fluoro-4-hydroxyl-3-(trifluoromethyl)-4,7-dihydropyran[3,4-*b*]pyrrol-1(5*H*)-yl)benzonitrile;
106) 2-fluoro-5-(2,5,5-trifluoro-4-hydroxyl-3-(thien-2-yl)-4,5,6,7-tetrahydro-1*H*-indol-1-yl)benzonitrile; or
107) 2-(difluoromethyl)-4-(5-fluoro-4-hydroxyl-3-(trifluoromethyl)-4,5,6,7-tetrahydro-1*H*-indol-1-yl)benzonitrile.

The present invention further provides a method for preparing the compound. The compound as shown in formula (I) of the present invention or the compound as described in the specific examples can be prepared using commercially available starting materials, compounds known in the literature, or easily prepared intermediates by adopting organic synthesis techniques known to those skilled in the art or known to those skilled in the art based on the contents described herein, and can be obtained by a preparation method similar to the specific embodiments.

Specifically, in another embodiment, the present invention relates to a method for preparing a compound as shown in general formula (IX-1), comprising reacting the compound as shown in formula (A-1) with a reducing agent, 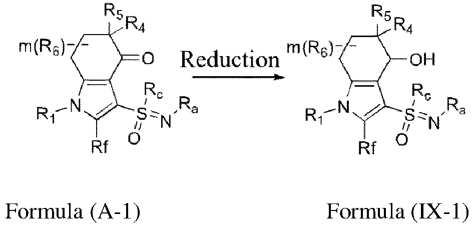 wherein R₁, R₄, R₅, R₆, R_{f}, R_{c}, Rₐ and m are as defined in the above embodiments.

In another embodiment, the present invention relates to a method for preparing a compound as shown in general formula (IX-2), comprising reacting the compound as shown in formula (A-2) with a reducing agent, 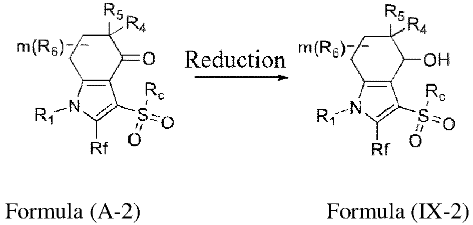 wherein R₁, R₄, R₅, R₆, R_{f}, R_{c}, Rₐ and m are as defined in the above embodiments; further, R₁ is 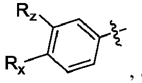 and Rₓ and R_{z} are as defined in the above embodiments;
the reducing agent can be a conventional reducing agent such as sodium borohydride and lithium borohydride; the chiral monomers corresponding to the hydroxyl positions of the backbone structures of the compounds of formula (IX-1) and formula (IX-2) can be obtained by chiral reduction (e.g., Noyori asymmetric reduction and CBS asymmetric reduction) of the compounds of formula (A-1) and formula (A-2).

In some embodiments, the reducing agent used in the chiral reduction is [(R,R)-Ts-DPEN]RuCl(p-cymene)].

In some other embodiments, the compound of formula (A-1) can be prepared by the following method, wherein R₁, R₄, R₅, R₆, R_{f}, R_{c}, Rₐ and m are as defined in the above embodiments, 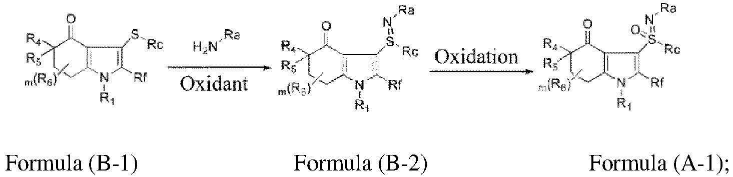 wherein the compound as shown in formula (B-1) can be reacted with NH₂-Rₐ (such as cyanamide) in the presence of an oxidant (such as iodobenzene diacetate) to obtain a compound as shown in formula (B-2), which is oxidized to obtain the compound as shown in formula (A-1).

In some other embodiments, the compound as shown in formula (A-1) can be prepared by the following method, wherein R₁, R₄, R₅, R₆, R_{f}, R_{c}, Rₐ and m are as defined in the above embodiments, 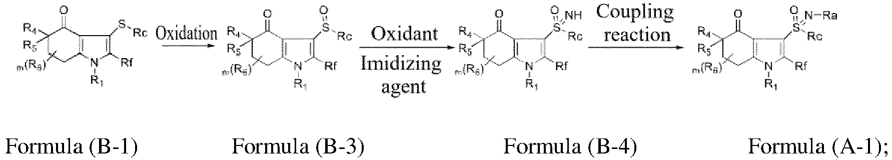 wherein the compound as shown in formula (B-1) is subjected to an oxidation reaction (such as meta-chloroperbenzoic acid and potassium peroxymonosulfonate) to obtain the compound as shown in formula (B-3), which is reacted with an imidizing agent (such as ammonium carbamate and trifluoroacetamide) in the presence of an oxidant (such as iodobenzene diacetate) to obtain the compound as shown in formula (B-4), into which Ra can be introduced by alkylation (such as iodomethane and iodoethane), or by coupling reaction (such as with cuprous cyanide) to obtain the compound as shown in formula (A-1). In some other embodiments, the compound as shown in formula (A-2) can be prepared by the following method, 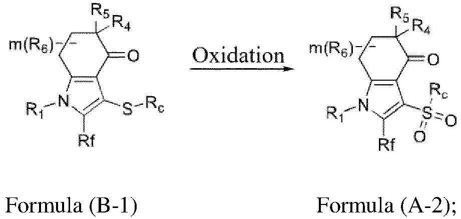 wherein R₁, R₄, R₅, R₆, R_{f}, R_{c} and m are as defined in the above embodiments, and further, R₁ is 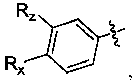 and Rₓ and R_{z} are as defined in the above embodiments.

The compound as shown in formula (B-1) can be subjected to an oxidation reaction to obtain the compound as shown in formula (A-2), wherein the oxidant includes m-chloroperbenzoic acid, potassium peroxymonosulfonate, ruthenium trichloride/sodium periodate system, ruthenium trichloride/sodium hypochlorite system, etc.

In some other embodiments, the compound as shown in formula (A-2) can be prepared by the following method, wherein R₁, R₄, R₅, R₆, R_{f}, R_{c} and m are as defined in the above embodiments, and X is halogen; 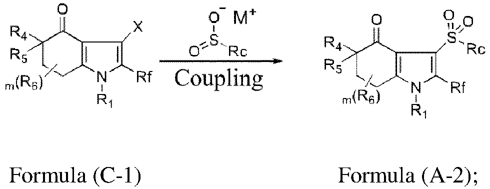 wherein the compound as shown in formula (A-2) can be prepared by coupling the compound as shown in formula (C-1) with the corresponding sulfinate (such as sodium methylsulfinate) under metal catalysis (such as cuprous salt).

In some other embodiments, the compound as shown in formula (A-2) can be prepared by the following method, wherein R₁, R₄, R₅, R₆, R_{f}, R_{c} and m are as defined in the above embodiments, and X is halogen; 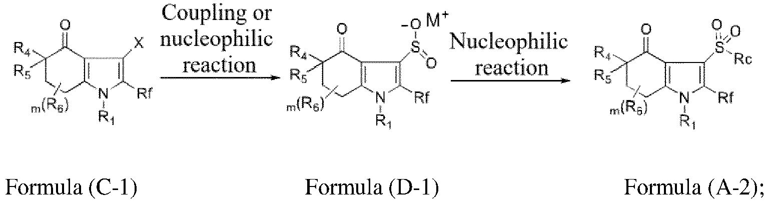 wherein a sulfinic acid group can be introduced into a compound as shown in formula (C-1) by a coupling reaction to obtain a compound as shown in formula (D-1), or a sulfinic acid group can be introduced by transforming the halogen atom X into a negative ion and reacting same with the sulfur dioxide equivalent (such as potassium pyrosulfite and 1,4-diazabicyclo[2.2.2]octane-1,4-diium-1,4-disulfinate) that generates sulfur dioxide in situ to obtain a compound as shown in formula (D-1); the compound as shown in formula (D-1) can be subjected to a nucleophilic reaction with an electrophilic agent (such as Togni's reagent, iodomethane, and trifluoroiodomethane) to prepare the compound as shown in formula (A-2), wherein M⁺ is any monovalent positive ion that can form a salt, such as Na⁺, K⁺ and MgBr⁺.

In some other embodiments, the compound as shown in formula (B-1) can be prepared by the following method, 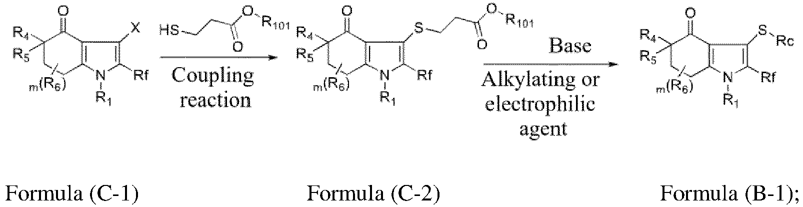 wherein R₁, R₄, R₅, R₆, R_{f}, R_{c} and m are as defined in the above embodiments, X is halogen, and R₁₀₁ is C₁₋₁₀ alkyl; further, R₁ is 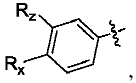 and Rₓ and R_{z} are as defined in the above embodiments;
the compound as shown in formula (C-1) can be coupled with an alkyl mercaptopropionate (such as methyl mercaptopropionate, ethyl mercaptopropionate, and tert-butyl mercaptopropionate) to obtain the compound as shown in formula (C-2), which is reacted with a corresponding alkylating or electrophilic agent (such as iodomethane, fluoroiodomethane, trifluoroiodomethane, bromoacetonitrile, and diethyl bromofluoromethylphosphate) to introduce the corresponding Rc group on the sulfur atom under alkaline conditions (potassium carbonate, cesium carbonate, potassium phosphate, potassium tert-butoxide, etc.) to prepare the compound as shown in formula (B-1).

In some other embodiments, the compound as shown in formula (B-1) can be prepared by the following method, wherein R₁, R₄, R₅, R₆, R_{f}, R_{c} and m are as defined in the above embodiments, and X is halogen; 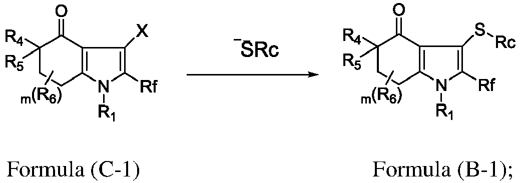 wherein the compound as shown in formula (C-1) can be subjected to a coupling reaction with a nucleophilic sulfur agent ⁻SRc (such as silver trifluoromethanethiol and copper trifluoromethanethiol) to obtain the compound as shown in formula (B-1).

In some other embodiments, the compound as shown in formula (C-1) can be prepared by the following method, wherein R₁, R₄, R₅, R₆, R_{f} and m are as defined in the above embodiments, X is halogen, X₁ is -TIPS, -Trt or -TBDPS, and R₁₀₂ is H or C₁₋₃ alkyl, or two R₁₀₂ together with the O atoms to which they are attached form a 5-membered heterocycle containing two oxygen atoms and one boron atom, and the 5-membered heterocycle can be optionally substituted with one or more C₁₋₃ alkyl; 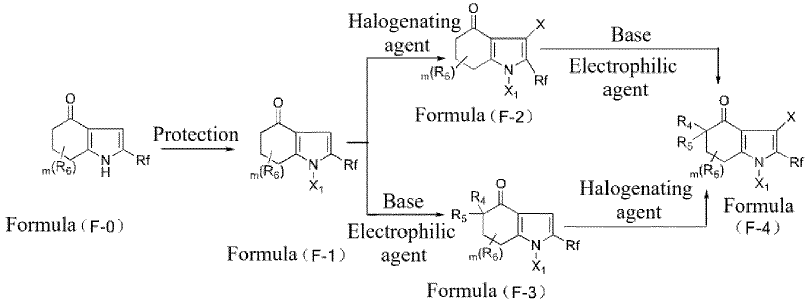 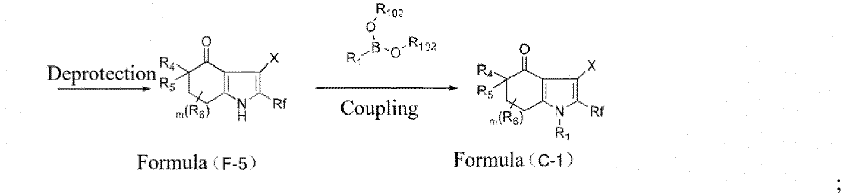 wherein the compound as shown in formula (F-0) can be purchased through commercial channels (for example, 1,5,6,7-tetrahydro-4H-indol-4-one), which is subjected to N atom protection under alkaline conditions to obtain the compound as shown in formula (F-1); the compound as shown in formula (F-1) is reacted with an electrophilic halogenating agent (such as NIS) to introduce a halogen atom at the 3-position of pyrrole to obtain the compound as shown in formula (F-2), which is deprived of the proton ortho to the carbonyl group to introduce R4 and R5 groups by reacting with an electrophilic agent (such as NFSI and Selectfluor) under alkaline conditions (such as LiHDMS and LDA) to obtain the compound as shown in formula (F-4); the compound as shown in formula (F-4) is deprotected to obtain the compound as shown in formula (F-5), which is then subjected to a coupling reaction to obtain the compound as shown in formula (C-1);
alternatively, the compound as shown in formula (F-1) can be first reacted with an electrophilic agent to introduce R₄ and R₅ groups at the position ortho to the carbonyl group under alkaline conditions to obtain the compound as shown in formula (F-3), which is then reacted with a corresponding electrophilic halogenating agent to introduce a halogen atom at the 3-position of pyrrole to obtain the compound as shown in formula (F-4).

In some other embodiments, the compound as shown in formula (B-1) can be prepared by the following method, wherein R₁, R₄, R₅, R₆, R_{f}, R_{c} and m are as defined in the above embodiments, X₁ is - TIPS, -Trt or -TBDPS, and R₁₀₂ is H or C₁₋₃ alkyl, or two R₁₀₂ together with the O atoms to which they are attached form a 5-membered heterocycle containing two oxygen atoms and one boron atom, and the 5-membered heterocycle can be optionally substituted with one or more C₁₋₃ alkyl; 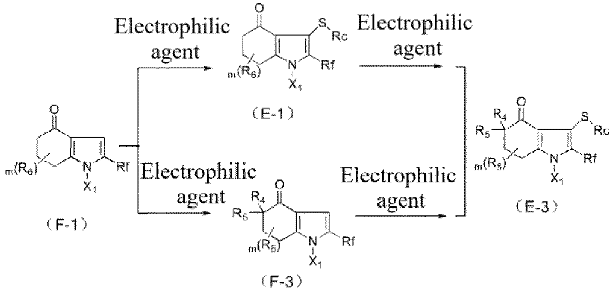 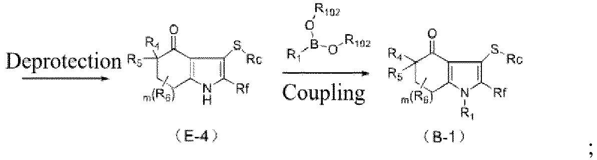 wherein the compound as shown in formula (F-1) is subjected to an electrophilic substitution reaction with an electrophilic agent (such as Shen reagent and N-(trifluoromethylthio)phthalimide) at the 3-position of pyrrole to prepare the compound as shown in formula (E-1), which is then deprived of the proton ortho to the carbonyl group to introduce R₄ and R₅ groups under alkaline conditions to obtain the compound as shown in formula (E-3); the compound as shown in formula (E-3) is deprotected to obtain the compound as shown in formula (E-4), which is then subjected to a coupling reaction to obtain the compound as shown in formula (B-1);
alternatively, the compound as shown in formula (F-1) can be first deprived of the proton ortho to the carbonyl group to introduce R₄ and R₅ groups under alkaline conditions to obtain the compound as shown in formula (F-3), which is then subjected to an electrophilic substitution reaction under alkaline conditions to obtain the compound as shown in formula (E-3).

In some other embodiments, the compound as shown in formula (A-2) can be prepared by the following method, wherein R₁, R₄, R₅, R₆, R_{f}, R_{c} and m are as defined in the above embodiments, X₁ is - TIPS, -Trt or -TBDPS, and R₁₀₂ is H or C₁₋₃ alkyl, or two R₁₀₂ together with the O atoms to which they are attached form a 5-membered heterocycle containing two oxygen atoms and one boron atom, and the 5-membered heterocycle can be optionally substituted with one or more C₁₋₃ alkyl; 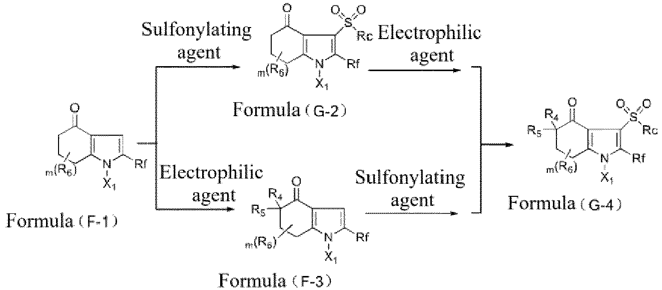 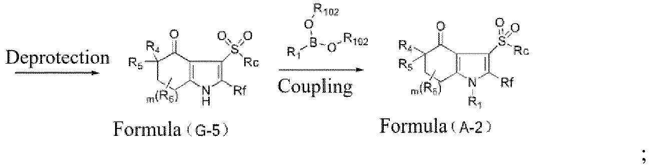 wherein the compound as shown in formula (F-1) is subjected to an electrophilic substitution reaction with a sulfonylating agent (such as trifluoromethanesulfonic anhydride) to introduce a sulfonyl group to obtain the compound as shown in formula (G-2), which is then deprived of the proton ortho to the carbonyl group to introduce R₄ and R₅ groups under alkaline conditions to obtain the compound as shown in formula (G-4); the compound as shown in formula (G-4) is deprotected to obtain the compound as shown in formula (G-5), which is then subjected to a coupling reaction to obtain the compound as shown in formula (A-2);
alternatively, the compound as shown in formula (F-1) can be first deprived of the proton ortho to the carbonyl group to introduce R₄ and R₅ groups under alkaline conditions to obtain the compound as shown in formula (F-3), which is then subjected to an electrophilic substitution reaction with a sulfonylating agent (such as trifluoromethanesulfonic anhydride) to introduce a sulfonyl group under alkaline conditions to obtain the compound as shown in formula (G-4).

In some other embodiments, the compound as shown in formula (G-5) can be prepared by the following method, wherein R₄, R₅, R₆, R_{f}, R_{c} and m are as defined in the above embodiments, X is halogen, and X₂ is -Boc or benzenesulfonyl; 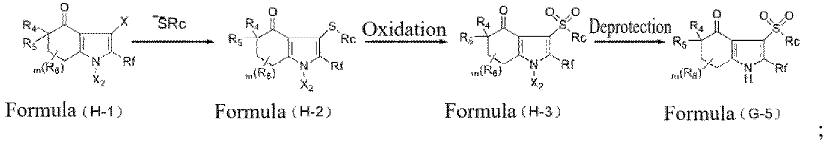 wherein the compound as shown in formula (H-1) can be obtained by subjecting the compound as shown in formula (F-0) to N atom protection and then to a halogenation reaction; the compound as shown in formula (H-1) is subjected to a coupling reaction with a nucleophilic sulfur agent ⁻SRc (such as silver trifluoromethanethiol and copper trifluoromethanethiol) to obtain the compound as shown in formula (H-2); the compound as shown in formula (H-2) is subjected to an oxidation reaction to obtain the compound as shown in formula (H-3), which is then deprotected to finally obtain the compound as shown in formula (G-5).

In another aspect, the present invention further provides an intermediate for preparing the compounds as shown in formula (IX-1) and formula (IX-2), which is selected from the following structures: 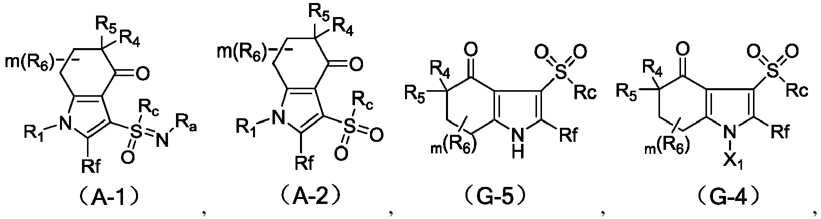 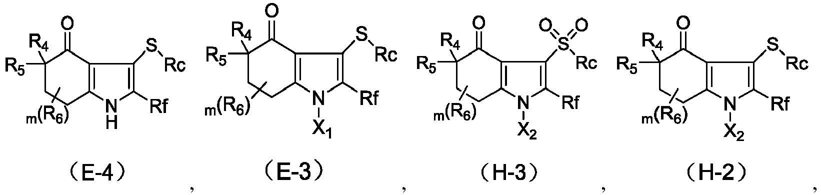 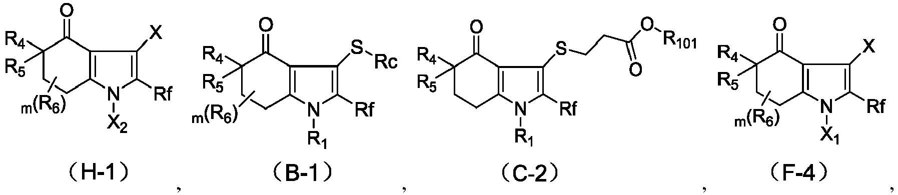 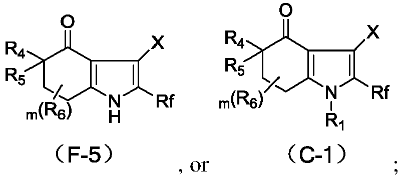 wherein R₁, R₄, R₅, R₆, R_{f}, R_{c}, Rₐ and m are as defined in the above embodiments, X is halogen, X₁ is selected from -TIPS, -Trt or -TBDPS, X₂ is -Boc or benzenesulfonyl, and R₁₀₁ is C₁₋₃ alkyl.

Further, the present invention further provides an intermediate for preparing the compounds as shown in formula (XI-1) and formula (XI-2), which is selected from the following structures: 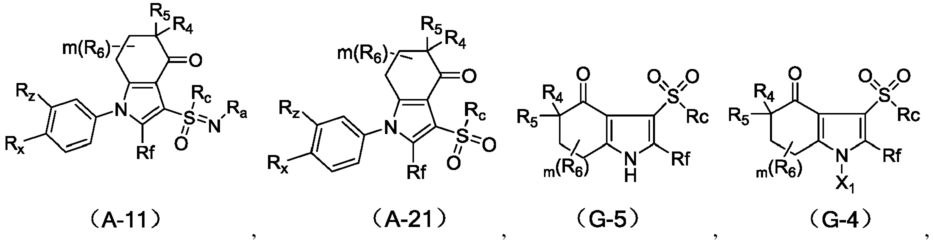 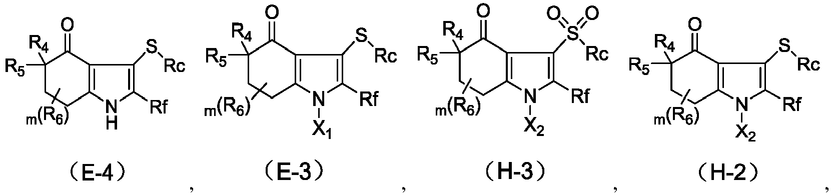 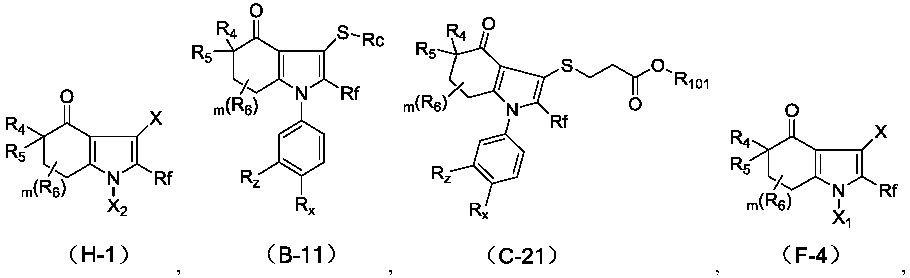 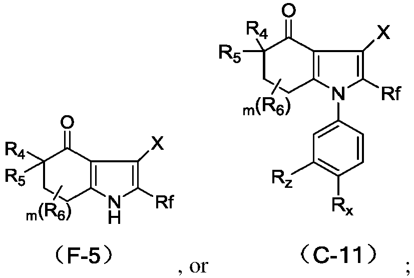 wherein R₁, R₄, R₅, R₆, R_{f}, R_{c}, Rₐ, Rₓ, R_{z} and m are as defined in the above embodiments, X is halogen, X₁ is selected from TIPS- or Trt-, X₂ is -Boc, and R₁₀₁ is C₁₋₁₀ alkyl.

The present invention further provides a pharmaceutical composition comprising a therapeutically effective amount of at least one of the above compounds and a pharmaceutically acceptable excipient, such as hydroxypropyl methylcellulose. In some compositions, the weight ratio of the compound to the excipient is about 0.001-10.

In addition, the present invention further provides a method for treating a subject suffering from a HIF-2α-mediated disease or disorder, comprising administering a therapeutically effective amount of the compound of formula (I) or the pharmaceutically acceptable salt thereof.

In certain aspects, the disease or disorder is selected from VHL syndrome, an autoimmune disease, cancer, an inflammatory disease, a neurodegenerative disease, a cardiovascular disorder, a renal disorder, viral infection, and obesity.

In certain aspects, the disease or disorder is selected from rheumatoid arthritis, osteoarthritis, atherosclerosis, psoriasis, systemic lupus erythematosus, multiple sclerosis, inflammatory bowel disease, asthma, chronic obstructive airway disease, pneumonia, dermatitis, alopecia, nephritis, vasculitis, atherosclerosis, Alzheimer's disease, hepatitis, primary biliary cirrhosis, sclerosing cholangitis, diabetes (including type I diabetes), and acute rejection of a transplanted organ.

In certain aspects, the disease or disorder is cancer selected from solid malignancies and hematological tumors.

In certain aspects, the cancer includes, but is not limited to, bone cancer, pancreatic cancer, skin cancer, head and neck cancer, malignant melanoma, uterine cancer, ovarian cancer, rectal cancer, gastric cancer, uterine cancer, cervical cancer, endometrial cancer, lymphoma, esophageal cancer, colon cancer, rectal cancer, thyroid cancer, prostate cancer, sarcoma, leukemia, bladder cancer, kidney cancer, glioma, epidermal cystadenoma, squamous cell carcinoma, pheochromocytoma, lung cancer, pancreatic cancer, liver cancer, kidney cancer, breast cancer, exothelioma, neurocytoma, paraganglioma, blastoma, endocrine tumor, meningioma and medulloblastoma.

In certain aspects, the disease or disorder is VHL syndrome.

In certain aspects, the disease or disorder is kidney cancer; still further, the disease is renal cell carcinoma; the disease is clear cell renal cell carcinoma.

In certain aspects, the subject is a human.

In certain aspects, the compound is administered intravenously, intramuscularly, parenterally, nasally, or orally. In one aspect, the compound is administered orally.

The present invention further provides the use of the compound of formula (I) or the pharmaceutically acceptable salt thereof in the preparation of a medicament for treating a HIF-2α-mediated disease or disorder.

The present invention further provides a compound as shown in formula (I) or a pharmaceutically acceptable salt thereof for use in treatment. The present invention further provides a compound of formula (I) or a pharmaceutically acceptable salt thereof for treating a subject suffering from a HIF-2α-mediated disease or disorder.

The present invention may be implemented in other specific forms without departing from its spirit or essential attributes. The present invention encompasses all combinations of preferred aspects of the present invention mentioned herein. It should be understood that the subject matter disclosed in the Summary of the Invention does not exhaustively or completely disclose the full scope of the present technical solutions or any embodiments thereof, and that any and all embodiments of the present invention may be employed in conjunction with any other embodiment or embodiments to describe an additional embodiment. It is also to be understood that each individual element of the embodiments is its own independent embodiment. Furthermore, any element of an embodiment is meant to be combined with any and all other elements from any embodiment to describe an additional embodiment.

In the present invention, unless otherwise specified, the term "halogen" refers to fluorine, chlorine, bromine or iodine. Preferred halogen groups are fluorine, chlorine and bromine.

In the present invention, unless otherwise specified, the term "alkyl" includes a linear or branched saturated monovalent hydrocarbon group. For example, alkyl includes methyl, ethyl, propyl, isopropyl, cyclopropyl, n-butyl, isobutyl, sec-butyl, tert-butyl, cyclobutyl, n-pentyl, 3-(2-methyl)butyl, 2-pentyl, 2-methylbutyl, neopentyl, cyclopentyl, n-hexyl, 2-hexyl, 2-methylpentyl, and cyclohexyl. Similarly, the "C₁-C₆" in the C₁-C₆ alkyl refers to a group containing 1, 2, 3, 4, 5 or 6 carbon atoms arranged in the form of a straight or branched chain.

The term "alkoxy" refers to an oxygen ether formed from the above-mentioned straight, branched or cyclic alkyl groups.

The term "alkylene" refers to a divalent alkyl linking group. Alkylene formally refers to an alkane with two C-H bonds replaced with the point by which the alkylene is attached to the rest of the compound. Similarly, the "C₁-C₄" in the C₁-C₄ alkylene refers to a alkylene group containing 1, 2, 3 or 4 carbon atoms.

The term "haloalkyl" refers to an alkyl group in which one or more H atoms have been replaced with halogen atom(s). The term "haloalkoxy" refers to -O-haloalkyl group.

The term "oxo" or "oxo group" refers to an oxygen atom in the form of a dimethyl substituent, which forms a carbonyl group when attached to C, and forms a sulfoxyl or sulfonyl group or an N-oxide group when attached to a heteroatom.

In the present invention, unless otherwise specified, the term "aromatic ring" or "aromatic heterocycle" refers to a polyunsaturated carbon ring or heterocycle with aromatic characteristics (having (4n+2) delocalized π electrons, where n is an integer).

The term "aryl" refers to substituted or unsubstituted stable aromatic hydrocarbon group of 6 to 10 ring carbon atoms, which may contain one aromatic ring or multiple aromatic rings (e.g., fused bicyclic rings). The aromatic ring contains no heteroatoms. Examples of aryl include, but are not limited to, phenyl, naphthyl, indenyl, and the like.

The term "heteroaryl" refers to a monocyclic or polycyclic (e.g., fused bicyclic) aromatic heterocycle having at least one heteroatom ring member selected from N, O and/or S. The "5- to 18-membered" in 5- to 18-membered heteroaryl refers to a heteroaryl group containing 5 to 18 carbon atoms or N, O or S as a ring-forming atom. Examples of such heteroaryl groups include, but are not limited to, pyridyl, pyrimidyl, pyrrolyl, imidazolyl, thiazolyl, thienyl, benzimidazole, benzothiophenyl, benzofuranyl, etc.

The term "cycloalkyl" refers to a ring system having at least one cyclized alkyl group. "C₃-C₁₀" in the term C₃-C₁₀ cycloalkyl refers to a cycloalkyl group which may have 3, 4, 5, 6, 7, 8, 9 or 10 ring-forming atoms. Cycloalkyl may include monocyclic and polycyclic rings (e.g., having 2, 3 or 4 rings which may exist in the form of a spiro ring, a fused ring, etc.). In some embodiments, cycloalkyl includes, but is not limited to, cyclopropyl, cyclobutyl, cyclopentyl, etc. In some embodiments, cycloalkyl further includes moieties having one or more aromatic rings fused to a cyclized alkyl ring, such as benzo or thienyl derivatives of cyclohexane.

The term "cycloalkenyl" refers to a ring system having at least one cyclized alkenyl group, wherein the cycloalkenyl has one or more carbon-carbon double bonds. "C₃-C₁₀" in the term C₃-C₁₀ cycloalkenyl refers to a cycloalkenyl group which may have 3, 4, 5, 6, 7, 8, 9 or 10 ring-forming atoms. Cycloalkenyl may include monocyclic and polycyclic rings (e.g., having 2, 3, or 4 rings which may exist in the form of a spiro ring, a bridged ring, etc.). In some embodiments, cycloalkenyl includes, but is not limited to, cyclohexenyl, cyclohexadiene, cycloheptatrienyl, etc. In some embodiments, cycloalkenyl further includes moieties having one or more aromatic rings fused to a cyclized alkenyl ring, such as benzo or thienyl derivatives of a cyclohexene ring, etc.

The term "heterocyclyl" refers to a cyclic system having at least one cyclized alkyl group or cyclized alkenyl group containing heteroatoms selected from N, O, and/or S. The heterocyclyl may include monocyclic or polycyclic rings (e.g., having 2, 3, or 4 rings which may exist in the form of a spiro ring, a bridged ring, etc.). Heterocyclyl may be connected to other parts of the compound through a ring-forming carbon atom or a ring-forming heteroatom. The definition of heterocyclyl further includes moieties having one or more aromatic rings fused with cyclized alkyl or cyclized alkenyl rings, such as benzo or thienyl derivatives of pyridine, morpholine, etc. In some embodiments, heterocyclyl includes, but is not limited to, pyrrolidinyl, pyrrolinyl, tetrahydrothienyl, tetrahydrofuranyl, piperidinyl, morpholinyl, azepane, dihydrobenzofuranyl, etc.

As used herein, the term "composition" is intended to encompass a product comprising the specified components in the specified amounts, as well as any product which results, directly or indirectly, from the specified components in the specified amounts. Therefore, pharmaceutical compositions comprising the compounds of the present invention as active components and methods for preparing the compounds are also disclosed in the present invention. Furthermore, some compounds may exist in polymorphic forms, which are also included in the present invention. In addition, some of the compounds form solvates with water (e.g., hydrates) or common organic solvents, and these solvates are also included in the present invention.

The "compound of the present invention" includes the compound of formula (I) and all pharmaceutically acceptable forms thereof. These pharmaceutically acceptable forms include salts, solvates, non-covalent complexes, chelates, stereoisomers (including diastereomers, enantiomers and racemates), geometric isomers, isotopically labeled compounds, tautomers, prodrugs, or any mixtures of all of the above.

The "enantiomers" are a pair of non-superimposable, mirror-image stereoisomers, and a 1 : 1 mixture of a pair of enantiomers is a "racemic" mixture. When specifying the stereochemistry of the compound of the invention, the conventional RS system is used (e.g., (1S,2S) specifies a single stereoisomer of known relative and absolute configurations having two chiral centers). Optically active (R)- and (S)-isomers may be prepared synthetically using optically active raw materials or chiral reagents, or resolved using conventional techniques (e.g., separation on a chiral SFC or HPLC column).

"Diastereomers" are stereoisomers that have at least two asymmetric atoms, but which are not mirror images of each other. When the compound is enantiomerically pure, the stereochemistry at each chiral carbon can be specified by either R or S.

Resolved compounds of unknown absolute configuration can be named (+) or (-) according to the direction in which they rotate plane polarized light (right-handed or left-handed) at the wavelength of the sodium D line. Alternatively, the resolved compounds can be defined by the corresponding retention times of the corresponding enantiomers/diastereoisomers by chiral HPLC.

Those skilled in the art will recognize that the compounds of the present invention may contain one or more chiral centers and may thereby give rise to diastereomers and optical isomers. The above formula (I) does not define the stereostructure of the compound at a certain position, and therefore the compound may exist in different isomeric forms. Unless otherwise specified, the present invention includes all possible stereoisomers of the compound as shown in formula (I) and pharmaceutically acceptable salts thereof, such as racemic mixtures, optically pure forms and isomer mixtures in any ratio. Further, mixtures of stereoisomers as well as isolated specific stereoisomers are also included in the present invention. During the synthetic process for preparing such compounds, or racemization or epimerization known to those skilled in the art, the products obtained may be mixtures of stereoisomers.

The drug precursors (prodrugs) of the compounds of the present invention are included in the scope of protection of the present invention. Generally, the drug precursor refers to a functional derivative that is readily converted into the desired compound in vivo. Therefore, the term "administering" in the treatment methods provided by the present invention includes administering the compounds disclosed in the present invention, or the prodrug compounds which are not explicitly disclosed, but can be converted into the compounds disclosed in the present invention in vivo after administration to the subject. Conventional methods for selecting and preparing suitable prodrug derivatives are described in a book such as Design of Prodrugs, ed. H. Bundgaard, Elsevier, 1985.

Obviously, the definition of any substituent or a variable at a particular position in a molecule is independent of its definitions at other positions in the molecule. It is easy to understand that a person skilled in the art can select substituents or substitution forms of the compounds of the present invention by means of existing techniques and the methods described in the present invention to provide chemically stable and easily synthesized compounds.

Obviously, the definition of any substituent or variable at a particular position in one molecule is independent of the definition of any substituent or variable at a particular position in any other molecule. It is easy to understand that, for the compounds of the present invention, suitable substituents or substitution forms can be selected according to the existing techniques in this subject to provide a compound which is chemically stable and easily prepared and synthesized using the existing techniques in this subject or the methods described in the present invention.

When the compound as shown in formula (I) and the pharmaceutically acceptable salt thereof are in the form of a solvate or polymorph, the present invention includes any possible solvates and polymorphs. The type of solvent forming the solvate is not particularly limited as long as the solvent is pharmacologically acceptable. For example, solvents, such as water, ethanol, propanol, and acetone, may be used.

The term "pharmaceutically acceptable salts" refers to salts prepared from pharmaceutically acceptable non-toxic bases or acids. When the compound provided by the present invention is an acid, its corresponding salt can be conveniently prepared from pharmaceutically acceptable non-toxic bases, including inorganic bases and organic bases. Salts derived from inorganic bases include aluminum, ammonium, calcium, copper (supervalent and subvalent), ferric, ferrous, lithium, magnesium, manganese (supervalent and subvalent), potassium, sodium, zinc and the like. Particularly preferred are the ammonium, calcium, magnesium, potassium and sodium salts. Non-toxic organic bases from which pharmaceutically acceptable salts can be derived include primary, secondary, and tertiary amines, including cyclic amines and substituent-containing amines, such as naturally occurring and synthetic substituent-containing amines. Other pharmaceutically acceptable non-toxic organic bases capable of forming salts include ion exchange resins and arginine, betaine, caffeine, choline, N',N'-dibenzylethylenediamine, diethylamine, 2-diethylaminoethanol, 2-dimethylaminoethanol, ethanolamine, ethylenediamine, N-ethylmorpholine, N-ethylpiperidine, reduced glucosamine, glucosamine, histidine, hydrabamine, isopropylamine, lysine, methylglucamine, morpholine, piperazine, piperidine, polyamine resins, procaine, chloroprocaine, purine, theobromine, triethylamine, trimethylamine, tripropylamine, tromethamine, and the like.

When the compound provided by the present invention is a base, its corresponding salt can be conveniently prepared from pharmaceutically acceptable non-toxic acids, including inorganic acids and organic acids. Such acids include, for example, acetic acid, benzenesulfonic acid, benzoic acid, camphorsulfonic acid, citric acid, ethanesulfonic acid, formic acid, fumaric acid, gluconic acid, glutamic acid, hydrobromic acid, hydrochloric acid, isethionic acid, lactic acid, maleic acid, malic acid, mandelic acid, methanesulfonic acid, mucic acid, nitric acid, pamoic acid, pantothenic acid, phosphoric acid, succinic acid, sulfuric acid, oxalic acid, propionic acid, glycolic acid, hydroiodic acid, perchloric acid, cyclamic acid, salicylic acid, 2-naphthalenesulfonic acid, saccharinic acid, trifluoroacetic acid, tartaric acid, p-toluenesulfonic acid, etc; preferably, citric acid, hydrobromic acid, formic acid, hydrochloric acid, maleic acid, phosphoric acid, sulfuric acid and tartaric acid; more preferably, formic acid and hydrochloric acid. Since the compound as shown in formula (I) is to be used as a medicament, it is preferably used in substantially pure form, for example at least 60% pure, more suitably at least 75% pure, particularly at least 98% pure (% represents weight ratio).

The pharmaceutical composition provided by the present invention comprises a compound as shown in formula (I) (or a pharmaceutically acceptable salt thereof) as an active component, a pharmaceutically acceptable excipient and other optional therapeutic components or excipients. Although the most suitable mode of administration of the active component in any given case depends on the specific subject to be administered, the nature of the subject and the severity of the condition, the pharmaceutical compositions of the present invention include those suitable for oral, rectal, topical and parenteral (including subcutaneous administration, intramuscular injection, and intravenous administration) administration. The pharmaceutical compositions of the present invention may be conveniently presented in unit dosage form well known in the art and prepared by any methods well known in the art of pharmacology.

In fact, according to conventional drug mixing techniques, the compound as shown in formula (I) of the present invention, or a drug precursor, or a metabolite, or a pharmaceutically acceptable salt, can be used as an active component and mixed with a drug carrier to form a pharmaceutical composition. The pharmaceutical carrier may take a wide variety of forms depending on the desired mode of administration, for example, oral administration or injection (including intravenous injection). Thus, the pharmaceutical compositions of the present invention can be presented as discrete units suitable for oral administration such as capsules, cachets or tablets containing a predetermined dose of the active component. Further, the pharmaceutical composition of the present invention may be in the form of a powder, granule, solution, aqueous suspension, non-aqueous liquid, oil-in-water emulsion, or water-in-oil emulsion. Furthermore, in addition to the common dosage forms mentioned above, the compound as shown in formula (I) or the pharmaceutically acceptable salt thereof can also be administered via a controlled release method and/or a delivery device. The pharmaceutical composition of the present invention can be prepared by any pharmaceutical method. In general, such methods include the step of bringing into association the active component with the carrier which constitutes one or more necessary ingredients. In general, the pharmaceutical compositions are prepared by uniformly and thoroughly admixing the active component with liquid carriers or finely divided solid carriers or a mixture of both. The product can then be conveniently prepared into the desired presentation.

Therefore, the pharmaceutical composition of the present invention comprises a pharmaceutically acceptable carrier and a compound as shown in formula (I) or a pharmaceutically acceptable salt thereof. The compound as shown in formula (I) or a pharmaceutically acceptable salt thereof, together with one or more other therapeutically active compounds, is also included in the pharmaceutical composition of the present invention.

The drug carrier used in the present invention may be, for example, a solid carrier, a liquid carrier or a gaseous carrier. Solid carriers include lactose, gypsum powder, sucrose, talc, gelatin, agar, pectin, gum arabic, magnesium stearate, and stearic acid. Liquid carriers include syrup, peanut oil, olive oil and water. Gaseous carriers include carbon dioxide and nitrogen. For preparing oral pharmaceutical preparations, any convenient pharmaceutical media may be employed. For example, water, glycols, oils, alcohols, flavor enhancers, preservatives, coloring agents, etc. can be used in oral liquid preparations such as suspensions, elixirs, and solutions; carriers such as starches, sugars, microcrystalline cellulose, diluents, granulating agents, lubricants, binders, disintegrants, etc. can be used in oral solid preparations such as powders, capsules and tablets. Considering the ease of administration, tablets and capsules are preferred for oral preparations, and accordingly solid pharmaceutical carriers are used. Alternatively, tablets may be coated using standard aqueous or nonaqueous preparation techniques.

Tablets containing the compounds or pharmaceutical compositions of the present invention can be prepared by compressing or molding, optionally together with one or more auxiliary components or adjuvants. Compressed tablets can be prepared by compressing in a suitable machine the active component in a free-flowing form such as powder or granules mixed with a binder, lubricant, inert diluent, surfactant or dispersing agent. Molded tablets may be made by impregnating the powdered compound or pharmaceutical composition with an inert liquid diluent and then molding same in a suitable machine. Preferably, each tablet contains from about 0.05 mg to 5 g of the active component and each cachet or capsule contains from about 0.05 mg to 5 g of the active component. For example, a dosage form intended for oral administration to humans contains from about 0.5 mg to about 5 g of the active component, compounded with suitable and conveniently metered auxiliary materials, which account for about 5% to 95% of the total pharmaceutical composition. Dosage unit forms generally contain from about 1 mg to about 2 g of the active component, typically 25 mg, 50 mg, 100 mg, 200 mg, 300 mg, 400 mg, 500 mg, 600 mg, 800 mg or 1000 mg.

The pharmaceutical composition suitable for parenteral administration provided by the present invention can be prepared into an aqueous solution or suspension by adding active components into water. A suitable surfactant such as hydroxypropylcellulose may be included. Dispersion systems can also be prepared in glycerol, liquid polyethylene glycols and mixtures thereof in oils. Further, a preservative may also be included in the pharmaceutical compositions of the present invention to prevent the growth of harmful microorganisms.

The present invention provides pharmaceutical compositions suitable for injection, including sterile aqueous solutions or dispersion systems. Further, the above pharmaceutical composition can be prepared in the form of sterile powders for the immediate preparation of sterile injections or dispersions. Regardless, the final injection form must be sterile and must be flowable for ease of injection. Furthermore, the pharmaceutical composition must be stable during manufacture and storage. Therefore, preservation against contamination by microorganisms such as bacteria and fungi is preferred. The carrier can be a solvent or dispersion medium, for example, water, ethanol, polyol (e.g., glycerol, propylene glycol, liquid polyethylene glycol), vegetable oils, and suitable mixtures thereof.

The pharmaceutical composition provided by the present invention may be in a form suitable for topical administration, for example, an aerosol, emulsion, ointment, lotion, dusting powder or other similar dosage forms. Further, the pharmaceutical compositions provided by the present invention may be in a form suitable for use with a transdermal administration device. These preparations can be prepared using the compound as shown in formula (I) of the present invention, or a pharmaceutically acceptable salt thereof, by conventional processing methods. As an example, the emulsion or ointment is prepared by adding a hydrophilic material and water to the above compound in the range of about 5 wt% to 10 wt% to prepare an emulsion or ointment having a desired consistency.

The pharmaceutical composition provided by the present invention can be prepared in a form which is suitable for rectal administration with a solid carrier. The preferred dosage form is a suppository in a unit dose formed as a mixture. Suitable excipients include cocoa butter and other materials commonly used in the art. Suppositories can be conveniently prepared by first mixing the pharmaceutical composition with softened or melted excipients, followed by cooling and molding.

In addition to the above-mentioned carrier components, the above-mentioned pharmaceutical preparations may also include, as appropriate, one or more additional excipient components, such as diluents, buffers, flavoring agents, binders, surfactants, thickeners, lubricants and preservatives (including antioxidants). In addition, other adjuvants may also include penetration enhancers that adjust the osmotic pressure of the drug and blood. The pharmaceutical composition comprising the compound as shown in formula (I) or a pharmaceutically acceptable salt thereof can also be prepared in the form of a powder or a concentrated solution.

Generally, for the treatment of the above-mentioned conditions or disorders, the dosage level of the drug is about 0.01 mg/kg to 150 mg/kg body weight per day, or 0.5 mg to 7 g per patient per day. However, it will be understood that the specific dosage level for any particular patient will depend on a variety of factors, including age, weight, general health, sex, diet, time of administration, route of administration, rate of excretion, concomitant drug use and the severity of the particular disease being treated.

### Detailed Description of Embodiments

In order to make the present invention to be easily understood, the present invention will be described in details below with reference to examples. These examples are only for illustration and are not intended to limit the application scope of the present invention. The experimental methods in the present invention are all conventional methods unless otherwise specified. Unless otherwise specified, the experimental materials used in the present invention are commercially available.

Unless otherwise specified, all fractions and percentages are by weight and all temperatures are in degrees Celsius.

The following abbreviations are used in the examples:
CuI: Cuprous iodide;
DAST: Diethylamine sulfur trifluoride;
DCM: Dichloromethane;
DIEA: N,N-diisopropylethylamine;
DMF: N,N-dimethylformamide;
DMSO: Dimethyl sulfoxide;
EA: Ethyl acetate;
ESI-MS: Electrospray ionization mass spectrometry;
K₂CO₃: Potassium carbonate;
NaH: Sodium hydride;
LDA: Diisopropylaminolithium;
LiHMDS: Lithium bis(trimethylsilyl)amide;
*m*-CPBA: m-Chloroperbenzoic acid;
MtBE: Methyl tert-butyl ether;
Na₂SO₄: Sodium sulfate;
NaBH₄: Sodium borohydride;
NBS: N-bromosuccinimide;
NFSI: N-Fluorobisbenzenesulfonamide;
NIS: N-iodosuccinimide;
NMP: N-Methylpyrrolidone;
PE: Petroleum ether;
Pd₂(dba)₃: Tris(dibenzylideneacetone)dipalladium;
Pd(dppf)Cl₂: 1,1'-Bis(diphenylphosphino)ferrocene]palladium dichloride;
Pd(PPh₃)₄: Tetrakis(triphenylphosphino)palladium;
Prep-TLC: Preparative thin layer chromatography;
TMSC₂F₅: (Pentafluoroethyl)trimethylsilane;
TBAF: Tetrabutylammonium fluoride;
TEA: Triethylamine;
THF: Tetrahydrofuran;
Xantphos: 4,5-Bis(diphenylphosphino)-9,9-dimethylxanthene; TLC: Thin layer chromatography;
ESI-MS: Electrospray ionization mass spectrometry;
LCMS or LC-MS: Liquid chromatography-mass spectrometry;
¹H NMR: Nuclear magnetic resonance hydrogen spectrum;
[(R,R) -Ts-DPEN]RuCl(p-cymene): Chloro{[(1R,2R)-(-)-2-amino-1,2-diphenylethyl](4-toluenesulfonyl)amino } (p-cymene)ruthenium(II).

### Synthesis of intermediate M1 (5,5-difluoro-3-iodo-1,5,6,7-tetrahydro-4H-indol-4-one)

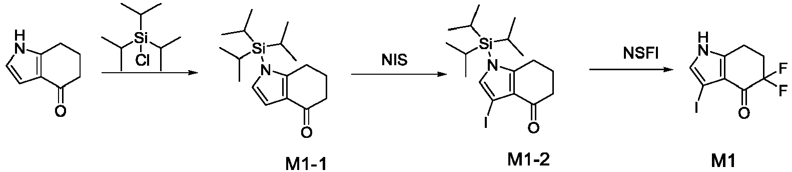

### Step 1: synthesis of compound M1-1

Under nitrogen protection, 6,7-dihydro-1H-indol-4(5H)-one (13.5 g) was suspended in THF (100 mL), and DBU (33.44 g) and triisopropylsilyl chloride (21.2 g) were slowly added dropwise at room temperature. The mixture was stirred at room temperature for 3 hours. MTBE (100 mL) and 10% aqueous citric acid solution (100 mL) were added to the reaction liquid and the mixture was subjected to liquid separation. The organic phase was dried over anhydrous sodium sulfate, filtered and concentrated to obtain the target compound M1-1 (20.3 g), which was used directly in the next step.

ESI-MS m/z: 292.10[M+H]⁺.

### Step 2: Synthesis of compound M1-2

Under nitrogen protection, compound M1-1 (13.5 g) was dissolved in acetonitrile (100 mL), and NIS (11.5 g) was added in batches. The mixture was reacted at room temperature for 5 hours. The resulting reaction mixture was cooled, and the precipitated solid was filtered, rinsed with methanol (50 mL*2) and dried to obtain the target compound M1-2 (15.4 g).

ESI-MS m/z: 418.10 [M+H]+.

### Step 3: Synthesis of compound M1

Compound M1-2 (4.2 g) and NFSI (7.9 g) were suspended in THF (100 mL) and cooled to -78°C, and 1.0 M LiHMDS tetrahydrofuran solution (28.2 mL) was slowly added dropwise. After stirring at - 60°C for 2 hours, saturated ammonium chloride aqueous solution (200 mL) was added to the reaction liquid, and the mixture was extracted twice with ethyl acetate, washed twice with saturated sodium chloride aqueous solution, dried and concentrated to obtain a crude product, which was rinsed with methanol to obtain the target compound M1 (2.1 g).

ESI-MS m/z: 297.99 [M+H]⁺.

¹H NMR (500 MHz, DMSO-d6): δ 12.05 (s, 1H), 7.12 (d, J = 2.3 Hz, 1H), 2.98 (t, J = 6.2 Hz, 2H), 2.60-2.53 (m, 2H).

### Synthesis of intermediate M2 (5,5-difluoro-3-(trifluoromethyl)-1,5,6,7-tetrahydro-4H-indol-4-one)

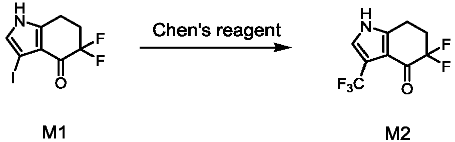

M1 (12 g), cuprous iodide (7.8 g), and methyl fluorosulfonyldifluoroacetate (24 g) were added to NMP (120 mL). The reaction system was replaced with nitrogen, and the mixture was heated to 120°C under nitrogen protection. The reaction was allowed to proceed overnight. TLC and LCMS showed that no raw material remained. The mixture was cooled down and diluted with ethyl acetate. The organic phase was washed with saturated sodium chloride aqueous solution, dried over sodium sulfate, and concentrated to obtain a crude product, which was purified by column chromatography (EA/PE = 1 : 5) to obtain the target compound M2 (8.4 g).

ESI-MS m/z: 238.00 [M-H]⁻.

¹H NMR (500 MHz, DMSO) δ 12.37 (s, 1H), 7.56 (s, 1H), 3.03 (t, *J* = 6.1 Hz, 2H), 2.68 - 2.55 (m, 2H).

### Synthesis of intermediate M3 (5-fluoro-3-(trifluoromethyl)-1,5,6,7-tetrahydro-4H-indol-4-one)

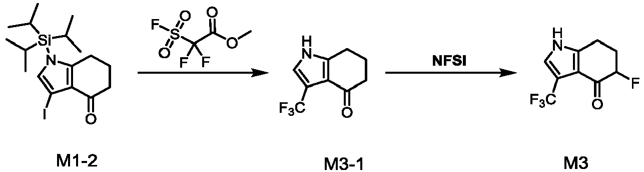

### Step 1: Synthesis of compound M3-1

Compound M1-2 (834 mg), cuprous iodide (380 mg), NMP (20 mL) and methyl fluorosulfonyldifluoroacetate (1.15 g) were added to a 50 mL single-necked bottle in sequence, the mixture was heated to 120°C, and the reaction was carried out for 6 h. The reaction mixture was diluted with EA, washed three times with water, washed once with saturated sodium chloride aqueous solution, dried, and concentrated to obtain a crude product, which was purified by column chromatography (DCM = 100%) to obtain the target compound M3-1 (264 mg).

ESI-MS m/z: 204.08[M+H⁺]⁺.

### Step 2: Synthesis of compound M3

### Method I:

Under nitrogen protection, compound M3-1 (264 mg) was dissolved in ultra-dry tetrahydrofuran (20 mL), cooled to -78°C, and 1.0 M LDA tetrahydrofuran solution (2.5 mL) was slowly added dropwise. After stirring for half an hour, NFSI (491 mg) tetrahydrofuran solution (5 mL) was added dropwise, and stirring was continued at -78°C for two hours. Saturated ammonium chloride aqueous solution was added to the reaction mixture to quench the reaction, and the resulting mixture was extracted twice with ethyl acetate, washed twice with saturated sodium chloride aqueous solution, dried and concentrated to obtain a crude product, which was purified by column chromatography (DCM = 100%) to obtain the target compound M3 (147 mg).

### Method II:

Under nitrogen protection, compound M3-1 (200 mg) was dissolved in methanol (3 mL), NFSI (465 mg) was added, and the mixture was refluxed at 70°C overnight. After the reaction was completed, the mixture was concentrated and purified by column chromatography (DCM = 100%) to obtain the target compound M3 (173 mg).

ESI-MS m/z: 222.12[M+H⁺]⁺.

### Synthesis of intermediate M4 (3-bromo-7-fluoro-1-(trifluoromethyl)-6,7-dihydroindolizin-8(5H)-one) and intermediate M5 (3-bromo-7,7-difluoro-1-(trifluoromethyl)-6,7-dihydroindolizin-8(5H)-one)

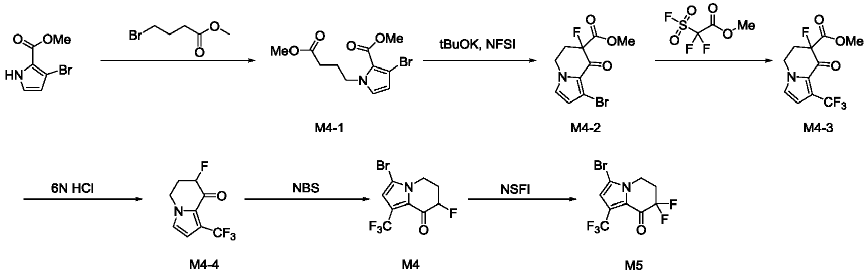

### Step 1: synthesis of compound M4-1

Under nitrogen protection, methyl 3-bromo-1H-pyrrole-2-carboxylate (20.4 g) and methyl 4-bromobutyrate (19.8 g) were dissolved in DMF (100 mL), and cesium carbonate (65.2 g) was added. After stirring at room temperature for 4 hours, the reaction liquid was poured into an ice-water mixture (500 mL), extracted with ethyl acetate, washed with saturated sodium chloride aqueous solution, dried over anhydrous sodium sulfate, and concentrated to obtain a crude product, which was purified by column chromatography (EA/PE = 1 : 10) to obtain the target compound M4-1 (28.3 g).

ESI-MS m/z: 304.10[M+H]⁺.

### Step 2: Synthesis of compound M4-2

Under nitrogen protection, compound M4-1 (15.2 g) was dissolved in THF (100 mL), and 1.0 M potassium tert-butoxide tetrahydrofuran solution (60 mL) was slowly added dropwise in an ice bath. After stirring at 0°C for 3 hours, the mixture was cooled to -78°C, and a solution of NFSI (18.9 g) in tetrahydrofuran (50 mL) was added dropwise. The reaction was continued at -78°C for 1 hour. After the reaction was quenched by adding saturated ammonium chloride aqueous solution, the mixture was extracted with ethyl acetate, washed with saturated sodium chloride aqueous solution, dried over anhydrous sodium sulfate, and concentrated to obtain a crude product, which was purified by column chromatography (EA/PE=1:5) to obtain the target compound M4-2 (9.1 g).

ESI-MS m/z: 277.15 [M+H]⁺.

### Step 3: Synthesis of compound M4-3

Under nitrogen protection, compound M4-2 (8.3 g) was dissolved in DMF (100 mL), palladium acetate (3.3 g), CuI (11.4 g) and methyl fluorosulfonyldifluoroacetate (23 g) were added. The mixture was stirred at 100°C for 3 hours and extracted with ethyl acetate, and the organic layer was washed with brine, dried over Na₂SO₄ and concentrated to obtain a crude product, which was purified by column chromatography (EA/PE = 1 : 4) to obtain the target compound M4-3 (4.23 g).

ESI-MS m/z: 279.20 [M+H]⁺.

### Step 4: Synthesis of compound M4-4

Compound M4-3 (4.23 g) was dissolved in a mixed solvent of ethanol (20 mL) and 6 M aqueous hydrochloric acid solution (20 mL), and the reaction was allowed to reflux for 3 hours. The mixture was concentrated under reduced pressure and the ethanol was removed by rotary evaporation, and the residue was extracted with ethyl acetate (100 mL*2). The organic layer was washed with brine (100 mL*2), then dried over Na₂SO₄ and concentrated to obtain a crude product, which was purified by column chromatography (EA/PE = 1 : 4) to obtain the target compound M4-4 (2.1 g).

ESI-MS m/z: 221.20 [M+H]⁺.

### Step 5: Synthesis of compound M4

Compound M4-4 (2.1 g) was dissolved in acetonitrile (20 mL), NBS (1.8 g) was added at room temperature, and the reaction liquid was refluxed for 4 hours, quenched with water, extracted with ethyl acetate, washed with saturated sodium chloride aqueous solution, dried over anhydrous sodium sulfate, and concentrated to obtain a crude product, which was purified by column chromatography (EA/PE = 1 : 3) to obtain the target compound M4 (2.5 g).

ESI-MS m/z: 300.10 [M+H]⁺.

### Step 6: Synthesis of compound M5

Compound M4 (1.3 g) was dissolved in THF (50 mL), cooled to -78°C, and 1.0 M LiHMDS tetrahydrofuran solution (6 mL) was slowly added dropwise. After stirring for half an hour, a solution of NFSI (1.5 g) in tetrahydrofuran (10 mL) was added dropwise. The mixture was stirred for another two hours. Saturated ammonium chloride aqueous solution was added to quench the reaction. The mixture was extracted twice with ethyl acetate, washed twice with saturated sodium chloride aqueous solution, dried, and concentrated to obtain a crude product, which was purified by column chromatography (EA/PE = 1 : 3) to obtain the target compound M5 (0.8 g).

### Example 1 Synthesis of 5-(5,5-difluoro-4-hydroxyl-3-(trifluoromethyl)-4,5,6,7-tetrahydro-1H-indol-1-yl)-2,3-difluorobenzonitrile (compound B1)

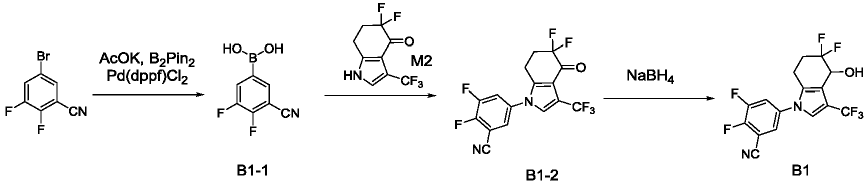

### Step 1: Synthesis of compound B1-1

Potassium acetate (0.76 g), 1,1'-bis(diphenylphosphino)ferrocene]palladium dichloride dichloromethane complex (0.21 g), bis(pinacolato)diboron (1.31 g), and 5-bromo-2,3-difluorobenzonitrile (0.56 g) were dissolved in dioxane (6 mL) and dimethyl sulfoxide (0.05 mL). The reaction system was replaced with nitrogen, and the mixture was stirred at 95°C overnight. After the reaction was completed, ethyl acetate and water were added. The mixture was subjected to liquid separation, washed with saturated sodium chloride aqueous solution, dried and concentrated to obtain a crude product B1-1 (0.7 g), which was directly used in the next step.

### Step 2: Synthesis of compound B1-2

Copper acetate (0.31 g), 4A molecular sieve (0.5 g), compound M2 (0.47 g) and compound B1-1 (0.7 g) were placed in a reaction bottle, and DMF (1 mL) and triethylamine (0.71 mL) were added. The reaction system was replaced with oxygen, and the mixture was stirred at 80°C overnight, cooled to room temperature, filtered, diluted with ethyl acetate, washed with water and saturated sodium chloride aqueous solution, dried, concentrated, and purified by column chromatography to obtain the target compound B1-2 (0.07 g).

### Step 3: Synthesis of compound B1

Compound B1-2 (0.07 g) was dissolved in methanol (1 mL) and tetrahydrofuran (1 mL), and cooled in an ice-water bath. Sodium borohydride (0.02 g) was added, and stirred at room temperature overnight, and ethyl acetate and water were added after the reaction was completed. The mixture was washed with saturated sodium chloride aqueous solution, dried, concentrated, and purified by Prep-TLC to obtain the target compound B1 (0.07 g).

ESI-MS m/z: 423.21 [M+HCOO⁻]⁻.

### Example 2 Synthesis of 5,5-difluoro-1-(4-fluoro-3-(fluoromethyl)phenyl)-3-(trifluoromethyl)-4,5,6,7-tetrahydro-1H-indol-4-ol (compound B2)

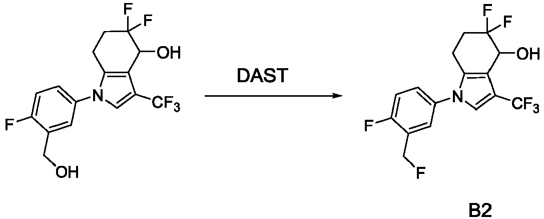

5,5-Difluoro-1-(4-fluoro-3-(hydroxymethyl)phenyl)-3-(trifluoromethyl)-4,5,6,7-tetrahydro-1H-indol-4-ol (100 mg) was dissolved in dichloromethane (2 mL), and cooled in an ice-water bath, and DAST (48 mg) was added. After stirring in an ice-water bath for 1 hour, saturated sodium bicarbonate was added to the reaction liquid, and the mixture was diluted with dichloromethane. The mixture was subjected to liquid separation, and the organic phase was washed with saturated sodium chloride aqueous solution, dried, and concentrated, and purified by Prep-TLC to obtain the target compound B2 (10 mg).

ESI-MS m/z: 350.10[M+H₂O-H⁺]⁺.

### Example 3 Synthesis of (S)-4-(S,S-difluoro-4-hydroxyl-3-(trifluoromethyl)-4,S,6,7-tetrahydro-1H-indol-1-yl)-2-(difluoromethyl)benzonitrile (compound B3)

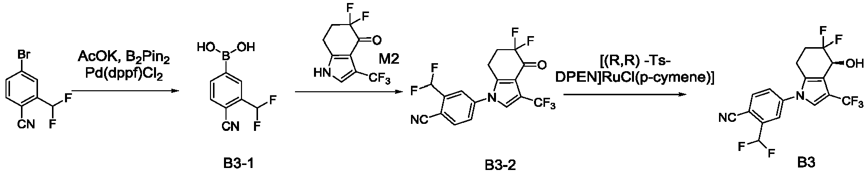

### Step 1: Synthesis of compound B3-1

Potassium acetate (224 mg), 1,1'-bis(diphenylphosphino)ferrocene]palladium dichloride dichloromethane complex (42 mg), bis(pinacolato)diboron (305 mg), and 4-bromo-2-(difluoromethyl)benzonitrile (265 mg) were dissolved in dioxane (6 mL), and the mixture was stirred at 80°C overnight under nitrogen protection. After the reaction was completed, ethyl acetate and water were added to the reaction liquid. The mixture was subjected to liquid separation, washed with saturated sodium chloride aqueous solution, dried and concentrated to obtain a crude product, which was purified by column chromatography to obtain compound B3-1 (165 mg).

### Step 2: Synthesis of compound B3-2

DCM (5 mL) and diisopropylethylamine (0.13 mL) were added to a mixture of copper acetate (92 mg), 4A molecular sieve (100 mg), compound M2 (61 mg) and compound B3-1 (50 mg). The reaction system was replaced with oxygen, and the mixture was stirred at room temperature overnight, filtered, concentrated, and purified by column chromatography to obtain the target compound B3-2 (64 mg).

### Step 3: Synthesis of compound B3

To a solution of compound B3-2 (64 mg) in dichloromethane, triethylamine (0.05 mL), chloro{ [(1R,2R)-(-)-2-amino-1,2-diphenylethyl](4-toluenesulfonyl)amino}(p-cymene)ruthenium(II) (10 mg) and formic acid (23 mg) were added, stirred at room temperature overnight, concentrated, and purified by column chromatography to obtain the target compound B3 (35 mg).

ESI-MS m/z: 437.24 [M+HCOO⁻]⁻.

### Example 4 Synthesis of (S)-5-(3-((difluoromethyl)sulfonyl)-5,5-difluoro-4-hydroxyl-4,5,6,7-tetrahydro-1H-indol-1-yl)-2-fluorobenzonitrile (compound A3)

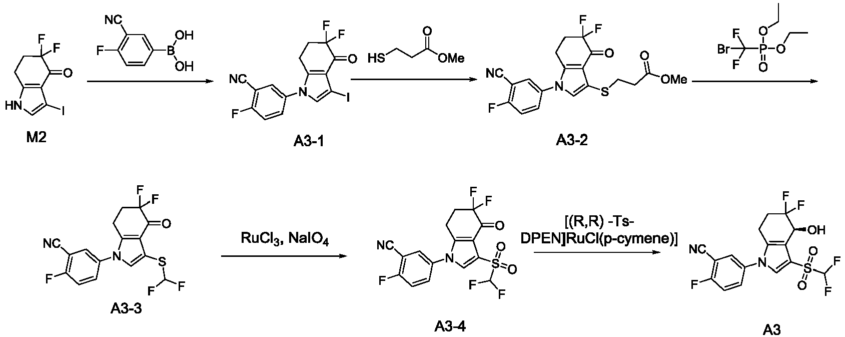

### Step 1: Synthesis of compound A3-1

To a solution of compound M2 (0.9 g) in DCM (50 mL) were added copper acetate (0.55 g), 3-cyano-4-fluorophenylboronic acid (1.0 g) and TEA (1.2 g), and the mixture was stirred at room temperature overnight. The reaction mixture was diluted with dichloromethane, washed twice with saturated sodium chloride aqueous solution, dried, and concentrated to obtain a crude product, which was purified by column chromatography (EA/PE = 1 : 5) to obtain the target compound A3-1 (0.92 g).

### Step 2: Synthesis of compound A3-2

A mixture of compound A3-1 (1.26 g), methyl 3-mercaptopropionate (0.73 g), Xantphos (0.35 g), tri(dibenzylideneacetone)dipalladium (0.28 g), DIEA (1.5 mL) and toluene (40 mL) was stirred at 70°C overnight. After the reaction was completed, the mixture was concentrated to obtain a crude product, which was purified by column chromatography to obtain the target compound A3-2 (1.10 g).

### Step 3: Synthesis of compound A3-3

To a solution of compound A3-2 (0.40 g) in tetrahydrofuran (10 mL) was added a 1.0 M solution of potassium tert-butoxide in tetrahydrofuran (1.5 mL) at -78°C. After stirring for 10 minutes, diethyl bromofluoromethylphosphonate (0.40 g) was added. After stirring for 2 hours, water was added to quench the reaction. The reaction liquid was heated to room temperature and stirring was continued overnight. The reaction solution was diluted with water and ethyl acetate. The organic phase was washed with saturated sodium chloride aqueous solution, dried, and concentrated to obtain a crude product, which was purified by column chromatography (EA/PE = 1 : 3) to obtain the target compound A3-3 (0.18 g).

### Step 4: Synthesis of compound A3-4

To a mixture of compound A3-3 (0.15 g), acetonitrile (1 mL), dichloromethane (1 mL) and water (2 mL) was added ruthenium trichloride (0.01 g) and sodium periodate (0.5 g) and the mixture was stirred at room temperature for 2 h. After the reaction was completed, the reaction liquid was diluted with ethyl acetate, washed with water and saturated sodium chloride aqueous solution, dried, and concentrated to obtain a crude product, which was purified by column chromatography (EA/PE = 1 : 3) to obtain the target compound A3-4 (0.1 g).

### Step 5: Synthesis of compound A3

Triethylamine (0.08 mL), chloro{[(1R,2R)-(-)-2-amino-1,2-diphenylethyl](4-toluenesulfonyl)amino}(P-cymene)ruthenium(II) (0.04 g) and formic acid (0.04 g) were added to a solution of compound A3-4 (0.12 g) in dichloromethane, and the mixture was stirred at room temperature overnight. The reaction liquid was diluted with dichloromethane, washed with water and saturated sodium chloride aqueous solution, dried, and concentrated to obtain a crude product, which was purified by column chromatography (EA/PE = 1 : 3) to obtain the target compound A3 (0.09 g).

ESI-MS m/z: 451.16 [M+HCOO⁻]⁻.

### Example 5 Synthesis of (S)-1-(3-(difluoromethyl)-4-fluorophenyl)-5,5-difluoro-3-(methylsulfonyl)-4,5,6,7 tetrahydro-1H-indol-4-ol (compound A4)

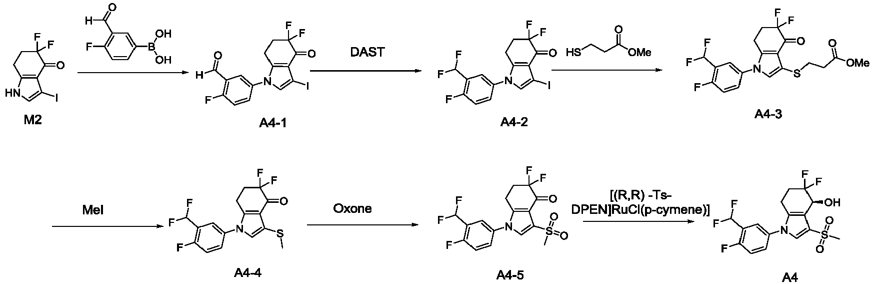

### Step 1: Synthesis of compound A4-1

(4-Fluoro-3-formylphenyl)boronic acid (2.0 g), compound M2 (3.5 g), copper acetate (2.5 g) and triethylamine (5.0 mL) were added to dichloromethane (40 mL) respectively, and the mixture was stirred at room temperature overnight under an oxygen atmosphere. After the reaction was completed, the reaction liquid was diluted with dichloromethane, washed twice with saturated sodium chloride aqueous solution, dried, filtered, and concentrated to obtain a crude product, which was purified by column chromatography (EA/PE = 1 : 3) to obtain the target compound A4-1 (1.8 g).

ESI-MS m/z: 420.0 [M+H]⁺.

### Step 2: Synthesis of compound A4-2

DAST (700 mg) was added to a solution of compound A4-1 (500 mg) in dichloromethane (10 mL) and the mixture was stirred at room temperature overnight. After the reaction was completed, the reaction liquid was diluted with dichloromethane and quenched with saturated sodium bicarbonate. The organic phase was washed with water, dried, filtered, and concentrated to obtain a crude product, which was purified by column chromatography (EA/PE = 1 : 3) to obtain the target compound A4-2 (150 mg).

ESI-MS m/z: 442.0 [M+H]⁺.

### Step 3: Synthesis of compound A4-3

A mixture of compound A4-2 (0.88 g), methyl 3-mercaptopropionate (0.36 g), Xantphos (0.23 g), tri(dibenzylideneacetone)dipalladium (0.18 g), DIEA (0.66 mL) and toluene (5 mL) was stirred at 70°C overnight. After the reaction was completed, the mixture was concentrated to obtain a crude product, which was purified by column chromatography to obtain the target compound A4-3 (0.8 g).

ESI-MS m/z: 434.0 [M+H]⁺.

### Step 4: Synthesis of compound A4-4

Compound A4-3 (0.30 g) was dissolved in tetrahydrofuran (10 mL), and the reaction solution was cooled to -78°C and stirred for half an hour. A 1.0 M solution of potassium tert-butoxide in tetrahydrofuran (1.0 mL) was then added, and stirred for 10 minutes. Iodomethane (0.09 mL) was added, and the stirring was continued at -78°C for 2 hours. The reaction liquid was diluted with water and ethyl acetate, and the organic phase was washed with saturated sodium chloride aqueous solution, dried and concentrated to obtain a crude product, which was purified by column chromatography (EA/PE = 1 : 3) to obtain the target compound A4-4 (0.20 g).

ESI-MS m/z: 362.0 [M+H]⁺.

### Step 5: Synthesis of compound A4-5

Potassium peroxymonosulfonate (0.71 g) was added to a mixture of compound A4-4 (0.2 g), acetonitrile (10 mL) and water (2 mL), and the mixture was stirred at 60°C for 2 h. After the reaction was completed, the reaction liquid was diluted with ethyl acetate, washed with water and saturated sodium chloride aqueous solution, dried, and concentrated to obtain a crude product, which was purified by column chromatography (EA/PE = 1 : 3) to obtain the target compound A4-5 (0.18 g).

ESI-MS m/z: 394.0 [M+H]⁺.

### Step 6: Synthesis of compound A4

To a solution of A4-5 (0.06 g) in dichloromethane (3 mL) were added triethylamine (0.03 g), chloro{[(1R,2R)-(-)-2-amino-1,2-diphenylethyl](4-toluenesulfonyl)amino}(P-cymene)ruthenium(II) (0.01 g) and formic acid (0.02 g), and the mixture was stirred at room temperature overnight. The reaction liquid was diluted with dichloromethane, washed with water and saturated sodium chloride aqueous solution, dried, and concentrated to obtain a crude product, which was purified by column chromatography (EA/PE = 1 : 3) to obtain the target compound A4 (0.03 g).

ESI-MS m/z: 451.16 [M+H⁺-H₂O]⁺.

### Example 6 Synthesis of (S)-1-(3-(difluoromethyl)-4-fluorophenyl)-3-((difluoromethyl)sulfonyl)-5,5-difluoro-4,5,6,7-tetrahydro-1H-indol-4-ol (compound A5)

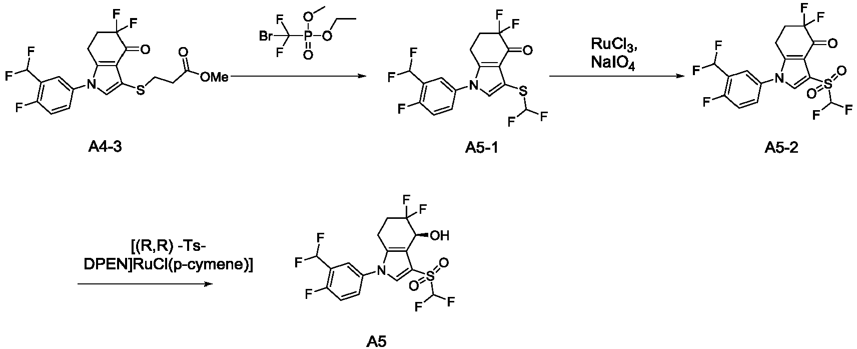

### Step 1: Synthesis of compound AS-1

Compound A4-3 (0.20 g) was dissolved in tetrahydrofuran (10 mL), and the solution was cooled to -78°C and stirred for half an hour. A 1.0 M solution of potassium tert-butoxide in tetrahydrofuran (0.7 mL) was added, and the mixture was stirred for 10 minutes. Diethyl bromofluoromethylphosphonate (0.20 g) was then added, and stirring was continued at -78°C for 2 hours. The reaction liquid was diluted with water and ethyl acetate, and the organic phase was washed with saturated sodium chloride aqueous solution, dried and concentrated, and purified by column chromatography to obtain the target compound A5-1 (0.15 g).

ESI-MS m/z: 398.0 [M+H]⁺.

### Step 2: Synthesis of compound A5-2

To a mixture of compound A5-1 (0.15 g), acetonitrile (2 mL), dichloromethane (2 mL) and water (4 mL) were added ruthenium trichloride (0.01 g) and sodium periodate (0.24 g), and the mixture was stirred at room temperature for 2 h. After the reaction was completed, the reaction liquid was diluted with ethyl acetate, washed with water and saturated sodium chloride aqueous solution, dried, concentrated, and purified by column chromatography to obtain the target compound A5-2 (0.08 g).

### Step 3: Synthesis of compound A5

To a solution of compound A5-2 (0.08 g) in dichloromethane (3 mL) were added triethylamine (0.05 mL), chloro{[(1R,2R)-(-)-2-amino-1,2-diphenylethyl](4-toluenesulfonyl)amino}(P-cymene)ruthenium(II) (0.01 g) and formic acid (0.03 g), and the mixture was stirred at room temperature overnight. The reaction liquid was diluted with dichloromethane, washed with water and saturated sodium chloride aqueous solution, dried, and concentrated, and purified by column chromatography to obtain the target compound A5 (0.03 g).

ESI-MS m/z: 414.05 [M+H⁺-H₂O]⁺.

### Example 7 and example 8 Synthesis of (S)-3-((S)-(difluoromethyl)sulfinyl)-5,5-difluoro-1-(4-fluoro-3-(trifluoromethyl)phenyl)-4,5,6,7-tetrahydro-1H-indol-4-ol (compound A13, presumed) and (S)-3-((R)-(difluoromethyl)sulfinyl)-5,5-difluoro-1-(4-fluoro-3-(trifluoromethyl)phenyl)-4,5,6,7-tetrahydro-1H-indol-4-ol (compound A14, presumed)

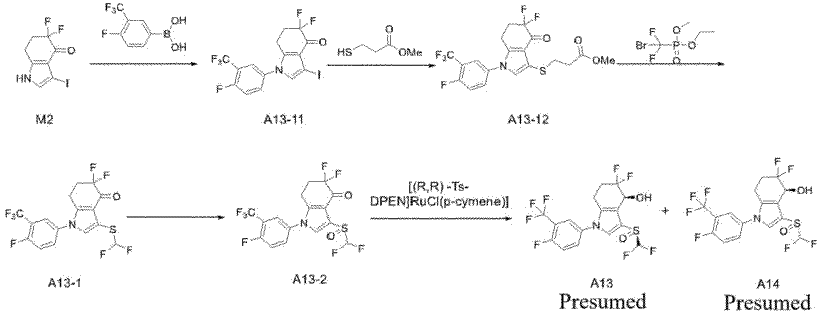

### Step 1: Synthesis of compound A13-11

3-Cyano-4-fluorophenylboronic acid was replaced with 3-trifluoromethyl-4-fluorophenylboronic acid and compound A13-11 was obtained with reference to the method of step 1 of example 4.

### Step 2: Synthesis of compound A13-12

Compound A3-1 was replaced with compound A13-11 and compound 13-12 was obtained with reference to the method of step 2 of example 4.

### Step 3: Synthesis of compound 13-1

Compound A3-2 was replaced with compound 13-11 and compound 13-1 was obtained with reference to the method of step 3 of example 4.

### Step 4: Synthesis of compound A13-2

To a mixed solution of compound A13-1 (120 mg) in acetonitrile (2 mL) and water (2 mL) was added potassium peroxymonosulfonate (210 mg), and the mixture was stirred at 65°C overnight. After the reaction was completed, the mixture was cooled, diluted with ethyl acetate, washed with water and saturated sodium chloride aqueous solution, dried, concentrated, and purified by column chromatography to obtain the target compound A13-2 (90 mg).

ESI-MS m/z: 430.0 [M-H⁺].

### Step 5: Synthesis of compounds A13 and A14

To a solution of A13-2 (100 mg) in dichloromethane (5 mL) were added chloro{[(1R,2R)-(-)-2-amino-1,2-diphenylethyl](4-toluenesulfonyl)amino}(p-cymene)ruthenium(II) (10 mg), triethylamine (0.06 mL) and formic acid (0.03 g), and the mixture was stirred at room temperature overnight. The reaction liquid was diluted with dichloromethane, washed with water and saturated sodium chloride aqueous solution, dried, and concentrated, and the crude product was purified by HPLC (Luna PREP C18, mobile phase A: water (0.1% formic acid), mobile phase B: acetonitrile (35% to 70%), flow rate: 40 mL/min, detection wavelength: 254 nm, temperature: room temperature), and the front peak was collected as the target compound A13 (20 mg), and the back peak was collected as the target compound A14 (20 mg).

ESI-MS m/z: 416.07 [M+H⁺-H₂O]⁺.

### Example 9 Synthesis of 5-(5,5-difluoro-4-hydroxyl-3-(perfluoroethyl)-4,5,6,7-tetrahydro-1H-indol-1-yl)-2-fluorobenzonitrile (compound A10)

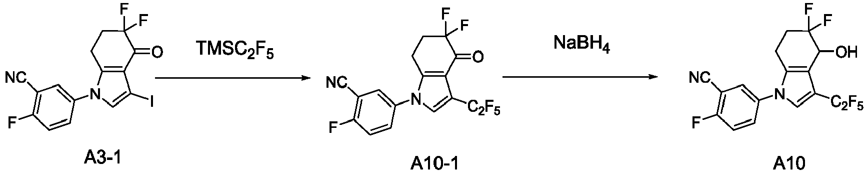

### Step 1: Synthesis of compound A10-1

TMSC₂F₅ (0.11 mL) and pyridine (0.12 mL) were added to a mixed solution of A3-1 (130 mg), cuprous iodide (0.12 g), silver fluoride (0.08 g) and dimethyl sulfoxide (2 mL), and stirred at 80°C overnight. The reaction liquid was diluted with ethyl acetate, washed with water and saturated sodium chloride aqueous solution, dried, concentrated, and purified by column chromatography to obtain the target compound A10-1 (100 mg).

### Step 2: Synthesis of compound A10

Compound A10-1 (100 mg) was dissolved in methanol (1 mL) and tetrahydrofuran (1 mL), and the mixture was cooled to 0°C in an ice-water bath. Sodium borohydride (30 mg) was added, and the reaction was allowed to react at room temperature overnight. The reaction liquid was diluted with water, and extracted with ethyl acetate, and the organic phase was washed with saturated sodium chloride aqueous solution, dried, concentrated, and purified by column chromatography (EA/PE = 1 : 3) to obtain the target compound A10 (90 mg).

ESI-MS m/z: 455.17 [M+HCOO⁻]⁻.

### Example 10 Synthesis of (S)-5-(5,5-difluoro-4-hydroxyl-3-((trifluoromethyl)sulfonyl)-4,5,6,7-tetrahydro-1H-indol-1-yl)-2-fluorobenzonitrile (compound A11)

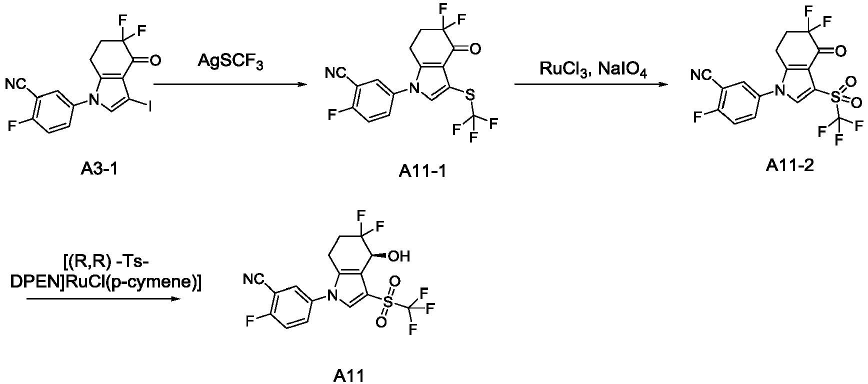

### Step 1: Synthesis of compound A11-1

N,N-dimethylacetamide (2 mL) was added to a mixture of copper(I) thiophene-2-carboxylate (270 mg), compound A3-1 (400 mg) and silver trifluoromethanethiol (300 mg), and the mixture was stirred at 100°C overnight. The mixture was cooled to room temperature, and the reaction liquid was diluted with ethyl acetate, washed with water and saturated sodium chloride aqueous solution, dried, concentrated, and purified by column chromatography (EA/PE = 1 : 3) to obtain the target compound A11-1 (210 mg).

ESI-MS m/z: 389.0 [M-H⁺]⁻.

### Step 2: Synthesis of compound A11-2

To a mixture of compound A11-1 (100 mg) in acetonitrile (2 mL), dichloromethane (2 mL) and water (4 mL), ruthenium trichloride (0.01 g) and sodium periodate (0.22 g) were added and stirred at room temperature for 2 h. After the reaction was completed, the reaction liquid was diluted with ethyl acetate, washed with water and saturated sodium chloride aqueous solution, dried, concentrated, and purified by column chromatography (EA/PE = 1 : 3) to obtain the target compound A11-2 (70 mg).

ESI-MS m/z: 421.0 [M-H⁺]⁻.

### Step 3: Synthesis of compound A11

To a solution of compound A11-2 (50 mg) in dichloromethane (5 mL) were added chloro {[(1R,2R)-(-)-2-amino-1,2-diphenylethyl] (4-toluenesulfonyl)amino}(p-cymene)ruthenium(II) (10 mg), triethylamine (0.03 mL) and formic acid (30 mg), and the mixture was stirred at room temperature overnight. The reaction liquid was diluted with dichloromethane, washed with water and saturated sodium chloride aqueous solution, dried, and concentrated, and purified by column chromatography (EA/PE = 1 : 3) to obtain the target compound A11 (10 mg).

ESI-MS m/z: 469.10 [M+HCOO⁻]⁻.

### Example 11 Synthesis of (S)-5-(5,5-difluoro-3-((fluoromethyl)sulfonyl)-4-hydroxyl-4,5,6,7-tetrahydro-1H-indol-1-yl)-2-fluorobenzonitrile (compound A16)

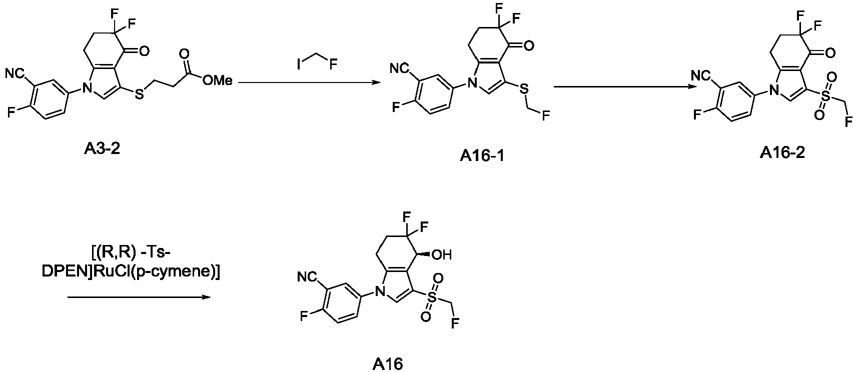

### Step 1: Synthesis of compound A16-1

To a solution of compound A3-2 (120 mg) in tetrahydrofuran (10 mL) was added a 1.0 M solution of potassium tert-butoxide in tetrahydrofuran (0.44 mL) at -78°C. After stirring for 10 minutes, fluoroiodomethane (70 mg) was added and stirring was continued for 2 hours. The reaction liquid was diluted with water and ethyl acetate. The organic phase was washed with saturated sodium chloride aqueous solution, dried, concentrated, and purified by column chromatography (EA/PE = 1 : 3) to obtain the target compound A16-1 (80 mg).

ESI-MS m/z: 353.0 [M-H⁺]⁻.

### Step 2: Synthesis of compound A16-2

Potassium peroxymonosulfonate (0.63 g) was added to a mixed solution of compound A16-1 (60 mg) in acetonitrile (3 mL) and water (3 mL), and the reaction solution was stirred at 60°C overnight. After the reaction was completed, the reaction liquid was diluted with ethyl acetate, washed with water and saturated sodium chloride aqueous solution, dried, concentrated, and purified by column chromatography (EA/PE = 1 : 3) to obtain the target compound A16-2 (50 mg).

ESI-MS m/z: 385.0 [M-H⁺]⁻.

### Step 3: Synthesis of compound A16

To a solution of compound A16-2 (70 mg) in dichloromethane (5 mL) were added triethylamine (0.05 mL), chloro{[(1R,2R)-(-)-2-amino-1,2-diphenylethyl](4-toluenesulfonyl)amino}(p-cymene)ruthenium(II) (10 mg), and formic acid (20 mg), and the mixture was stirred at room temperature overnight. The reaction liquid was diluted with dichloromethane, washed with water and saturated sodium chloride aqueous solution, dried, and concentrated, and purified by column chromatography (EA/PE = 1 : 3) to obtain the target compound A16 (30 mg).

ESI-MS m/z: 433.16 [M+HCOO⁻]⁻.

### Example 12 Synthesis of (S)-1-(3-(difluoromethyl)-4-fluorophenyl)-5,5-difluoro-3-((trifluoromethyl)sulfonyl)-4,5,6,7-tetrahydro-1H-indol-4-ol (compound A17)

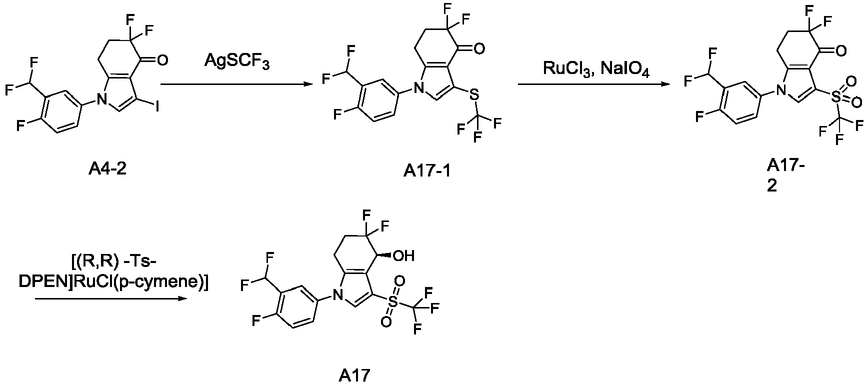

### Step 1: synthesis of compound A17-1

N,N-dimethylacetamide (5 mL) was added to a mixture of copper(I) thiophene-2-carboxylate (140 mg), compound A4-2 (220 mg) and silver trifluoromethanethiol (160 mg), and the mixture was stirred at 100°C overnight. The mixture was cooled to room temperature, and the reaction liquid was diluted with ethyl acetate, washed with water and saturated sodium chloride aqueous solution, dried, concentrated, and purified by column chromatography to obtain the target compound A17-1 (160 mg).

ESI-MS m/z: 416.0 [M+H⁺]⁺.

### Step 2: Synthesis of compound A17-2

To a mixture of compound A17-1 (0.17 g) in acetonitrile (3 mL), dichloromethane (3 mL) and water (6 mL), ruthenium trichloride (0.01 g) and sodium periodate (0.44 g) were added and stirred at room temperature overnight. After the reaction was completed, the reaction liquid was diluted with ethyl acetate, washed with water and saturated sodium chloride aqueous solution, dried, concentrated, and purified by column chromatography (EA/PE = 1 : 3) to obtain the target compound A17-2 (0.07 g).

ESI-MS m/z: 446.0 [M-H⁺]⁻.

### Step 3: Synthesis of compound A17

To a solution of A17-2 (70 mg) in dichloromethane (5 mL) were added chloro{[(1R,2R)-(-)-2-amino-1,2-diphenylethyl](4-toluenesulfonyl)amino}(p-cymene)ruthenium(II) (10 mg), triethylamine (0.04 mL) and formic acid (0.02 g), and the mixture was stirred at room temperature overnight. The reaction liquid was diluted with dichloromethane, washed with water and saturated sodium chloride aqueous solution, dried, and concentrated, and purified by column chromatography (EA/PE = 1 : 3) to obtain the target compound A17 (60 mg).

ESI-MS m/z: 494.12 [M+HCOO⁻]⁻.

### Example 13 Synthesis of (S)-2-((5,5-difluoro-1-(4-fluoro-3-(trifluoromethyl)phenyl)-4-hydroxyl-4,5,6,7-tetrahydro-1H-indol-3-yl)sulfonyl)acetonitrile (compound A18)

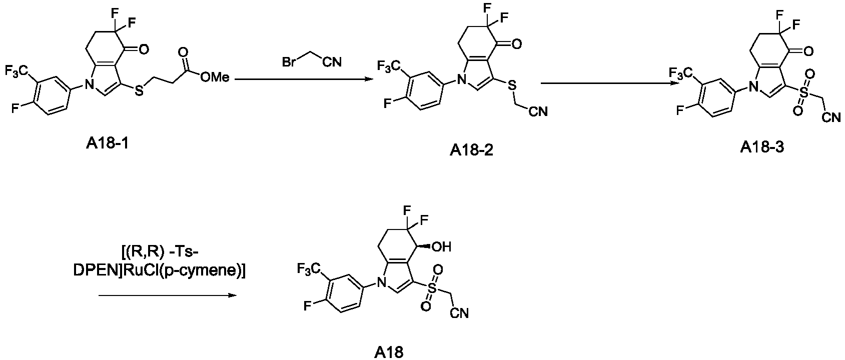

### Step 1: Synthesis of compound A18-2

To a solution of A18-1 (160 mg) in tetrahydrofuran (10 mL) was added a 1.0 M solution of potassium tert-butoxide in tetrahydrofuran (0.5 mL) at -78°C. After stirring for 10 minutes, bromoacetonitrile (60 mg) was added and stirring was continued for 2 hours. The reaction liquid was diluted with water and ethyl acetate. The organic phase was washed with saturated sodium chloride aqueous solution, dried, concentrated, and purified by column chromatography (EA/PE = 1 : 3) to obtain the target compound A18-2 (120 mg).

ESI-MS m/z: 405 [M+H⁺]⁺.

### Step 2: Synthesis of compound A18-3

Potassium peroxymonosulfonate (457 mg) was added to a mixture of compound A18-2 (120 mg) in acetonitrile (3 mL) and water (3 mL), and the reaction solution was stirred at 60°C for 2h. After the reaction was completed, the reaction liquid was diluted with ethyl acetate, washed with water and saturated sodium chloride aqueous solution, dried, concentrated, and purified by column chromatography (EA/PE = 1 : 3) to obtain the target compound A18-3 (100 mg).

ESI-MS m/z: 435 [M-H⁺]⁻.

### Step 3: Synthesis of compound A18

To a solution of compound A18-3 (100 mg) in dichloromethane (5 mL) were added triethylamine (0.06 mL), chloro{[(1R,2R)-(-)-2-amino-1,2-diphenylethyl](4-toluenesulfonyl)amino}(p-cymene)ruthenium(II) (10 mg), and formic acid (30 mg), and the mixture was stirred at room temperature overnight. The reaction liquid was diluted with dichloromethane, washed with water and saturated sodium chloride aqueous solution, dried, and concentrated, and purified by column chromatography (EA/PE = 1 : 3) to obtain the target compound A18 (50 mg).

ESI-MS m/z: 437.12 [M-H⁻]⁻.

### Example 14 Synthesis of (S)-1-(3-(difluoromethyl)-4-fluorophenyl)-5,5-difluoro-3-((fluoromethyl)sulfonyl)-4,5,6,7-tetrahydro-1H-indol-4-ol (compound A19)

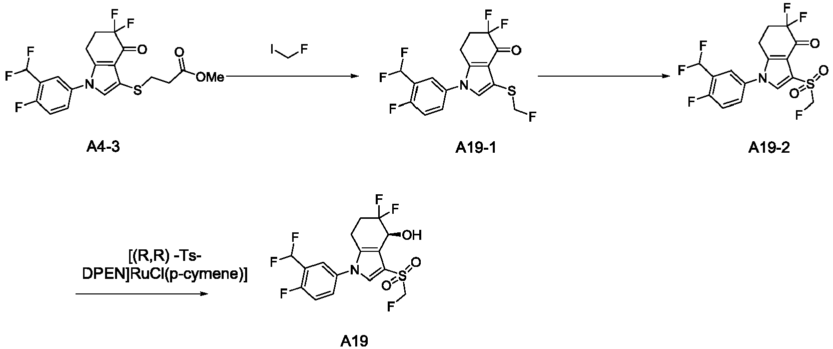

### Step 1: Synthesis of compound A19-1

To a solution of compound A4-3 (270 mg) in tetrahydrofuran (10 mL) was added a 1.0 M solution of potassium tert-butoxide in tetrahydrofuran (1.0 mL) at -78°C. After stirring for 10 minutes, fluoroiodomethane (0.15 g) was added and stirring was continued for 2 hours while keeping the temperature unchanged. The reaction liquid was diluted with water and ethyl acetate. The organic phase was washed with saturated sodium chloride aqueous solution, dried, concentrated, and purified by column chromatography (EA/PE = 1 : 3) to obtain the target compound A19-1 (180 mg).

ESI-MS m/z: 380 [M+H⁺]⁺.

### Step 2: Synthesis of compound A19-2

Potassium peroxymonosulfonate (1.85 g) was added to a solution of compound A19-1 (190 mg) in acetonitrile (4 mL) and water (4 mL), and the reaction solution was stirred at 60°C for 3h. After the reaction was completed, the reaction liquid was diluted with ethyl acetate, washed with water and saturated sodium chloride aqueous solution, dried, concentrated, and purified by column chromatography (EA/PE = 1 : 3) to obtain the target compound A19-2 (150 mg).

ESI-MS m/z: 412 [M+H⁺]⁺.

### Step 3: Synthesis of compound A19

To a solution of compound A19-2 (150 mg) in dichloromethane (5 mL) were added triethylamine (0.11 mL), chloro{[(1R,2R)-(-)-2-amino-1,2-diphenylethyl](4-toluenesulfonyl)amino}(p-cymene)ruthenium(II) (20 mg), and formic acid (50 mg), and the mixture was stirred at room temperature overnight. The reaction liquid was diluted with dichloromethane, washed with water and saturated sodium chloride aqueous solution, dried, and concentrated, and purified by column chromatography (EA/PE = 1 : 3) to obtain the target compound A19 (90 mg).

ESI-MS m/z: 458.11 [M+HCOO⁻]⁻.

### Example 15 Synthesis of 2-(1-(3-cyano-4-fluorophenyl)-5,5-difluoro-4-hydroxyl-4,5,6,7-tetrahydro-1H-indol-3-yl)-2,2-difluoroacetamide (compound A20)

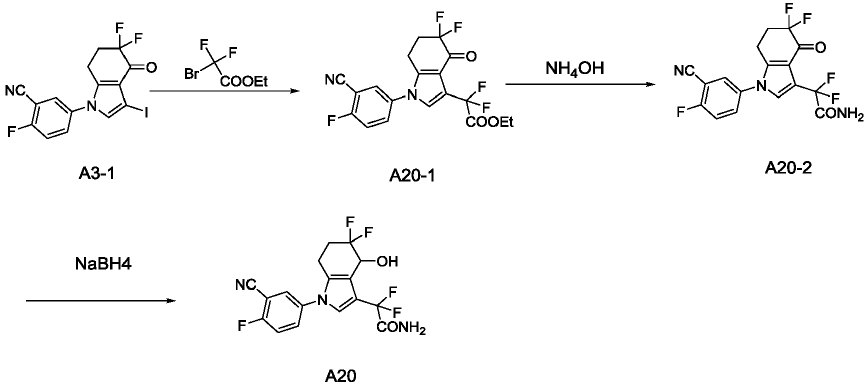

### Step 1: Synthesis of compound A20-1

To a solution of ethyl difluorobromoacetate (100 mg) in dimethyl sulfoxide (1 mL), copper powder (30 mg) and compound A3-1 (100 mg) were added, and the mixture was stirred at 95°C overnight. The mixture was cooled to room temperature, and the reaction liquid was diluted with ethyl acetate, washed with water and saturated sodium chloride aqueous solution, dried, concentrated, and purified by column chromatography to obtain the target compound A20-1 (70 mg).

### Step 2: Synthesis of compound A20-2

To a solution of compound A20-1 (70 mg) in methanol (1 mL) was added 7 mol/L ammonia methanol solution (0.49 mL), and the mixture was stirred at room temperature for 4 hours. The reaction liquid was concentrated to obtain the target compound A20-2, which was used directly in the next step without further purification.

ESI-MS m/z: 384.1 [M+H⁺]⁺.

### Step 3: Synthesis of compound A20

A20-2 (70 mg) was dissolved in methanol (1 mL) and tetrahydrofuran (1 mL). After cooling in an ice-water bath, sodium borohydride (20 mg) was added and the reaction was allowed to react at room temperature overnight. After the reaction was completed, the reaction liquid was diluted with ethyl acetate, washed with water and saturated sodium chloride aqueous solution, dried, concentrated, and purified by column chromatography (EA/PE = 1 : 3) to obtain the target compound A20 (40 mg).

ESI-MS m/z: 430.0 [M+HCOO⁻]⁻.

### Example 16 Synthesis of (S)-3-(ethylsulfonyl)-5,5-difluoro-1-(4-fluoro-3-(trifluoromethyl)phenyl)-4,5,6,7-tetrahydro-1H-indol-4-ol (compound A21)

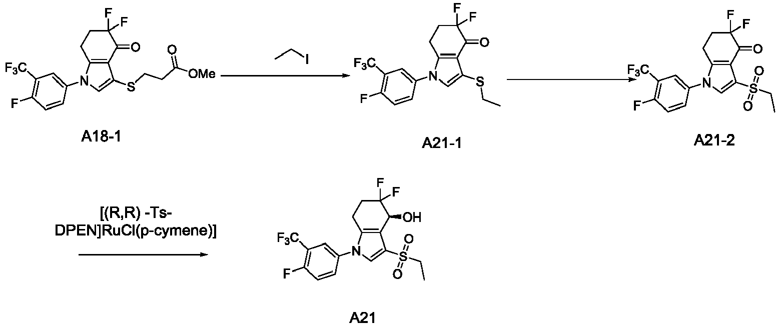

### Step 1: Synthesis of compound A21-1

To a solution of compound A18-1 (180 mg) in tetrahydrofuran (10 mL) was added a 1.0 M solution of potassium tert-butoxide in tetrahydrofuran (0.6 mL) at -78°C. After stirring for 10 minutes, iodoethane (90 mg) was added. The reaction solution was stirred for 2 hours. The reaction liquid was diluted with water and ethyl acetate. The organic phase was washed with saturated sodium chloride aqueous solution, dried, concentrated, and purified by column chromatography (EA/PE = 1 : 3) to obtain the target compound A21-1 (150 mg).

ESI-MS m/z: 394 [M+H⁺]⁺.

### Step 2: Synthesis of compound A21-2

Potassium peroxymonosulfonate (0.7 g) was added to a mixture of compound A21-1 (150 mg), acetonitrile (5 mL) and water (5 mL), and stirred at 60°C for 2 h. After the reaction was completed, the reaction liquid was diluted with ethyl acetate, washed with water and saturated sodium chloride aqueous solution, dried, concentrated, and purified by column chromatography (EA/PE = 1 : 3) to obtain the target compound A21-2 (150 mg).

ESI-MS m/z: 424 [M-H⁺]⁻.

### Step 3: Synthesis of compound A21

To a solution of compound A21-2 (150 mg) in dichloromethane (5 mL) were added triethylamine (0.10 mL), chloro{[(1R,2R)-(-)-2-amino-1,2-diphenylethyl](4-toluenesulfonyl)amino}(p-cymene)ruthenium(II) (20 mg), and formic acid (50 mg), and the mixture was stirred at room temperature overnight. The reaction liquid was diluted with dichloromethane, washed with water and saturated sodium chloride aqueous solution, dried, and concentrated, and purified by column chromatography (EA/PE = 1 : 3) to obtain the target compound A21 (60 mg).

ESI-MS m/z: 472.15 [M+HCOO⁻]⁻.

### Example 17 Synthesis of 5-(3-(cyanodifluoromethyl)-5,5-difluoro-4-hydroxyl-4,5,6,7-tetrahydro-1H-indol-1-yl)-2-fluorobenzonitrile (compound A22)

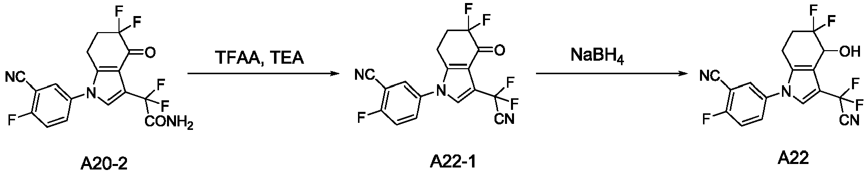

### Step 1: Synthesis of compound A22-1

A solution of compound A20-2 (0.7 g) in dichloromethane (2 mL) was cooled to 0°C, and then triethylamine (0.07 mL) and trifluoroacetic anhydride (0.07 mL) were added. The mixture was stirred at 50°C overnight and concentrated to obtain the target compound A22-1, which was used directly in the next step without further purification.

### Step 2: Synthesis of compound A22

Sodium borohydride (20 mg) was added to a solution of compound A22-1 (60 mg) in methanol (1 mL) and tetrahydrofuran (1 mL), and the mixture was reacted at room temperature overnight. The reaction liquid was diluted with ethyl acetate, washed with water and saturated sodium chloride aqueous solution, dried, concentrated, and purified by column chromatography (EA/PE = 1 : 3) to obtain the target compound A22 (20 mg).

ESI-MS m/z: 412.0 [M+HCOO⁻]⁻.

### Example 18 Synthesis of N-(((S)-1-(3-cyano-4-fluorophenyl)-5,5-difluoro-4-hydroxyl-4,5,6,7-tetrahydro-1H-indol-3-yl)(methyl)(oxo)-16-sulfino)cyanamide (compound A23)

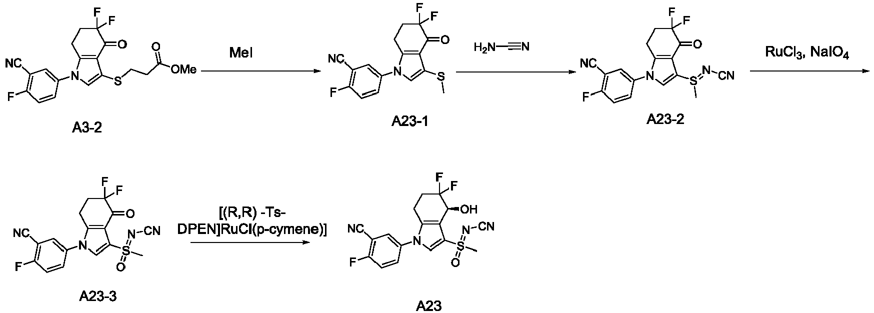

### Step 1: Synthesis of compound A23-1

To a solution of compound A3-2 (180 mg) in tetrahydrofuran (10 mL) was added a 1.0 M solution of potassium tert-butoxide in tetrahydrofuran (0.7 mL) at -78°C. After stirring for 10 minutes, iodomethane (90 mg) was added. The reaction solution was stirred for another 2 hours. The reaction liquid was diluted with water and ethyl acetate. The organic phase was washed with saturated sodium chloride aqueous solution, dried, concentrated, and purified by column chromatography (EA/PE = 1 : 3) to obtain the target compound A23-1 (116 mg).

ESI-MS m/z: 337 [M+H⁺]⁺.

### Step 2: Synthesis of compound A23-2

To a solution of compound A23-1 (200 mg) in acetonitrile (5 mL) were added cyanamide (40 mg) and iodobenzene diacetate (250 mg), and the mixture was stirred at room temperature overnight, and concentrated. The reaction liquid was diluted with ethyl acetate, washed with water and saturated sodium chloride aqueous solution, dried, concentrated, and purified by column chromatography (EA/PE = 1 : 3) to obtain the target compound A23-2 (120 mg).

ESI-MS m/z: 377 [M+H⁺]⁺.

### Step 3: Synthesis of compound A23-3

To a mixture of compound A23-2 (120 mg) dissolved in acetonitrile (2 mL), dichloromethane (2 mL) and water (4 mL), ruthenium trichloride (10 mg) and sodium periodate (370 mg) were added and stirred at room temperature for 4 hours. After the reaction was completed, the reaction liquid was diluted with ethyl acetate, washed with water and saturated sodium chloride aqueous solution, dried, concentrated, and purified by column chromatography (EA/PE = 1 : 3) to obtain the target compound A23-3 (40 mg).

ESI-MS m/z: 393.0[M+H⁺]⁺.

### Step 4: Synthesis of compound A23

To a solution of compound A23-3 (40 mg) in dichloromethane (5 mL) were added chloro{ [(1R,2R)-(-)-2-amino-1,2-diphenylethyl](4-toluenesulfonyl)amino}(p-cymene)ruthenium(II) (10 mg), triethylamine (0.03 mL) and formic acid (14 mg), and the mixture was stirred at room temperature overnight. The reaction liquid was diluted with dichloromethane, washed with water and saturated sodium chloride aqueous solution, dried, concentrated, and purified by column chromatography to obtain the target compound A23 (10 mg).

ESI-MS m/z: 395.11 [M+H⁺]⁺.

### Example 19 Synthesis of (S)-2-((1-(3-(difluoromethyl)-4-fluorophenyl)-5,5-difluoro-4-hydroxyl-4,5,6,7-tetrahydro-1H-indol-3-yl)sulfonyl)acetonitrile (compound A24)

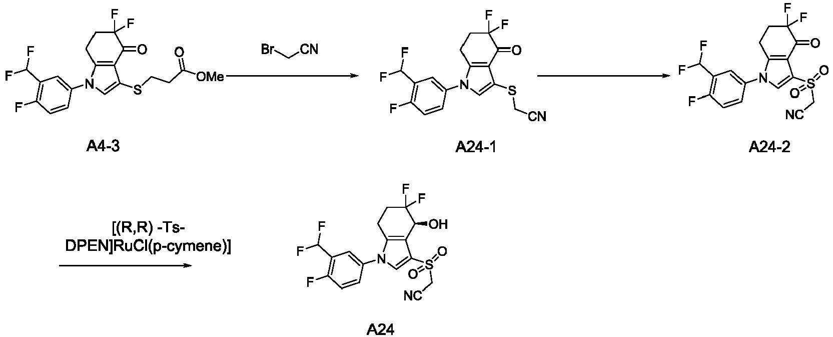

### Step 1: Synthesis of compound A24-1

To a solution of A4-3 (0.86 g) in tetrahydrofuran (20 mL) was added a 1.0 M solution of potassium tert-butoxide in tetrahydrofuran (3.0 mL) at -78°C. After stirring for 10 minutes, bromoacetonitrile (0.36 g) was added. The reaction mixture was stirred for another 2 hours. The reaction liquid was diluted with water and ethyl acetate. The organic phase was washed with saturated sodium chloride aqueous solution, dried, concentrated, and purified by column chromatography to obtain the target compound A24-1 (0.38 g).

ESI-MS m/z: 387 [M+H⁺]⁺.

### Step 2: Synthesis of compound A24-2

Potassium peroxymonosulfonate (3.6 g) was added to a mixture of compound A24-1 (0.38 g) in acetonitrile (6 mL) and water (6 mL), and the mixture was stirred at 60°C overnight. After the reaction was completed, the reaction liquid was diluted with ethyl acetate, washed with water and saturated sodium chloride aqueous solution, dried, concentrated, and purified by column chromatography (EA/PE = 1 : 3) to obtain the target compound A24-2 (0.34 g).

ESI-MS m/z: 417 [M-H⁺]⁻.

### Step 3: Synthesis of compound A24

To a solution of compound A24-2 (0.34 g) in dichloromethane (10 mL) were added triethylamine (0.23 mL), chloro{[(1R,2R)-(-)-2-amino-1,2-diphenylethyl](4-toluenesulfonyl)amino}(P-cymene)ruthenium(II) (0.08 g) and formic acid (0.11 g), and the mixture was stirred at room temperature overnight. The reaction liquid was diluted with dichloromethane, washed with water and saturated sodium chloride aqueous solution, dried, concentrated, and purified by column chromatography (EA/PE = 1 : 3) to obtain the target compound A24 (0.18 g).

ESI-MS m/z: 419 [M-H⁻]⁻.

The following examples were prepared using methods substantially similar to those of examples 1-19.

| **Example number** | **Structural formula** | **Compound name** | **ESI-MS** |
|---|---|---|---|
| 20 | 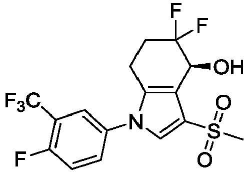 | (*S*)-5,5-Difluoro-1-(4-fluoro-3-(trifluoromethyl)phenyl)-3-(methylsulfonyl)-4,5,6,7-tetrahydro-1*H*-indol-4-ol | [M+HCOO⁻]⁻: 458 |
| 21 | 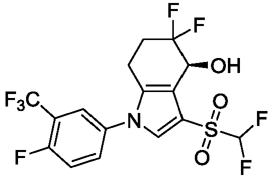 | (*S*)-3-((Difluoromethyl)sulfonyl)-5,5-difluoro-1-(4-fluoro-3-(trifluoromethyl)phenyl)-4,5,6,7-tetrahydro-1*H*-indol-4-ol | [M+HCOO⁻]⁻: 494 |
| 22 | 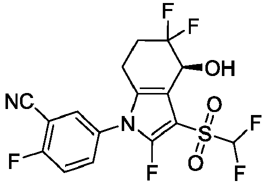 | (*S*)-5-(3-((Difluoromethyl)sulfonyl)-2,5,5-trifluoroether-4-hydroxyl-4,5,6,7-tetrahydro-1*H*-indol-1-yl)-2-fluorobenzonitrile | [M+HCOO⁻]⁻: 469 |
| 23 | 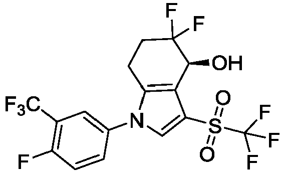 | (*S*)-5,5-Difluoro-1-(4-fluoro-3-(trifluoromethyl)phenyl)-3-((trifluoromethyl)sulfonyl)-4,5,6,7-tetrahydro-1*H*-indol-4-ol | [M+HCOO⁻]⁻: 512 |
| 24 | 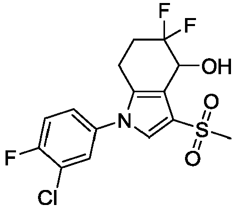 | 1-(3-Chloro-4-fluorophenyl)-5,5-difluoro-3-(methylsulfonyl)-4,5,6,7-tetrahydro-1*H*-indol-4-ol | [M+HCOO⁻]⁻: 424 |
| 25 | 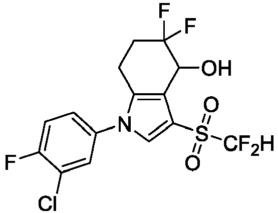 | 1-(3-Chloro-4-fluorophenyl)-3-((difluoromethyl)sulfonyl)-5,5-difluoro-4,5,6,7-tetrahydro-1*H-*indol-4-ol | [M+HCOO⁻]⁻: 460 |
| 26 | 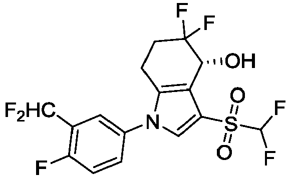 | (*R*)-1-(3-(Difluoromethyl)-4-fluorophenyl)-3-((difluoromethyl)sulfonyl)-5,5-difluoro-4,5,6,7-tetrahydro-1*H-*indol-4-ol | [M+HCOO⁻]⁻: 476 |
| 27 | 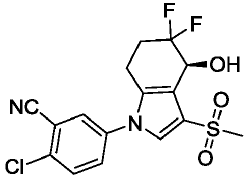 | (*S*)-2-Chloro-5-(5,5-difluoro-4-hydroxyl-3-(methylsulfonyl)-4,5,6,7-tetrahydro-1*H*-indol-1-yl)benzonitrile | [M+HCOO⁻]⁻: 431 |
| 28 | 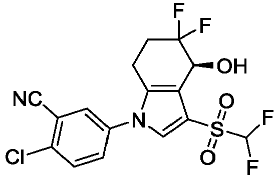 | (*S*)-2-Chloro-5-(3-((difluoromethyl)sulfonyl)-5,5-difluoro-4-hydroxyl-4,5,6,7-tetrahydro-1*H*-indol-1-yl)benzonitrile | [M+HCOO⁻]⁻: 467 |
| 29 | 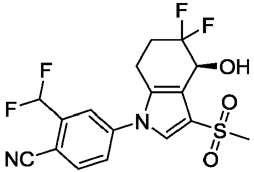 | (*S*)-4-(5,5-Difluoro-4-hydroxyl-3-(methylsulfonyl)-4,5,6,7-tetrahydro-1*H*-indol-1-yl)-2-(difluoromethyl)benzonitrile | [M+HCOO⁻]⁻: 447 |
| 30 | 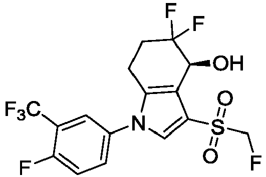 | (*S*)-5,5-Difluoro-1-(4-fluoro-3-(trifluoromethyl)phenyl)-3-((fluoromethyl)sulfonyl)-4,5,6,7-tetrahydro-1*H*-indol-4-ol | [M+HCOO⁻]⁻: 476 |
| 31 | 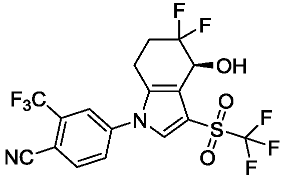 | (*S*)-4-(5,5-Difluoro-4-hydroxyl-3-((trifluoromethyl)sulfonyl)-4,5,6,7-tetrahydro-1*H*-indol-1-yl)-2-(trifluoromethyl)benzonitrile | [M+HCOO⁻]⁻: 519 |
| 32 | 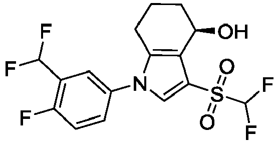 | (*R*)-1-(3-(Difluoromethyl)-4-fluorophenyl)-3-((difluoromethyl)sulfonyl)-4,5,6,7-tetrahydro-1*H*-indol-4-ol | [M+HCOO⁻]⁻: 440 |
| 33 | 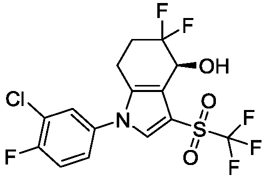 | (*S*)-1-(3-Chloro-4-fluorophenyl)-5,5-difluoro-3-((trifluoromethyl)sulfonyl)-4,5,6,7-tetrahydro-1*H*-indol-4-ol | [M+HCOO⁻]⁻: 478 |
| 34 | 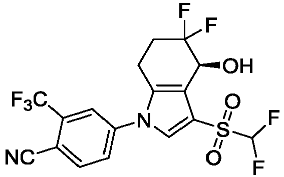 | (*S*)-4-(3-((Difluoromethyl)sulfonyl)-5,5-difluoro-4-hydroxyl-4,5,6,7-tetrahydro-1*H*-indol-1-yl)-2-(trifluoromethyl)benzonitrile | [M+HCOO⁻]⁻: 501 |
| 35 | 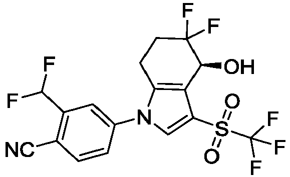 | (*S*)-4-(5,5-Difluoro-4-hydroxyl-3-((trifluoromethyl)sulfonyl)-4,5,6,7-tetrahydro-1*H*-indol-1-yl)-2-(difluoromethyl)benzonitrile | [M+HCOO⁻]⁻: 501 |
| 36 | 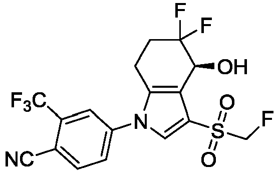 | (*S*)-4-(5,5-Difluoro-3-((fluoromethyl)sulfonyl)-4-hydroxyl-4,5,6,7-tetrahydro-1*H*-indol-1-yl)-2-(trifluoromethyl)benzonitrile | [M+HCOO⁻]⁻: 483 |
| 37 | 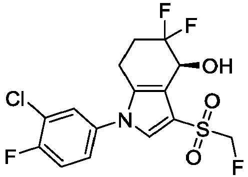 | (*S*)-1-(3-Chloro-4-fluorophenyl)-5,5-difluoro-3-((fluoromethyl)sulfonyl)-4,5,6,7-tetrahydro-1*H*-indol-4-ol | [M+HCOO⁻]⁻: 442 |
| 38 | 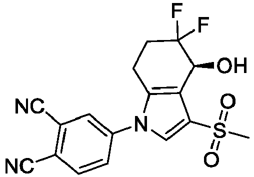 | (*S*)-4-(5,5-Difluoro-4-hydroxyl-3-(methylsulfonyl)-4,5,6,7-tetrahydro-1*H*-indol-1-yl)phthalonitrile | [M+HCOO⁻]⁻: 422 |
| 39 | 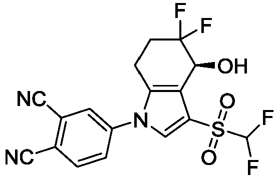 | (*S*)-4-(3-((Difluoromethyl)sulfonyl)-5,5-difluoro-4-hydroxyl-4,5,6,7-tetrahydro-1*H*-indol-1-yl)phthalonitrile | [M+HCOO⁻]⁻: 458 |
| 40 | 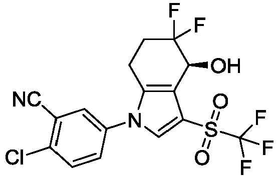 | (*S*)-2-Chloro-5-(5,5-difluoro-4-hydroxyl-3-((trifluoromethyl)sulfonyl)-4,5,6,7-tetrahydro-1*H*-indol-1-yl)benzonitrile | [M+HCOO⁻]⁻: 485 |
| 41 | 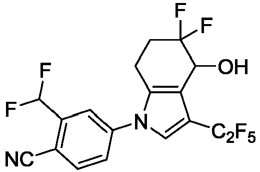 | 4-(5,5-Difluoro-4-hydroxyl-3-(perfluoroethyl)-4,5,6,7-tetrahydro-1*H*-indol-1-yl)-2-(difluoromethyl)benzonitrile | [M+HCOO⁻]⁻: 487 |
| 42 | 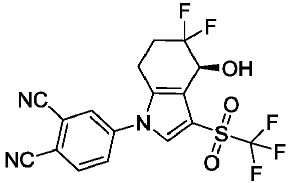 | (*S*)-4-(5,5-Difluoro-4-hydroxyl-3-((trifluoromethyl)sulfonyl)-4,5,6,7-tetrahydro-1*H*-indol-1-yl)phthalonitrile | [M+HCOO⁻]⁻: 476 |
| 43 | 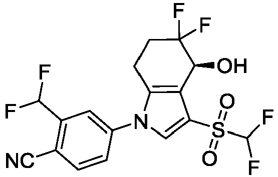 | (*S*)-2-(Difluoromethyl)-4-(3-((difluoromethyl)sulfonyl)-5,5-difluoro-4-hydroxyl-4,5,6,7-tetrahydro-1*H*-indol-1-yl)benzonitrile | [M+H⁺]⁺: 483 |
| 44 | 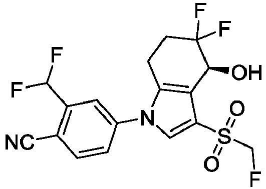 | (*S*)-4-(5,5-Difluoro-3-((fluoromethyl)sulfonyl)-4-hydroxyl-4,5,6,7-tetrahydro-1*H*-indol-1-yl)-2-(difluoromethyl)benzonitrile | [M+HCOO⁻]⁻: 465 |
| 45 | 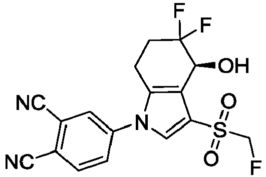 | (*S*)-4-(5,5-Difluoro-3-((fluoromethyl)sulfonyl)-4-hydroxyl-4,5,6,7-tetrahydro-1*H*-indol-1-yl)phthalonitrile | [M+HCOO⁻]⁻: 440 |
| 46 | 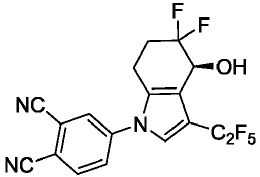 | (*S*)-4-(5,5-Difluoro-4-hydroxyl-3-(perfluoroethyl)-4,5,6,7-tetrahydro-1*H*-indol-1-yl)phthalonitrile | [M+HCOO⁻]⁻: 462 |
| 47 | 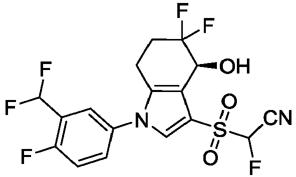 | 2-(((*S*)-1-(3-(Difluoromethyl)-4-fluorophenyl)-5,5-difluoro-4-hydroxyl-4,5,6,7-tetrahydro-1*H*-indol-3-yl)sulfonyl)-2-fluoroacetonitrile | [M-H⁻]⁻: 437 |
| 48 | 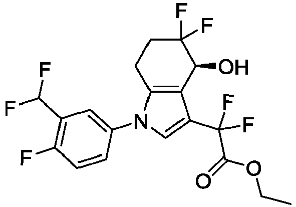 | Ethyl (*S*)-2-(1-(3-(difluoromethyl)-4-fluorophenyl)-5,5-difluoro-4-hydroxyl-4,5,6,7-tetrahydro-1*H*-indol-3-yl)-2,2-difluoroacetate | [M+HCOO⁻]⁻: 484 |
| 49 | 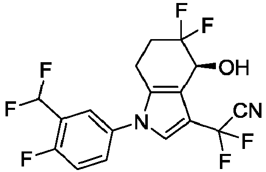 | (*S*)-2-(1-(3-(Difluoromethyl)-4-fluorophenyl)-5,5-difluoro-4-hydroxyl-4,5,6,7-tetrahydro-1*H*-indol-3-yl)-2,2-difluoroacetonitrile | [M+HCOO⁻]⁻: 437 |
| 50 | 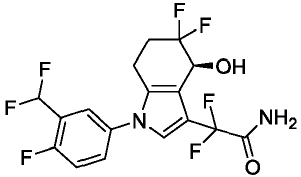 | (*S*)-2-(1-(3-(Difluoromethyl)-4-fluorophenyl)-5,5-difluoro-4-hydroxyl-4,5,6,7-tetrahydro-1*H*-indol-3-yl)-2,2-difluoroacetamide | [M-H⁻]⁻: 409 |
| 51 | 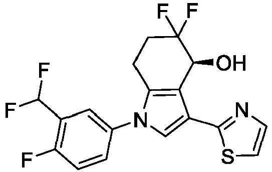 | (*S*)-1-(3-(Difluoromethyl)-4-fluorophenyl)-5,5-difluoro-3-(thiazol-2-yl)-4,5,6,7-tetrahydro-1*H*-indol-4-ol | [M+H⁺]⁺: 401 |
| 52 | 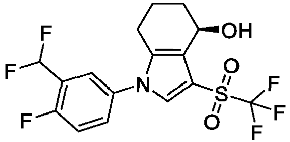 | (*R*)-1-(3-(Difluoromethyl)-4-fluorophenyl)-3-((trifluoromethyl)sulfonyl)-4,5,6,7-tetrahydro-1*H*-indol-4-ol | [M+H⁺-H₂O]⁺: 396 |
| 53 | 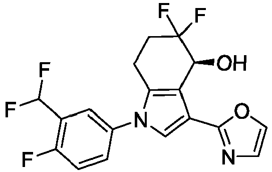 | (*S*)-1-(3-(Difluoromethyl)-4-fluorophenyl)-5,5-difluoro-3-(oxazol-2-yl)-4,5,6,7-tetrahydro-1*H*-indol-4-ol | [M+H⁺]⁺: 401 |
| 54 | 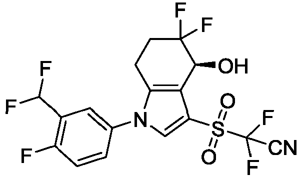 | (*S*)-2-((1-(3-(Difluoromethyl)-4-fluorophenyl)-5,5-difluoro-4-hydroxyl-4,5,6,7-tetrahydro-1*H*-indol-3-yl)sulfonyl)-2,2-difluoroacetonitrile | [M-H⁺]⁻: 455 |
| 55 | 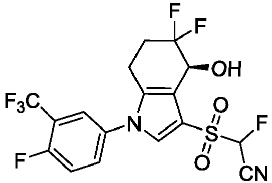 | 2-(((*S*)-5,5-Difluoro-1-(4-fluoro-3-(trifluoromethyl)phenyl)-4-hydroxyl-4,5,6,7-tetrahydro-1*H*-indol-3-yl)sulfonyl)-2-fluoroacetonitrile | [M-H⁺]⁻: 455 |
| 56 | 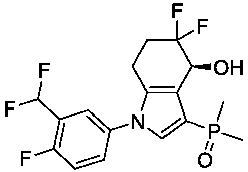 | (*S*)-(1-(3-(Difluoromethyl)-4-fluorophenyl)-5,5-difluoro-4-hydroxyl-4,5,6,7-tetrahydro-1*H*-indol-3-yl) dimethylphosphine oxide methyl | [M+H⁺]⁺: 394 |
| 57 | 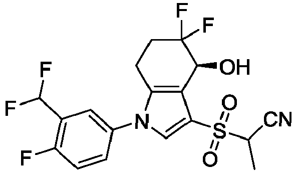 | 2-(((*S*)-1-(3-(Difluoromethyl)-4-fluorophenyl)-5,5-difluoro-4-hydroxyl-4,5,6,7-tetrahydro-1*H*-indol-3-yl)sulfonyl) propanenitrile | [M-H⁺]⁻: 433 |
| 58 | 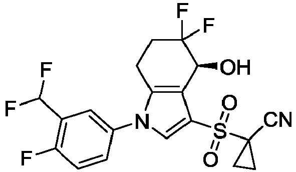 | (*S*)-1-((1-(3-(Difluoromethyl)-4-fluorophenyl)-5,5-difluoro-4-hydroxyl-4,5,6,7-tetrahydro-1*H*-indol-3-yl)sulfonyl) cyclopropane-1-nitrile | [M+HCOO⁻]⁻: 491 |
| 59 | 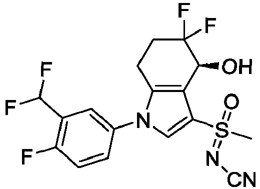 | *N*-(((*S*)-1-(3-(Difluoromethyl)-4-fluorophenyl)-5,5-difluoro-4-hydroxyl-4,5,6,7-tetrahydro-1*H*-indol-3-yl)(methyl)(oxo)-16-sulfino)cyanamide | [M+H⁺]⁺: 420 |
| 60 | 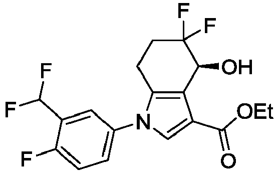 | Ethyl (*S*)-1-(3-(difluoromethyl)-4-fluorophenyl)-5,5-difluoro-4-hydroxyl-4,5,6,7-tetrahydro-1*H*-indole -3-carboxylate | [M+H⁺-H₂O]⁺: 372 |
| 61 | 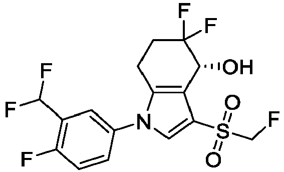 | (*R*)-1-(3-(Difluoromethyl)-4-fluorophenyl)-5,5-difluoro-3-((fluoromethyl)sulfonyl)-4,5,6,7-tetrahydro-1*H*-indol-4-ol | [M+HCOO⁻]⁻: 458 |
| 62 | 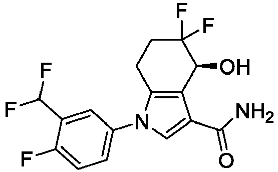 | (*S*)-1-(3-(Difluoromethyl)-4-fluorophenyl)-5,5-difluoro-4-hydroxyl-4,5,6,7-tetrahydro-1*H*-indole -3-carboxamide | [M+H⁺-H₂O]⁺: 343 |
| 63 | 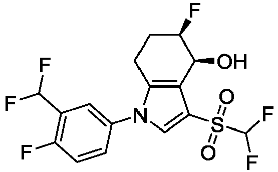 | (4*S*,5*R*)-1-(3-(Difluoromethyl)-4-fluorophenyl)-3-((difluoromethyl)sulfonyl)-5-fluoro-4,5,6,7-tetrahydro-1*H-*indol-4-ol | [M+HCOO⁻]⁻: 458 |
| 64 | 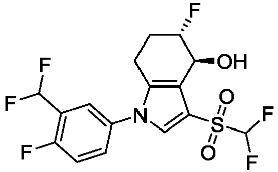 | (4*S*,5*S*)-1-(3-(Difluoromethyl)-4-fluorophenyl)-3-((difluoromethyl)sulfonyl)-5-fluoro-4,5,6,7-tetrahydro-1*H-*indol-4-ol | [M+HCOO⁻]⁻: 458 |
| 65 | 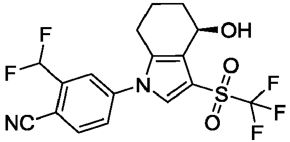 | (*R*)-2-(Difluoromethyl)-4-(4-hydroxyl-3-((trifluoromethyl)sulfonyl)-4,5,6,7-tetrahydro-1*H*-indol-1-yl)benzonitrile | [M+HCOO⁻]⁻: 458 |
| 66 | 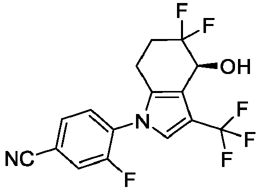 | (*S*)-4-(5,5-Difluoro-4-hydroxyl-3-(trifluoromethyl)-4,5,6,7-tetrahydro-1*H*-indol-1-yl)-3-fluorobenzonitrile | [M+HCOO⁻]⁻: 405 |
| 67 | 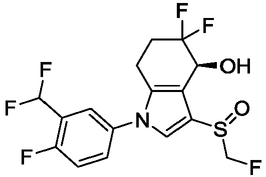 | (4*S*)-1-(3-(Difluoromethyl)-4-fluorophenyl)-5,5-difluoro-3-((fluoromethyl)sulfinyl)-4,5,6,7-tetrahydro-1*H*-indol-4-ol | [M+HCOO⁻]⁻: 442 |
| 68 | 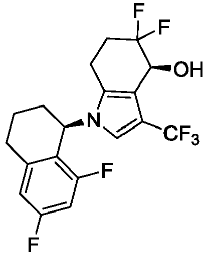 | (*S*)-1-((R)-6,8-Difluoro-1,2,3,4-tetrahydronaphthalen-1-yl-yl)-5,5-difluoro-3-(trifluoromethyl)-4,5,6,7-tetrahydro-1*H*-indol-4-ol | [M+H⁺-H₂O]⁺: 390 |
| 69 | 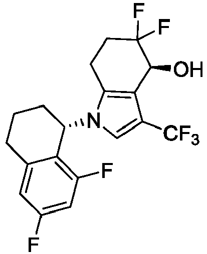 | (*S*)-1-((*S*)-6,8-Difluoro-1,2,3,4-tetrahydronaphthalen-1-yl-yl)-5,5-difluoro-3-(trifluoromethyl)-4,5,6,7-tetrahydro-1*H*-indol-4-ol | [M+H⁺-H₂O]⁺: 390 |
| 70 | 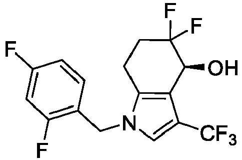 | (*S*)-1-(2,4-Difluorobenzyl)-5,5-difluoro-3-(trifluoromethyl)-4,5,6,7-tetrahydro-1*H*-indol-4-ol | [M+H⁺-H₂O]⁺: 350 |
| 71 | 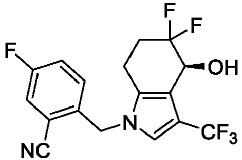 | (*S*)-2-((5,5-Difluoro-4-hydroxyl-3-(trifluoromethyl)-4,5,6,7-tetrahydro-1*H*-indol-1-yl)methyl) -5 -fluorobenzonitrile | [M+H⁺-H₂O]⁺: 357 |
| 72 | 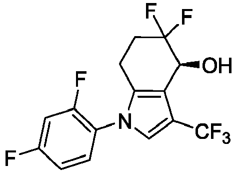 | (*S*)-1-(2,4-Difluorophenyl)-5,5-difluoro-3-(trifluoromethyl)-4,5,6,7-tetrahydro-1*H*-indol-4-ol | [M+H⁺-H₂O]⁺: 336 |
| 73 | 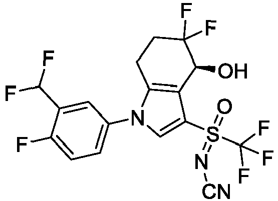 | *N*-(((*S*)-1-(3-(Difluoromethyl)-4-fluorophenyl)-5,5-difluoro-4-hydroxyl-4,5,6,7-tetrahydro-1*H*-indol-3-yl)(oxy)(trifluoromethyl)-16-sulfino)cyanamide | [M+H⁺-H₂O]⁺: 456 |
| 74 | 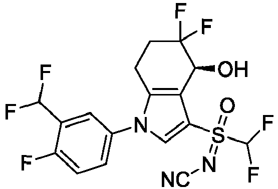 | *N*-((Difluoromethyl)((*S*)-1-(3-(difluoromethyl)-4-fluorophenyl)-5,5-difluoro-4-hydroxyl-4,5,6,7-tetrahydro-1*H*-indol-3-yl)(oxo)-16-sulfino)cyanamide | [M+H⁺-H₂O]⁺: 438 |
| 75 | 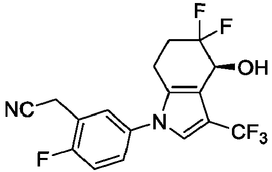 | (*S*)-2-(5-(5,5-Difluoro-4-hydroxyl-3-(trifluoromethyl)-4,5,6,7-tetrahydro-1*H*-indol-1-yl)-2-fluorophenyl)acetonitrile | [M+H⁺-H₂O]⁺: 357 |
| 76 | 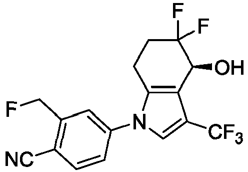 | (*S*)-4-(5,5-Difluoro-4-hydroxyl-3-(trifluoromethyl)-4,5,6,7-tetrahydro-1*H*-indol-1-yl)-2-(fluoromethyl)benzonitrile | [M+H⁺-H₂O]⁺: 357 |
| 77 | 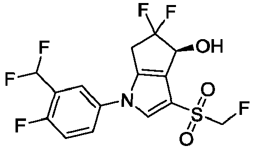 | (*S*)-1-(3-(Difluoromethyl)-4-fluorophenyl)-5,5-difluoro-3-((fluoromethyl)sulfonyl)-1,4,5,6-tetrahydrocyclopentadiene[*b*]py rrol-4-ol | [M+H⁺-H₂O]⁺: 382 |
| 78 | 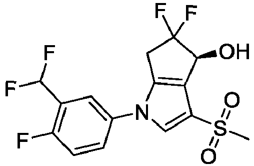 | (*S*)-1-(3-(Difluoromethyl)-4-fluorophenyl)-5,5-difluoro-3-(methylsulfonyl)-1,4,5,6-tetrahydrocyclopentadiene[*b*] pyrrol-4-ol | [M+H⁺-H₂O]⁺: 364 |
| 79 | 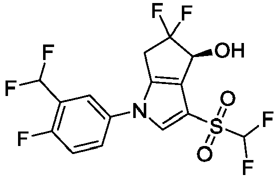 | (*S*)-1-(3-(Difluoromethyl)-4-fluorophenyl)-3-((difluoromethyl)sulfonyl)-5,5-difluoro-1,4,5,6-tetrahydrocyclopentadiene[*b*] pyrrol-4-ol | [M+H⁺-H₂O]⁺: 400 |
| 80 | 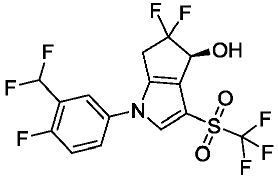 | (*S*)-1-(3-(Difluoromethyl)-4-fluorophenyl)-5,5-difluoro-3-((trifluoromethyl)sulfonyl)-1,4,5,6-tetrahydrocyclopentadiene[*b*]py rrol-4-ol | [M+H⁺-H₂O]⁺: 418 |
| 81 | 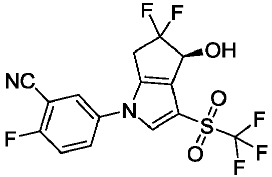 | (*S*)-5-(5,5-Difluoro-4-hydroxyl-3-((trifluoromethyl)sulfonyl)-5,6-dihydrocyclopentadiene[b]pyrr ol-1(4*H*)-yl)-yl2-fluorobenzonitrile | [M+H⁺-H₂O]⁺: 393 |
| 82 | 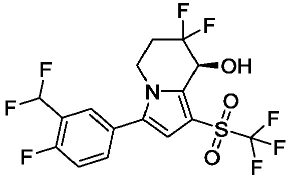 | (*S*)-3-(3-(Difluoromethyl)-4-fluorophenyl)-7,7-difluoro-1-((trifluoromethyl)sulfonyl)-5,6,7,8-tetrahydroindol-8-ol | [M+H⁺-H₂O]⁺: 432 |
| 83 | 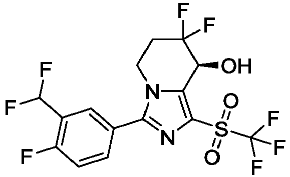 | (*S*)-3-(3-(Difluoromethyl)-4-fluorophenyl)-7,7-difluoro-1-((trifluoromethyl)sulfonyl)-5,6,7,8-tetrahydroimidazo[1,5-*a*]pyridin-8-ol | [M+H⁺]⁺: 451 |
| 84 | 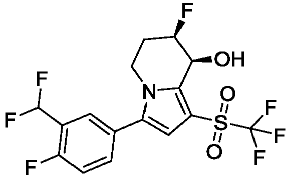 | (7*R*,8*S*)-3-(3-(Difluoromethyl)-4-fluorophenyl)-7-fluoro-1-((trifluoromethyl)sulfonyl)-5,6,7,8-tetrahydroindolizin-8-ol | [M+H⁺-H₂O]⁺: 414 |
| 85 | 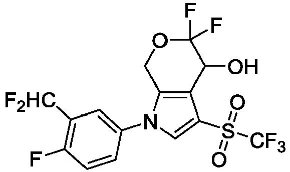 | 1-(3-(Difluoromethyl)-4-fluorophenyl)-5,5-difluoro-3-((trifluoromethyl)sulfonyl)-1,4,5,7-tetrahydropyran[3,4-*b*]pyrrol-4-ol | [M+H⁺-H₂O]⁺: 434 |
| 86 | 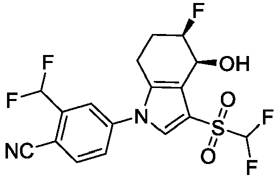 | 2-(Difluoromethyl)-4-((4*S*,5*R*)-3-((difluoromethyl)sulfonyl)-5-fluoro-4-hydroxyl-4,5,6,7-tetrahydro-1*H*-indol-1-yl)benzonitrile | [M+H⁺-H₂O]⁺: 403 |
| 87 | 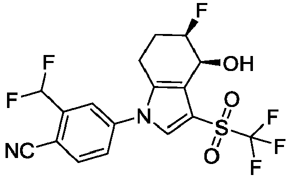 | 2-(Difluoromethyl)-4-((4*S*,5*R*)-5-fluoro-4-hydroxyl-3-((trifluoromethyl)sulfonyl)-4,5,6,7-tetrahydro-1*H*-indol-1-yl)benzonitrile | M+HCOO⁻]⁺: 451.11 |
| 88 | 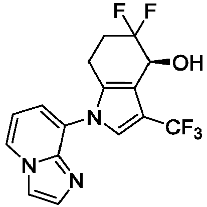 | (*S*)-5,5-Difluoro-1-(imidazo[1,2-*a*]pyridin-8-yl)-3-(trifluoromethyl)-4,5,6,7-tetrahydro-1*H*-indol-4-ol | [M+H⁺]⁺: 358 |
| 89 | 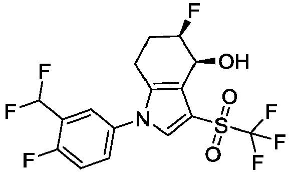 | (4*S*,5*R*)-1-(3-(Difluoromethyl)-4-fluorophenyl)-5-fluoro-3-((trifluoromethyl)sulfonyl)-4,5,6,7-tetrahydro-1*H*-indol-4-ol | [M+H⁺-H₂O]⁺: 414 |
| 90 | 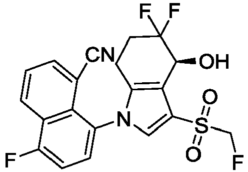 | (*S*)-8-(5,5-Difluoro-3-((fluoromethyl)sulfonyl)-4-hydroxyl-4,5,6,7-tetrahydro-1*H*-indol-1-yl)- 5-fluoro-1-naphthonitrile | [M+H⁺-H₂O]⁺: 421 |
| 91 | 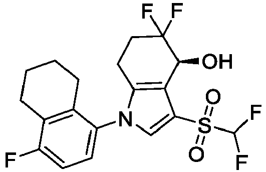 | (*S*)-3-((Difluoromethyl)sulfonyl)-5,5-difluoro-1-(4-fluoro-5,6,7,8-tetrahydronaphthalen-1-yl)-4,5,6,7-tetrahydro-1*H*-indol-4-ol | [M+H⁺-H₂O]⁺: 418 |
| 92 | 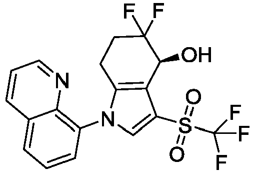 | (*S*)-5,5-Difluoro-1-(quinolin-8-yl)-3-((trifluoromethyl)sulfonyl)-4,5,6,7-tetrahydro-1*H*-indol-4-ol | [M+H⁺]⁺: 433 |
| 93 | 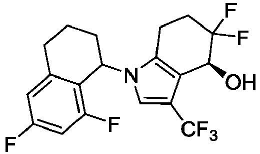 | (4*S*)-1-(6,8-Difluoro-1,2,3,4-tetrahydronaphthalen-1-yl)-5,5-difluoro-3-(trifluoromethyl)-4,5,6,7-tetrahydro-1*H*-indol-4-ol | [M+H⁺-H₂O]⁺: 390 |
| 94 | 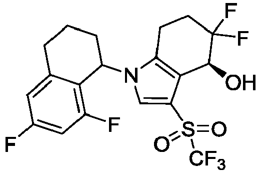 | (4*S*)-1-(6,8-Difluoro-1,2,3,4-tetrahydronaphthalen-1-yl)-5,5-difluoro-3-((trifluoromethyl)sulfonyl)-4,5,6,7-tetrahydro-1*H*-indol-4-ol | [M+H⁺-H₂O]⁺: 454 |
| 95 | 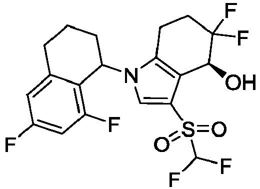 | (4*S*)-1-(6,8-Difluoro-1,2,3,4-tetrahydronaphthalen-1-yl)-3-((difluoromethyl)sulfonyl)-5,5-difluoro-4,5,6,7-tetrahydro-1*H-*indol-4-ol | [M+H⁺-H₂O]⁺: 436 |
| 96 | 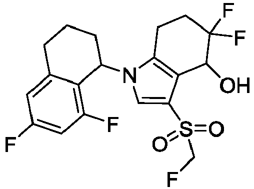 | 1-(6,8-Difluoro-1,2,3,4-tetrahydronaphthalen-1-yl)-5,5-difluoro-3-((fluoromethyl)sulfonyl)-4,5,6,7-tetrahydro-1*H*-indol-4-ol | [M+H⁺-H₂O]⁺: 418 |
| 97 | 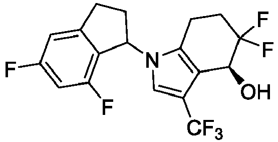 | (4*S*)-1-(5,7Difluoro-2,3-dihydro-1*H*-inden-1-yl)-5,5-difluoro-3-(trifluoromethyl)-4,5,6,7-tetrahydro-1*H*-indol-4-ol | [M+H⁺-H₂O]⁺: 376 |
| 98 | 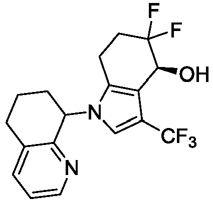 | (4*S*)-5,5-Difluoro-1-(5,6,7,8-tetrahydroquinolin-8-yl)-3-(trifluoromethyl)-4,5,6,7-tetrahydro-1*H*-indol-4-ol | [M+H⁺-H₂O]⁺: 355 |
| 99 | 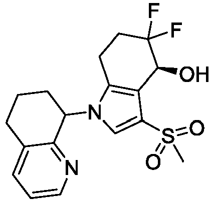 | (4*S*)-5,5-Difluoro-3-(methylsulfonyl)-1-(5,6,7,8-tetrahydroquinolin-8-yl)-4,5,6,7-tetrahydro-1*H*-indol-4-ol | [M+H⁺-H₂O]⁺: 365 |
| 100 | 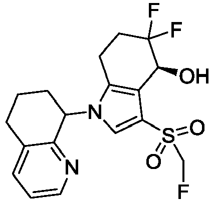 | 5,5-Difluoro-3-((fluoromethyl)sulfonyl)-1-(5,6,7,8-tetrahydroquinolin-8-yl)- 4,5,6,7-tetrahydro-1*H-*indol-4-ol | [M+H⁺-H₂O]⁺: 383 |
| 101 | 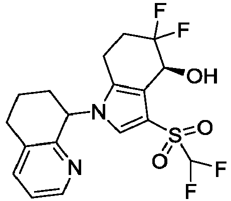 | (4*S*)-3-((Difluoromethyl)sulfonyl)-5,5-difluoro-1-(5,6,7,8-tetrahydroquinolin-8-yl)-4,5,6,7-tetrahydro-1*H*-indol-4-ol | [M+H⁺-H₂O]⁺: 401 |
| 102 | 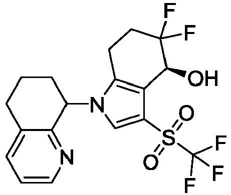 | (4*S*)-5,5-Difluoro-1-(5,6,7,8-tetrahydroquinolin-8-yl)-3-((trifluoromethyl)sulfonyl)-4,5,6,7-tetrahydro-1*H*-indol-4-ol | [M+H⁺-H₂O]⁺: 419 |
| 103 | 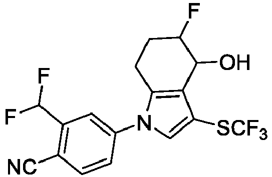 | 2-(Difluoromethyl)-4-(5-fluoro-4-hydroxyl-3-((trifluoromethyl)thio)- 4,5,6,7-tetrahydro-1*H*-indol-1-yl)benzonitrile | [M+HCOO⁻]⁻: 451.1 |
| 104 | 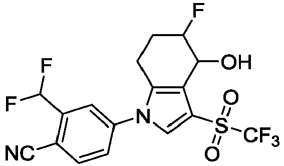 | 2-(Difluoromethyl)-4-(5-fluoro-4-hydroxyl-3-((trifluoromethyl)sulfonyl)-4,5,6,7-tetrahydro-1*H*-indol-1-yl)benzonitrile | [M+HCOO⁻]⁻: 483.1 |
| 105 | 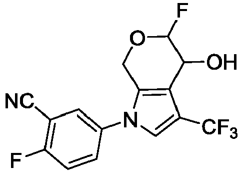 | 2-Fluoro-5-(5-fluoro-4-hydroxyl-3-(trifluoromethyl)-4,7-dihydropyran[3,4-*b*]pyrrol-1(5*H*)-yl)benzonitrile | [M+HCOO⁻]⁻: 389.1 |
| 106 | 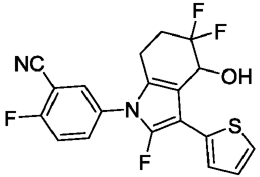 | 2-Fluoro-5-(2,5,5-trifluoro-4-hydroxyl-3-(thien-2-yl)-4,5,6,7-tetrahydro-1*H*-indol-1-yl)benzonitrile | [M+HCOO⁻]⁻: 437.1 |
| 107 | 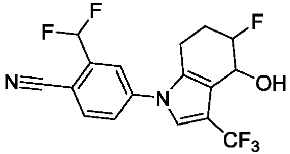 | 2-(Difluoromethyl)-4-(5-fluoro-4-hydroxyl-3-(trifluoromethyl)-4,5,6,7-tetrahydro-1*H*-indol-1-yl)benzonitrile | [M+HCOO⁻]⁻: 419.1 |

**The ¹H NMR data for some of the exemplary compounds are as shown below:**
¹H NMR (500 MHz, CDCl₃) δ 7.91 (d, *J* = 8.3 Hz, 1H), 7.71 (s, 1H), 7.56 (d, *J* = 8.2 Hz, 1H), 7.21 (s, 1H), 7.00 (dd, *J* = 70.0, 38.6 Hz, 1H), 4.93 (t, *J* = 6.2 Hz, 1H), 2.92 - 2.81 (m, 1H), 2.74 - 2.64 (m, 1H), 2.60 - 2.45 (m, 1H), 2.30 - 2.20 (m, 1H). (Example 3)
¹H NMR (500 MHz, CDCl₃) δ 7.67 (dd, *J* = 5.2, 2.7 Hz, 1H), 7.62 (ddd, *J* = 8.9, 4.4, 2.8 Hz, 1H), 7.50 (s, 1H), 7.46 - 7.40 (m, 1H), 6.39 (t, *J* = 54.0 Hz, 1H), 5.06 (t, *J* = 6.3 Hz, 1H), 3.11 (s, 1H), 2.83 - 2.70 (m, 1H), 2.62 (ddd, *J* = 16.3, 6.3, 2.3 Hz, 1H), 2.55 - 2.38 (m, 1H), 2.31 - 2.19 (m, 1H). (Example 4)
¹H NMR (500 MHz, DMSO) δ 7.85 - 7.75 (m, 2H), 7.65 (s, 1H), 7.57 (t, *J* = 9.2 Hz, 1H), 7.23 (t, *J* = 53.9 Hz, 1H), 6.35 (d, *J* = 6.0 Hz, 1H), 4.85 - 4.80 (m, 1H), 3.24 (s, 3H), 2.80 - 2.70 (m, 1H), 2.70 - 2.59 (m, 1H), 2.35 - 2.25 (m, 1H), 2.20 - 2.10 (m, 1H). (Example 5)
¹H NMR (500 MHz, CDCl₃) δ 7.62 (dd, *J* = 5.7, 2.7 Hz, 1H), 7.58 (d, *J* = 9.2 Hz, 1H), 7.52 - 7.44 (m, 1H), 7.35 (t, *J* = 8.9 Hz, 1H), 7.08 - 6.78 (m, 1H), 5.03 (dt, *J* = 7.5, 3.7 Hz, 1H), 3.02 (d, *J* = 7.4 Hz, 1H), 2.85 - 2.73 (m, 1H), 2.67 - 2.43 (m, 2H), 2.35 - 2.18 (m, 1H). (Example 12)
¹H NMR (500 MHz, CDCl₃) δ 7.60 (dd, *J* = 5.8, 2.7 Hz, 1H), 7.49 - 7.43 (m, 2H), 7.32 (t, *J* = 8.9 Hz, 1H), 6.94 (dd, *J* = 59.6, 49.5 Hz, 1H), 5.47 (dd, *J* = 47.2, 9.8 Hz, 1H), 5.25 - 5.07 (m, 2H), 3.00 (d, *J* = 3.4 Hz, 1H), 2.76 (ddd, *J* = 16.5, 10.5, 6.1 Hz, 1H), 2.64 (ddd, *J* = 16.3, 6.2, 2.7 Hz, 1H), 2.55 - 2.37 (m, 1H), 2.31 - 2.16 (m, 1H). (Example 14)
¹H NMR (500 MHz, CDCl₃) δ 7.63 - 7.58 (m, 1H), 7.56 (s, 1H), 7.51 - 7.42 (m, 1H), 7.33 (t, *J* = 8.9 Hz, 1H), 6.94 (t, *J* = 54.5 Hz, 1H), 5.15 (s, 1H), 4.63 (d, *J* = 16.4 Hz, 1H), 4.17 (d, *J* = 16.4 Hz, 1H), 2.90 (s, 1H), 2.83 - 2.61 (m, 2H), 2.53 - 2.16 (m, 2H). (Example 19)
¹H NMR (500 MHz, CDCl₃) δ 7.52 (s, *J* = 6.3 Hz, 1H), 7.28 - 7.24 (m, 1H), 7.19 (d, *J* = 0.9 Hz, 1H), 7.02 (dt, *J* = 8.3, 2.1 Hz, 1H), 6.39 (t, *J* = 54.0 Hz, 1H), 5.05 (t, *J* = 6.4 Hz, 1H), 3.08 (s, 1H), 2.85 - 2.75 (m, 1H), 2.70 (ddd, *J* = 16.4, 6.3, 2.4 Hz, 1H), 2.56 - 2.38 (m, 1H), 2.32 - 2.17 (m, 1H). (Example 23)
¹H NMR (500 MHz, CDCl₃) δ 7.41 (dd, *J* = 6.2, 2.6 Hz, 1H), 7.36 (s, 1H), 7.29 (t, *J* = 8.5 Hz, 1H), 7.21 (ddd, *J* = 8.7, 4.0, 2.7 Hz, 1H), 5.14 (t, *J* = 6.4 Hz, 1H), 3.25 (s, 3H), 3.21 (d, *J* = 2.2 Hz, 1H), 2.80 - 2.59 (m, 2H), 2.55 - 2.35 (m, 1H), 2.28 - 2.19 (m, 1H). (Example 24)
¹H NMR (500 MHz, CDCl₃) δ 7.74 - 7.67 (m, 2H), 7.55 (dd, *J* = 8.6, 2.6 Hz, 1H), 7.51 (s, 1H), 6.38 (t, *J* = 54.0, 1H), 5.10 - 5.02 (m, 1H), 3.11 (d, *J* = 3.4 Hz, 1H), 2.85 - 2.75 (m, 1H), 2.64 (ddd, *J* = 16.3, 6.2, 2.4 Hz, 1H), 2.57 - 2.36 (m, 1H), 2.33 - 2.20 (m, 1H). (Example 28)
¹H NMR (500 MHz, CDCl₃) δ 7.96 (d, *J* = 8.3 Hz, 1H), 7.76 (s, 1H), 7.61 (dd, *J* = 8.3, 2.1 Hz, 1H), 7.47 (s, 1H), 7.03 (t, *J* = 67.7 Hz, 1H), 5.22 - 5.15 (m, 1H), 3.21 (d, J = 3.0 Hz, 1H), 2.92 - 2.82 (m, 1H), 2.73 (ddd, *J* = 16.2, 6.0, 2.9 Hz, 1H), 2.56 - 2.40 (m, 1H), 2.34 - 2.24 (m, 1H). (Example 29)
¹H NMR (500 MHz, CDCl₃) δ 8.00 (d, *J* = 8.3, 1H), 7.81 (d, *J* = 9.4, 1H), 7.79 - 7.68 (m, 1H), 7.53 (s, 1H), 5.51 (dd, *J* = 47.2, 9.9 Hz, 1H), 5.25 - 5.05 (m, 2H), 3.00 (s, 1H), 2.94 - 2.79 (m, 1H), 2.75 - 2. 65 (m, 1H), 2.56 - 2.38 (m, 1H), 2.31 - 2.22 (m, 1H). (Example 45)
¹H NMR (500 MHz, CDCl₃) δ 7.97 (d, *J* = 8.4 Hz, 1H), 7.78 (d, *J* = 2.1 Hz, 1H), 7.71 (dd, *J* = 8.4, 2.2 Hz, 1H), 7.14 (s, 1H), 4.89 (t, *J* = 5.9 Hz, 1H), 2.92 - 2.83 (m, 1H), 2.69 (dd, *J* = 16.1, 6.4 Hz, 1H), 2.63 - 2.42 (m, 2H), 2.34 - 2.17 (m, 1H). (Example 46)

### Pharmacological experiments

### VEGFA ELISA assay (IC₅₀)

786-O cells in the logarithmic growth phase were inoculated into a 96-well plate at a cell concentration of 65,000 cells per ml of culture liquid, 180 µL per well. The compounds were diluted to the corresponding concentrations, and 20 µL of compound solutions of various concentrations were added to the corresponding cell wells, so that the final concentrations of the compounds were 1.5 nM, 4.6 nM, 13.7 nM, 41.2 nM, 123.5 nM, 370.4 nM, 1111.1 nM, 3333.3 nM, and 10000 nM, respectively. After culturing for 24 h, the cell culture supernatant was collected and the VEGFA concentration was determined using an ELISA kit (purchased from Abcam). Finally, the reaction was terminated and the absorbance value of each well was measured at a wavelength of 450 nm using an ELISA reader. The IC₅₀ values were calculated by GraphPadPrism. CellTiter-Glo reagent was used to measure cell viability.

The IC₅₀ data of the exemplary examples are provided in Table 1, where A stands for IC₅₀ ≤ 0.5 µM; B stands for 0.5 µM < IC₅₀ ≤ 1.5µM; C stands for 1.5µM < IC₅₀ ≤ 10µM, and D stands for IC₅₀ > 10µM.

**Table 1**

| **Example number** | **IC₅₀ (µM)** | **Example number** | **IC₅₀ (µM)** |
|---|---|---|---|
| 1 | A | 33 | 0.041 |
| 2 | 0.0083 | 34 | A |
| 3 | 0.003 | 35 | 0.018 |
| 4 | 0.098 | 36 | 0.108 |
| 5 | 0.072 | 37 | A |
| 6 | 0.011 | 39 | 0.140 |
| 9 | 0.047 | 40 | A |
| 10 | A | 41 | A |
| 11 | 0.027 | 42 | 0.074 |
| 12 | 0.010 | 43 | A |
| 13 | B | 44 | A |
| 14 | 0.040 | 45 | A |
| 15 | B | 46 | A |
| 16 | 0.537 | 47 | 0.008 |
| 17 | A | 48 | 1.621 |
| 18 | A | 49 | 0.099 |
| 19 | B | 51 | 1.250 |
| 20 | 0.039 | 52 | 0.046 |
| 21 | 0.012 | 57 | A |
| 22 | 0.086 | 58 | 0.639 |
| 23 | 0.011 | 59 | 0.109 |
| 24 | 0.072 | 61 | A |
| 25 | 0.063 | 62 | A |
| 26 | 0.628 | 63 | 0.050 |
| 27 | A | 64 | 0.033 |
| 28 | 0.118 | 65 | 0.030 |
| 29 | 0.174 | 72 | B |
| 30 | 0.045 | 105 | C |
| 31 | 0.021 | 106 | 0.015 |
| 32 | 0.062 | 107 | 0.034 |

### Luciferase assay

The luciferase LUC gene was stably transfected into 786-O cells (purchased from ATCC) using Lipofectamine 3000 transfection reagent (purchased from Invitrogen) to construct HIF2α reporter gene cells (786-O-HIF2α-Luc cells). The experiment was performed when 786-O-HIF2α-Luc cells were in the logarithmic growth phase, the culture medium (RPMI MEDIUM 1640, purchased from Invitrogen) was discarded, and the cells were rinsed three times with PBS; trypsin (TrypLE, purchased from Invitrogen) was added to digest the cells, and the digestion was terminated with culture medium, 10% fetal bovine serum, 1% penicillin, and streptomycin. The cells were collected by centrifugation, and washed twice with PBS to remove phenol red in the culture medium. The cells were resuspended to an appropriate concentration. The cell density and viability were measured, to ensure that the cells with a viability of at least 90% were used in the assay.

The compounds at a gradient concentration were transferred into 384 wells using Echo550 (noncontact acoustic pipetting system, purchased from Labcyte), 25 nL/well. The cells were seeded into 384-well plates, with 4500 cells/well and 25 µL of culture medium, and the final concentrations of the compounds were 10000 nM, 3333 nM, 1111 nM, 370 nM, 123 nM, 41.1 nM, 13.7 nM, 4.6 nM, 1.5 nM, and 0.5 nM, respectively. The cells were cultured for 18-20 h under conditions of 37°C and 5% CO₂. Steady-Glo^{™} luciferase assay system (purchased from Promega) was added to a 384-well plate, 25 µL/well. Luminescence values were detected using Envision. The inhibition rate% was calculated based on the RLU (Record Luminescence) signal value of each well, and then the IC₅₀ values of the corresponding compounds were calculated by fitting using Graphpad 8.0.

The IC₅₀ data of the exemplary examples are provided in Table 2, where A stands for IC₅₀ ≤ 0.1 µM; B stands for 0.1 µM < IC₅₀ ≤ 0.5 µM; and C stands for IC₅₀ > 0.5 µM.

**Table 2**

| **Example number** | **IC₅₀ (µM)** |
|---|---|
| 6 | 0.038 |
| 11 | B |
| 12 | A |
| 14 | 0.054 |
| 23 | 0.040 |
| 32 | B |
| 33 | 0.052 |
| 35 | 0.010 |
| 52 | 0.045 |
| 59 | B |
| 107 | A |

### In vivo PK

Compounds were formulated with 5% DMSO, 5% Solutol and 90% NaCl. SD rats and Balb/c mice were selected for drug administration. The intravenous dose was 1 mg/kg and the oral dose was 5 mg/kg. Blood was collected from the eye sockets at 5 min, 15 min, 30 min, 1 h, 2 h, 4 h, 7 h, and 24 h. After blood collection, centrifugation was performed at 4000 rpm for 10 min, and the supernatant was taken. 200 µL of internal standard solution was added to 30 µL of the supernatant for precipitation, and the mixture was vortexed and centrifuged at 12000 rpm for 10 min. 100 µL of the supernatant solution was taken and mixed with purified water in a 1:1 ratio for sample injection. The concentration of compounds in plasma was detected by a high performance liquid chromatography-mass spectrometer and the internal standard quantitative method was used to quantify the concentration of compounds in plasma samples. After measuring the compound concentration, relevant pharmacokinetic parameters including Cmax and AUC were calculated using Winnonln software. The experiment reveals that the exemplary compounds of the present invention have good in vivo PK properties.

## Claims

1. A compound as shown in formula (I), or a stereoisomer, tautomer, pharmaceutically acceptable salt, prodrug, chelate, non-covalent complex or solvate thereof, 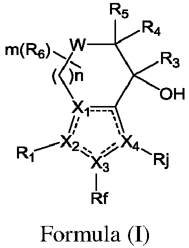
wherein -̅ -̅ -̅ -̅ -̅ -̅ represents a single bond or double bond;
X₁, X₂, X₃ and X₄ are each independently selected from C or N, and at least one of X₁, X₂, X₃ and X₄ is N; W is selected from C, N, O or S;
R₁ is selected from C₁-C₁₀ alkyl, C₂-C₁₀ alkenyl, C₂-C₁₀ alkynyl, C₁-C₁₀ alkoxy, C₆-C₁₀ aryl, 5- to 18-membered heteroaryl, C₅-C₁₀ cycloalkyl, or 5- to 10-membered heterocyclyl; wherein the 5- to 18-membered heteroaryl and 5- to 10-membered heterocyclyl contain 1, 2 or 3 heteroatoms each independently selected from N, O and S; the C₁-C₁₀ alkyl, C₂-C₁₀ alkenyl, C₂-C₁₀ alkynyl, C₁-C₁₀ alkoxy, C₆-C₁₀ aryl, 5- to 18-membered heteroaryl, C₅-C₁₀ cycloalkyl and 5- to 10-membered heterocyclyl can be optionally substituted with one or more substituents selected from H, halogen, -OH, -CN, oxo, amino, C₁-C₆ alkyl, C₁-C₆ alkoxy, C₂-C₆ alkenyl, C₃-C₅ cycloalkyl, C₂-C₆ alkynyl, C₁-C₆ haloalkyl, cyano-substituted C₁-C₆ alkyl, cyano-substituted C₁-C₆ haloalkyl, C₁-C₆ haloalkoxy, -C₁-C₆ alkylene-OR_{c}, -C₁-C₆ alkylene-C=O-R_{c}, -NO₂, -OR_{c}, -SR_{c}, -NRₐR_{b}, -C(=O)R_{c}, -C(=O)OR_{c}, -C(=O)NRₐR_{b}, -NC(=O)R_{c}, - S(=O)R_{c}, -S(=O)₂R_{c}, -S(=O)₂NRₐR_{b}, -S(=O)(=NRₐ)R_{b}, -P(=O)RₐR_{b} or -P(=S)RₐR_{b};
R₃ is selected from H, deuterium, halogen, -NO₂, -NRₐR_{b}, -CN, -OH, C₁-C₁₀ alkyl, C₂-C₁₀ alkenyl, C₁-C₅ haloalkyl, C₁-C₁₀ alkoxy, -O-C(=O)-C₁₋₃ alkyl, -C(=O)-O-C₁₋₃ alkyl or C₂-C₁₀ alkynyl, wherein the C₁-C₁₀ alkyl, C₂-C₁₀ alkenyl, C₁-C₅ haloalkyl, C₁-C₁₀ alkoxy, -O-C(=O)-C₁₋₃ alkyl, -C(=O)-O-C₁₋₃ alkyl, and C₂-C₁₀ alkynyl can be optionally substituted with one or more substituents selected from H, halogen, -CN, - OH, amino, C₁-C₅ alkyl, C₁-C₆ alkoxy, C₂-C₆ alkenyl or C₁-C₅ haloalkyl;
R₄ and R₅ are each independently selected from H, halogen, -NO₂, -OH, C₁-C₆ alkyl, C₁-C₆ alkoxy, C₁-C₆ haloalkyl, C₂-C₁₀ alkenyl, C₂-C₁₀ alkynyl, C₃-C₅ cycloalkyl and 3- to 6-membered heterocyclyl; the C₁-C₆ alkyl, C₁-C₆ alkoxy, C₁-C₆ haloalkyl, C₂-C₁₀ alkenyl, C₂-C₁₀ alkynyl, C₃-C₅ cycloalkyl and 3- to 6-membered heterocyclyl can be optionally substituted with one or more substituents selected from H, halogen, -CN, -OH, amino, oxo, C₁-C₅ alkyl, C₁-C₆ alkoxy, C₂-C₆ alkenyl or C₁-C₅ haloalkyl; or R₄ and R₅ together form oxo; or R₄ and R₅ together with the C atom to which they are attached form substituted or unsubstituted cyclopropyl;
R₆ is selected from H, -CN, halogen, -NO₂, -OH, -NO₂, -NRₐR_{b}, oxo, C₁-C₆ alkyl, C₁-C₆ alkoxy, C₁-C₆ haloalkyl, C₂-C₁₀ alkenyl, C₂-C₁₀ alkynyl, C₃-C₅ cycloalkyl and 3- to 6-membered heterocyclyl; the C₁-C₆ alkyl, C₁-C₆ alkoxy, C₁-C₆ haloalkyl, C₂-C₁₀ alkenyl, C₂-C₁₀ alkynyl, C₃-C₅ cycloalkyl and 3- to 6-membered heterocyclyl can be optionally substituted with one or more substituents selected from H, halogen, -CN, -OH, amino, oxo, C₁-C₅ alkyl, C₁-C₆ alkoxy, C₂-C₆ alkenyl or C₁-C₅ haloalkyl; or two R₆ together with the C atom to which they are attached form substituted or unsubstituted C₃-C₅ cycloalkyl or 3- to 5-membered heterocyclyl;
or R₆ and R₅ together with the C atom to which they are attached form substituted or unsubstituted C₃-C₄ cycloalkyl;
R_{f} is absent or selected from H, -CN, halogen, C₁-C₁₀ alkyl, C₁-C₁₀ haloalkyl, C₃-C₁₀ cycloalkyl, oxo, or - NRₐR_{b}; the C₁-C₁₀ alkyl, C₁-C₁₀ haloalkyl and C₃-C₁₀ cycloalkyl can be optionally substituted with one or more substituents selected from H, halogen, -CN, -OH, amino, C₁-C₅ alkyl, C₁-C₆ alkoxy, C₂-C₆ alkenyl or C₁-C₅ haloalkyl;
Rⱼ is selected from -C₁-C₆ alkylene-C(=O)R_{c}, -C₁-C₆ alkylene-C(=O)OR_{c}, -C₂F₅, -C(=O)R_{c}, -C(=O)OR_{c}, - SR_{c}, -S(=O)R_{c}, -S(=O)₂R_{c} or -S(=O)(=NRₐ)R_{c}, wherein the C₁-C₆ alkylene can be optionally substituted with one or more substituents selected from H, halogen, cyano, C₁-C₄ alkyl, C₂-C₄ alkenyl, C₂-C₄ alkynyl, C₁-C₄ haloalkyl, C₁-C₄ alkoxy and C₁-C₄ haloalkoxy;
Rₐ and R_{b} are each independently selected from H, CN, C₁-C₁₀ alkyl, C₁-C₁₀ haloalkyl, C₁-C₁₀ alkoxy, C₂-C₁₀ alkenyl, C₂-C₁₀ alkynyl, C₃-C₁₀ cycloalkyl, 3- to 10-membered heterocyclyl, C₆-C₁₀ aryl or 5- to 10-membered heteroaryl;
R_{c} is selected from C₁-C₁₀ alkyl, C₁-C₁₀ alkoxy, C₃-C₆ cycloalkyl and -NRₐR_{b}, wherein the C₁-C₁₀ alkyl, C₁-C₁₀ alkoxy and C₃-C₆ cycloalkyl can be optionally substituted with one or more substituents selected from H, halogen, CN, -NH₂, oxo, C₁-C₄ haloalkyl, C₁-C₄ haloalkoxy, and C₃-C₆ cycloalkyl;
n is 0 or 1;
m is 0, 1, 2, 3 or 4;
provided that the compound is not:
3-fluoro-5-(4-hydroxyl-3-(methylsulfonyl)-4,5,6,7-tetrahydro-1H-indol-1-yl)benzonitrile;
1-(3-chloro-5-fluorophenyl)-5,5-difluoro-3-(methylsulfonyl)-4,5,6,7-tetrahydro-1H-indol-4-ol;
3-(5,5-difluoro-4-hydroxyl-3-(methylsulfonyl)-4,5,6,7-tetrahydro-1H-indol-1-yl)-5-fluorobenzonitrile;
3-(3-((difluoromethyl)sulfonyl)-5,5-difluoro-4-hydroxyl-4,5,6,7-tetrahydro-1H-indol-1-yl)-5-fluorobenzonitrile;
1-(3,5-difluorophenyl)-5,5-difluoro-3-(methylsulfonyl)-4,5,6,7-tetrahydro-1H-indol-4-ol;
1-(3-chloro-5-fluorophenyl)-5,6-difluoro-3-(methylsulfonyl)-4,5,6,7-tetrahydro-1H-indol-4-ol;
(S)-5-(5,5-difluoro-4-hydroxyl-3-(methylsulfonyl)-4,5,6,7-tetrahydro-1H-indol-1-yl)-2-fluorobenzonitrile;
1-(3-chloro-5-fluorophenyl)-5,5-difluoro-3-((S)-methylsulfoxyl)-4,5,6,7-tetrahydro-1H-indol-4-ol;
1-(3-chloro-5-fluorophenyl)-5,5-difluoro-3-((R)-methylsulfoxyl)-4,5,6,7-tetrahydro-1H-indol-4-ol;
3-fluoro-5-(5-fluoro-4-hydroxyl-3-(methylsulfonyl)-4,5,6,7-tetrahydro-1H-indol-1-yl)benzonitrile;
(S)-1-(3,5-difluorophenyl)-5,5-difluoro-3-(methylsulfonyl)-2-(trifluoromethyl)-4,5,6,7-tetrahydro-1H-indol-4-ol;
2-chloro-5-(5,5-difluoro-4-hydroxyl-3-(methylsulfonyl)-5,6-dihydrocyclopenta[b]pyrrol-1(4H)-yl)benzonitrile; and
1-(3-chloro-5-fluorophenyl)-3-((difluoromethyl)sulfonyl)-5,5-difluoro-1,4,5,6-tetrahydrocyclopenta[b]pyrrol-4-ol.

2. The compound, or the stereoisomer, tautomer, pharmaceutically acceptable salt, prodrug, chelate, non-covalent complex or solvate thereof according to claim 1, wherein the compound is as shown in formula (II-1) or formula (II-2):

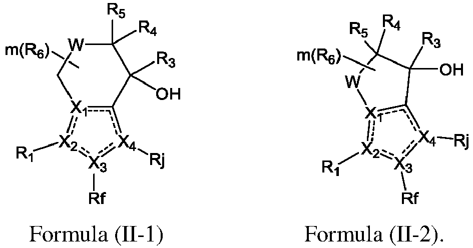

3. The compound, or the stereoisomer, tautomer, pharmaceutically acceptable salt, prodrug, chelate, non-covalent complex or solvate thereof according to claim 1, wherein the compound is as shown in formula (III-1), formula (III-2), formula (III-3) or formula (III-4)

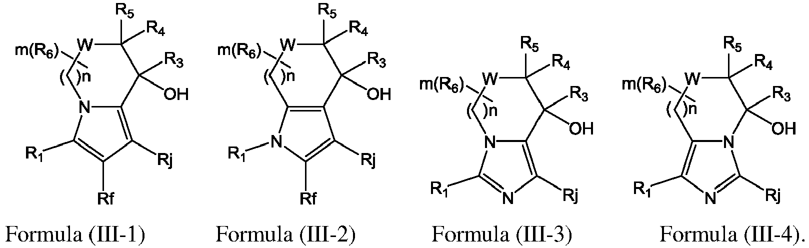

4. The compound, or the stereoisomer, tautomer, pharmaceutically acceptable salt, prodrug, chelate, non-covalent complex or solvate thereof according to any one of claims 1 to 3, wherein the R₃ is selected from H, deuterium, C₁-C₃ alkyl, or C₂-C₅ alkenyl, wherein the C₁-C₃ alkyl and C₂-C₅ alkenyl can be optionally substituted with one or more substituents selected from H, halogen, -CN, -OH, amino, or C₁-C₅ haloalkyl.

5. The compound, or the stereoisomer, tautomer, pharmaceutically acceptable salt, prodrug, chelate, non-covalent complex or solvate thereof according to any one of claims 1 to 4, wherein the R₃ is selected from H, deuterium or C₁-C₃ alkyl.

6. The compound, or the stereoisomer, tautomer, pharmaceutically acceptable salt, prodrug, chelate, non-covalent complex or solvate thereof according to any one of claims 1 to 5, wherein the R₃ is H or deuterium.

7. The compound, or the stereoisomer, tautomer, pharmaceutically acceptable salt, prodrug, chelate, non-covalent complex or solvate thereof according to any one of claims 1 to 6, wherein R₄ and R₅ are each independently selected from H, halogen or C₁-C₆ alkyl, wherein the C₁-C₆ alkyl can be optionally substituted with one or more substituents selected from H, halogen, -CN, -OH, amino or oxo.

8. The compound, or the stereoisomer, tautomer, pharmaceutically acceptable salt, prodrug, chelate, non-covalent complex or solvate thereof according to any one of claims 1 to 7, wherein R₄ and R₅ are each independently selected from H, halogen or C₁-C₆ alkyl.

9. The compound, or the stereoisomer, tautomer, pharmaceutically acceptable salt, prodrug, chelate, non-covalent complex or solvate thereof according to any one of claims 1 to 8, wherein R₄ and R₅ are each independently selected from H or halogen.

10. The compound, or the stereoisomer, tautomer, pharmaceutically acceptable salt, prodrug, chelate, non-covalent complex or solvate thereof according to any one of claims 1 to 9, wherein R₆ is H, -CN, halogen, C₁-C₆ alkyl, C₁-C₆ alkoxy or C₁-C₆ haloalkyl, wherein the C₁-C₆ alkyl, C₁-C₆ alkoxy or C₁-C₆ haloalkyl can be optionally substituted with one or more substituents selected from H, halogen, -CN, -OH, oxo, or amino.

11. The compound, or the stereoisomer, tautomer, pharmaceutically acceptable salt, prodrug, chelate, non-covalent complex or solvate thereof according to any one of claims 1 to 10, wherein R₆ is H, halogen or C₁-C₆ alkyl, wherein the C₁-C₆ alkyl can be optionally substituted with one or more substituents selected from H, halogen, -CN, -OH, oxo, or amino.

12. The compound, or the stereoisomer, tautomer, pharmaceutically acceptable salt, prodrug, chelate, non-covalent complex or solvate thereof according to any one of claims 1 to 11, wherein R₆ is H or halogen.

13. The compound, or the stereoisomer, tautomer, pharmaceutically acceptable salt, prodrug, chelate, non-covalent complex or solvate thereof according to any one of claims 1 to 12, wherein the R_{f} is selected from H, -CN, -NH₂, halogen, C₁-C₃ alkyl, cyclopropyl or C₁-C₃ haloalkyl, wherein the C₁-C₃ alkyl, cyclopropyl or C₁-C₃ haloalkyl can be optionally substituted with one or more substituents selected from H, halogen, -CN, -OH, amino, C₁-C₃ alkyl, C₂-C₄ alkenyl, and C₁-C₃ haloalkyl.

14. The compound, or the stereoisomer, tautomer, pharmaceutically acceptable salt, prodrug, chelate, non-covalent complex or solvate thereof according to any one of claims 1 to 13, wherein the R_{f} is selected from H, -CN, halogen, C₁-C₃ alkyl or C₁-C₃ haloalkyl.

15. The compound, or the stereoisomer, tautomer, pharmaceutically acceptable salt, prodrug, chelate, non-covalent complex or solvate thereof according to any one of claims 1 to 14, wherein the R_{f} is selected from H, -CN or halogen.

16. The compound, or the stereoisomer, tautomer, pharmaceutically acceptable salt, prodrug, chelate, non-covalent complex or solvate thereof according to any one of claims 1 to 15, wherein the W is C or O.

17. The compound, or the stereoisomer, tautomer, pharmaceutically acceptable salt, prodrug, chelate, non-covalent complex or solvate thereof according to any one of claims 1 to 16, wherein the R₁ is C₆-C₁₀ aryl, 5- to 10-membered heteroaryl, C₅-C₁₀ cycloalkyl or 5- to 10-membered heterocyclyl, wherein the 5- to 10-membered heteroaryl and 5- to 10-membered heterocyclyl contain 1, 2 or 3 heteroatoms each independently selected from N, O and S, and the C₆-C₁₀ aryl, 5- to 10-membered heteroaryl, C₅-C₁₀ cycloalkyl or 5- to 10-membered heterocyclyl can be optionally substituted with one or more substituents optionally selected from halogen, -OH, -CN, oxo, amino, C₁-C₆ alkyl, C₁-C₆ alkoxy, C₁-C₆ haloalkyl, cyano-substituted C₁-C₆ alkyl, cyano-substituted C₁-C₆ haloalkyl, -C₁-C₆ alkylene-OR_{c}, -C₁-C₆ alkylene-C=O-R_{c}, -NO₂, C(=O)OR_{c} or -S(=O)₂R_{c}.

18. The compound, or the stereoisomer, tautomer, pharmaceutically acceptable salt, prodrug, chelate, non-covalent complex or solvate thereof according to any one of claims 1 to 17, wherein the R₁ is C₆-C₁₀ aryl, 5- to 10-membered heteroaryl, C₅-C₁₀ cycloalkyl or 5- to 10-membered heterocyclyl, wherein the 5- to 10-membered heteroaryl and 5- to 10-membered heterocyclyl contain 1 or 2 N heteroatoms, and the C₆-C₁₀ aryl, 5- to 10-membered heteroaryl, C₅-C₁₀ cycloalkyl or 5- to 10-membered heterocyclyl can be optionally substituted with one or more substituents selected from halogen, hydroxyl, cyano, amino, C₁-C₆ alkyl, C₃₋₅ cycloalkyl, C₁-C₆ haloalkyl, cyano-substituted C₁-C₆ alkyl, cyano-substituted C₁-C₆ haloalkyl or C₁-C₆ haloalkoxy.

19. The compound, or the stereoisomer, tautomer, pharmaceutically acceptable salt, prodrug, chelate, non-covalent complex or solvate thereof according to any one of claims 1 to 18, wherein the R₁ is C₆-C₁₀ aryl, 5- to 10-membered heteroaryl, C₅-C₁₀ cycloalkyl or 5- to 10-membered heterocyclyl, wherein the 5- to 10-membered heteroaryl and 5- to 10-membered heterocyclyl contain 1 or 2 N heteroatoms, and the C₆-C₁₀ aryl, 5- to 10-membered heteroaryl, C₅-C₁₀ cycloalkyl or 5- to 10-membered heterocyclyl can be optionally substituted with one or more substituents selected from halogen, cyano, cyano-substituted C₁-C₆ haloalkyl, cyano-substituted C₁-C₆ alkyl or C₁-C₆ haloalkyl.

20. The compound, or the stereoisomer, tautomer, pharmaceutically acceptable salt, prodrug, chelate, non-covalent complex or solvate thereof according to any one of claims 1 to 19, wherein the R₁ is phenyl, pyridyl, naphthyl, 10-membered heteroaryl, C₈-C₁₀ cycloalkyl or 8- to 10-membered heterocyclyl, wherein the 10-membered heteroaryl and 8- to 10-membered heterocyclyl optionally contain 1 or 2 N heteroatoms, and the phenyl, pyridyl, naphthyl, 10-membered heteroaryl, C₈-C₁₀ cycloalkyl or 8- to 10-membered heterocyclyl can be optionally substituted with one or more substituents selected from halogen, cyano or C₁-C₆ haloalkyl.

21. The compound, or the stereoisomer, tautomer, pharmaceutically acceptable salt, prodrug, chelate, non-covalent complex or solvate thereof according to any one of claims 1 to 20, wherein the Rⱼ is selected from -C₁-C₆ alkylene-C(=O)R_{c}, -C₁-C₆ haloalkylene-C(=O)R_{c}, -C₁-C₆ haloalkylene-C(=O)OR_{c}, -C₂F₅, - C₁-C₆ cyano-substituted alkylene-C(=O)R_{c}, -C(=O)R_{c}, -C(=O)OR_{c}, -SR_{c}, -S(=O)R_{c}, -S(=O)₂R_{c} or - S(=O)(=NRₐ)R_{c}.

22. The compound, or the stereoisomer, tautomer, pharmaceutically acceptable salt, prodrug, chelate, non-covalent complex or solvate thereof according to any one of claims 1 to 21, wherein the Rⱼ is selected from -C₂F₅, -C₁-C₆ haloalkylene-C(=O)R_{c}, -C₁-C₆ haloalkylene-C(=O)OR_{c}, -C(=O)OR_{c}, -C(=O)R_{c}, -SR_{c}, -S(=O)₂R_{c} or -S(=O)(=NRₐ)R_{c}.

23. The compound, or the stereoisomer, tautomer, pharmaceutically acceptable salt, prodrug, chelate, non-covalent complex or solvate thereof according to any one of claims 1 to 22, wherein the R_{c} is selected from C₁-C₆ alkyl, C₁-C₆ alkoxy, C₃-C₆ cycloalkyl and -NH₂, wherein the C₁-C₆ alkyl, C₁-C₆ alkoxy and C₃-C₆ cycloalkyl can be optionally substituted with one or more substituents selected from H, halogen, CN, -NH₂, C₁-C₄ haloalkyl or C₃-C₆ cycloalkyl.

24. The compound, or the stereoisomer, tautomer, pharmaceutically acceptable salt, prodrug, chelate, non-covalent complex or solvate thereof according to any one of claims 1 to 23, wherein the R_{c} is selected from C₁-C₆ alkyl, wherein the C₁-C₆ alkyl can be optionally substituted with one or more substituents selected from H, halogen or CN.

25. The compound, or the stereoisomer, tautomer, pharmaceutically acceptable salt, prodrug, chelate, non-covalent complex or solvate thereof according to any one of claims 1 to 24, wherein the Rₐ is selected from H, CN or C₁-C₄ alkyl.

26. The compound, or the stereoisomer, tautomer, pharmaceutically acceptable salt, prodrug, chelate, non-covalent complex or solvate thereof according to any one of claims 1 to 25, wherein the R_{b} is selected from H, CN or C₁-C₄ alkyl.

27. The compound, or the stereoisomer, tautomer, pharmaceutically acceptable salt, prodrug, chelate, non-covalent complex or solvate thereof according to any one of claims 1 to 26, wherein the Rⱼ is selected from

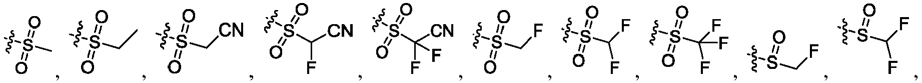

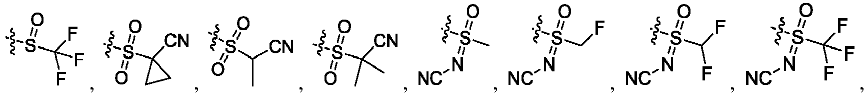

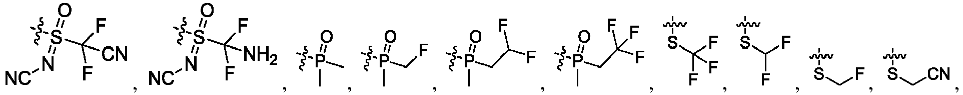

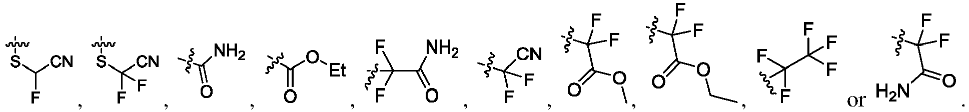

28. The compound, or the stereoisomer, tautomer, pharmaceutically acceptable salt, prodrug, chelate, non-covalent complex or solvate thereof according to any one of claims 1 to 27, wherein the compound is as shown in formula (IX-1) or formula (IX-2),

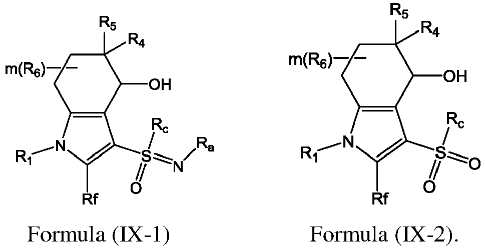

29. A compound, or a stereoisomer, tautomer, pharmaceutically acceptable salt, prodrug, chelate, non-covalent complex or solvate thereof, wherein the compound is selected from:
1) 5-(5,5-difluoro-4-hydroxyl-3-(trifluoromethyl)-4,5,6,7-tetrahydro-1*H*-indol-1-yl)-2,3-difluorobenzonitrile;
2) 5,5-difluoro-1-(4-fluoro-3-(fluoromethyl)phenyl)-3-(trifluoromethyl)-4,5,6,7-tetrahydro-1*H-*indol-4-ol;
3) (*S*)-4-(5,5-difluoro-4-hydroxyl-3-(trifluoromethyl)-4,5,6,7-tetrahydro-1*H*-indol-1-yl)-2-(difluoromethyl)benzonitrile;
4) (*S*)-5-(3-((difluoromethyl)sulfonyl)-5,5-difluoro-4-hydroxyl-4,5,6,7-tetrahydro-1*H*-indol-1-yl)-2-fluorobenzonitrile;
5) (*S*)-1-(3-(difluoromethyl)-4-fluorophenyl)-5,5-difluoro-3-(methylsulfonyl)-4,5,6,7-tetrahydro-1*H-*indol-4-ol;
6) (*S*)-1-(3-(difluoromethyl)-4-fluorophenyl)-3-((difluoromethyl)sulfonyl)-5,5-difluoro-4,5,6,7-tetrahydro-1*H*-indol-4-ol;
7) (*S*)-3-((*S*)-(difluoromethyl)sulfinyl)-5,5-difluoro-1-(4-fluoro-3-(trifluoromethyl)phenyl)-4,5,6,7-tetrahydro-1*H*-indol-4-ol;
8) (*S*)-3-((*R*)-(difluoromethyl)sulfinyl)-5,5-difluoro-1-(4-fluoro-3-(trifluoromethyl)phenyl)-4,5,6,7-tetrahydro-1*H*-indol-4-ol;
9) 5-(5,5-difluoro-4-hydroxyl-3-(perfluoroethyl)-4,5,6,7-tetrahydro-1*H*-indol-1-yl)-2-fluorobenzonitrile;
10) (*S*)-5-(5,5-difluoro-4-hydroxyl-3-((trifluoromethyl)sulfonyl)-4,5,6,7-tetrahydro-1*H*-indol-1-yl)-2-fluorobenzonitrile;
11) (*S*)-5-(5,5-difluoro-3-((fluoromethyl)sulfonyl)-4-hydroxyl-4,5,6,7-tetrahydro-1*H*-indol-1-yl)-2-fluorobenzonitrile;
12) (*S*)-1-(3-(difluoromethyl)-4-fluorophenyl)-5,5-difluoro-3-((trifluoromethyl)sulfonyl)-4,5,6,7-tetrahydro-1*H*-indol-4-ol;
13) (*S*)-2-((5,5-difluoro-1-(4-fluoro-3-(trifluoromethyl)phenyl)-4-hydroxyl-4,5,6,7-tetrahydro-1*H-*indol-3-yl)sulfonyl)acetonitrile;
14) (*S*)-1-(3-(difluoromethyl)-4-fluorophenyl)-5,5-difluoro-3-((fluoromethyl)sulfonyl)-4,5,6,7-tetrahydro-1*H*-indol-4-ol;
15) 2-(1-(3-cyano-4-fluorophenyl)-5,5-difluoro-4-hydroxyl-4,5,6,7-tetrahydro-1*H*-indol-3-yl)-2,2-difluoroacetamide;
16) (*S*)-3-(ethylsulfonyl)-5,5-difluoro-1-(4-fluoro-3-(trifluoromethyl)phenyl)-4,5,6,7-tetrahydro-1H-indol-4-ol;
17) 5-(3-(cyanodifluoromethyl)-5,5-difluoro-4-hydroxyl-4,5,6,7-tetrahydro-1*H*-indol-1-yl)-2-fluorobenzonitrile;
18) *N*-(((*S*)-1-(3-cyano-4-fluorophenyl)-5,5-difluoro-4-hydroxyl-4,5,6,7-tetrahydro-1*H*-indol-3-yl)(methyl)(oxo)-16-sulfino)cyanamide;
19) (S)-2-((1-(3-(difluoromethyl)-4-fluorophenyl)-5,5-difluoro-4-hydroxyl-4,5,6,7-tetrahydro-1*H-*indol-3-yl)sulfonyl)acetonitrile;
20) (*S*)-5,5-difluoro-1-(4-fluoro-3-(trifluoromethyl)phenyl)-3-(methylsulfonyl)-4,5,6,7-tetrahydro-1*H*-indol-4-ol;
21) (*S*)-3-((difluoromethyl)sulfonyl)-5,5-difluoro-1-(4-fluoro-3-(trifluoromethyl)phenyl)-4,5,6,7-tetrahydro-1*H*-indol-4-ol;
22) (*S*)-5-(3-((difluoromethyl)sulfonyl)-2,5,5-trifluoroether-4-hydroxyl-4,5,6,7-tetrahydro-1*H*-indol-1-yl)-2-fluorobenzonitrile;
23) (*S*)-5,5-difluoro-1-(4-fluoro-3-(trifluoromethyl)phenyl)-3-((trifluoromethyl)sulfonyl)-4,5,6,7-tetrahydro-1*H*-indol-4-ol;
24) 1-(3-chloro-4-fluorophenyl)-5,5-difluoro-3-(methylsulfonyl)-4,5,6,7-tetrahydro-1*H*-indol-4-ol;
25) 1-(3-chloro-4-fluorophenyl)-3-((difluoromethyl)sulfonyl)-5,5-difluoro-4,5,6,7-tetrahydro-1*H-*indol-4-ol;
26) (*R*)-1-(3-(difluoromethyl)-4-fluorophenyl)-3-((difluoromethyl)sulfonyl)-5,5-difluoro-4,5,6,7-tetrahydro-1*H*-indol-4-ol;
27) (*S*)-2-chloro-5-(5,5-difluoro-4-hydroxyl-3-(methylsulfonyl)-4,5,6,7-tetrahydro-1*H*-indol-1-yl)benzonitrile;
28) (*S*)-2-chloro-5-(3-((difluoromethyl)sulfonyl)-5,5-difluoro-4-hydroxyl-4,5,6,7-tetrahydro-1*H-*indol-1-yl)benzonitrile;
29) (*S*)-4-(5,5-difluoro-4-hydroxyl-3-(methylsulfonyl)-4,5,6,7-tetrahydro-1*H*-indol-1-yl)-2-(difluoromethyl)benzonitrile;
30) (*S*)-5,5-difluoro-1-(4-fluoro-3-(trifluoromethyl)phenyl)-3-((fluoromethyl)sulfonyl)-4,5,6,7-tetrahydro-1*H*-indol-4-ol;
31) (*S*)-4-(5,5-difluoro-4-hydroxyl-3-((trifluoromethyl)sulfonyl)-4,5,6,7-tetrahydro-1*H*-indol-1-yl)-2-(trifluoromethyl)benzonitrile;
32) (*R*)-1-(3-(difluoromethyl)-4-fluorophenyl)-3-((difluoromethyl)sulfonyl)-4,5,6,7-tetrahydro-1*H-*indol-4-ol;
33) (*S*)-1-(3-chloro-4-fluorophenyl)-5,5-difluoro-3-((trifluoromethyl)sulfonyl)-4,5,6,7-tetrahydro-1*H-*indol-4-ol;
34) (*S*)-4-(3-((difluoromethyl)sulfonyl)-5,5-difluoro-4-hydroxyl-4,5,6,7-tetrahydro-1*H*-indol-1-yl)-2-(trifluoromethyl)benzonitrile;
35) (*S*)-4-(5,5-difluoro-4-hydroxyl-3-((trifluoromethyl)sulfonyl)-4,5,6,7-tetrahydro-1*H*-indol-1-yl)-2-(difluoromethyl)benzonitrile;
36) (*S*)-4-(5,5-difluoro-3-((fluoromethyl)sulfonyl)-4-hydroxyl-4,5,6,7-tetrahydro-1*H*-indol-1-yl)-2-(trifluoromethyl)benzonitrile;
37) (*S*)-1-(3-chloro-4-fluorophenyl)-5,5-difluoro-3-((fluoromethyl)sulfonyl)-4,5,6,7-tetrahydro-1*H-*indol-4-ol;
38) (*S*)-4-(5,5-difluoro-4-hydroxyl-3-(methylsulfonyl)-4,5,6,7-tetrahydro-1H-indol-1-yl)phthalonitrile;
39) (*S*)-4-(3-((difluoromethyl)sulfonyl)-5,5-difluoro-4-hydroxyl-4,5,6,7-tetrahydro-1*H*-indol-1-yl)phthalonitrile;
40) (*S*)-2-chloro-5-(5,5-difluoro-4-hydroxyl-3-((trifluoromethyl)sulfonyl)-4,5,6,7-tetrahydro-1*H-*indol-1-yl)benzonitrile;
41) 4-(5,5-difluoro-4-hydroxyl-3-(perfluoroethyl)-4,5,6,7-tetrahydro-1*H*-indol-1-yl)-2-(difluoromethyl)benzonitrile;
42) (*S*)-4-(5,5-difluoro-4-hydroxyl-3-((trifluoromethyl)sulfonyl)-4,5,6,7-tetrahydro-1*H*-indol-1-yl)phthalonitrile;
43) (*S*)-2-(difluoromethyl)-4-(3-((difluoromethyl)sulfonyl)-5,5-difluoro-4-hydroxyl-4,5,6,7-tetrahydro-1*H*-indol-1-yl)benzonitrile;
44) (*S*)-4-(5,5-difluoro-3-((fluoromethyl)sulfonyl)-4-hydroxyl-4,5,6,7-tetrahydro-1*H*-indol-1-yl)-2-(difluoromethyl)benzonitrile;
45) (*S*)-4-(5,5-difluoro-3-((fluoromethyl)sulfonyl)-4-hydroxyl-4,5,6,7-tetrahydro-1*H*-indol-1-yl)phthalonitrile;
46) (*S*)-4-(5,5-difluoro-4-hydroxyl-3-(perfluoroethyl)-4,5,6,7-tetrahydro-1*H*-indol-1-yl)phthalonitrile;
47) 2-(((*S*)-1-(3-(difluoromethyl)-4-fluorophenyl)-5,5-difluoro-4-hydroxyl-4,5,6,7-tetrahydro-1*H-*indol-3-yl)sulfonyl)-2-fluoroacetonitrile;
48) ethyl (*S*)-2-(1-(3-(difluoromethyl)-4-fluorophenyl)-5,5-difluoro-4-hydroxyl-4,5,6,7-tetrahydro-1*H*-indol-3-yl)-2,2-difluoroacetate;
49) (*S*)-2-(1-(3-(difluoromethyl)-4-fluorophenyl)-5,5-difluoro-4-hydroxyl-4,5,6,7-tetrahydro-1*H-*indol-3-yl)-2,2-difluoroacetonitrile;
50) (*S*)-2-(1-(3-(difluoromethyl)-4-fluorophenyl)-5,5-difluoro-4-hydroxyl-4,5,6,7-tetrahydro-1*H-*indol-3-yl)-2,2-difluoroacetamide;
51) (*S*)-1-(3-(difluoromethyl)-4-fluorophenyl)-5,5-difluoro-3-(thiazol-2-yl)-4,5,6,7-tetrahydro-1*H-*indol-4-ol;
52) (*R*)-1-(3-(difluoromethyl)-4-fluorophenyl)-3-((trifluoromethyl)sulfonyl)-4,5,6,7-tetrahydro-1*H-*indol-4-ol;
53) (*S*)-1-(3-(difluoromethyl)-4-fluorophenyl)-5,5-difluoro-3-(oxazol-2-yl)-4,5,6,7-tetrahydro-1*H-*indol-4-ol;
54) (*S*)-2-((1-(3-(difluoromethyl)-4-fluorophenyl)-5,5-difluoro-4-hydroxyl-4,5,6,7-tetrahydro-1*H-*indol-3-yl)sulfonyl)-2,2-difluoroacetonitrile;
55) 2-(((*S*)-5,5-difluoro-1-(4-fluoro-3-(trifluoromethyl)phenyl)-4-hydroxyl-4,5,6,7-tetrahydro-1*H-*indol-3-yl)sulfonyl)-2-fluoroacetonitrile;
56) (*S*)-(1-(3-(difluoromethyl)-4-fluorophenyl)-5,5-difluoro-4-hydroxyl-4,5,6,7-tetrahydro-1*H*-indol-3-yl) dimethylphosphine oxide methyl;
57) 2-(((*S*)-1-(3-(difluoromethyl)-4-fluorophenyl)-5,5-difluoro-4-hydroxyl-4,5,6,7-tetrahydro-1*H-*indol-3-yl)sulfonyl) propanenitrile;
58) (*S*)-1-((1-(3-(difluoromethyl)-4-fluorophenyl)-5,5-difluoro-4-hydroxyl-4,5,6,7-tetrahydro-1*H-*indol-3-yl)sulfonyl) cyclopropane-1-nitrile;
59) *N*-(((*S*)-1-(3-(difluoromethyl)-4-fluorophenyl)-5,5-difluoro-4-hydroxyl-4,5,6,7-tetrahydro-1*H-*indol-3-yl)(methyl)(oxo)-16-sulfino)cyanamide;
60) ethyl (*S*)-1-(3-(difluoromethyl)-4-fluorophenyl)-5,5-difluoro-4-hydroxyl-4,5,6,7-tetrahydro-1*H-*indole-3-carboxylate;
61) (*R*)-1-(3-(difluoromethyl)-4-fluorophenyl)-5,5-difluoro-3-((fluoromethyl)sulfonyl)-4,5,6,7-tetrahydro-1*H*-indol-4-ol;
62) (*S*)-1-(3-(difluoromethyl)-4-fluorophenyl)-5,5-difluoro-4-hydroxyl-4,5,6,7-tetrahydro-1*H*-indole-3-carboxamide;
63) (4*S*,5*R*)-1-(3-(difluoromethyl)-4-fluorophenyl)-3-((difluoromethyl)sulfonyl)-5-fluoro-4,5,6,7-tetrahydro-1*H*-indol-4-ol;
64) (4*S*,5*S*)-1-(3-(difluoromethyl)-4-fluorophenyl)-3-((difluoromethyl)sulfonyl)-5-fluoro-4,5,6,7-tetrahydro-1*H*-indol-4-ol;
65) (*R*)-2-(difluoromethyl)-4-(4-hydroxyl-3-((trifluoromethyl)sulfonyl)-4,5,6,7-tetrahydro-1*H*-indol-1-yl)benzonitrile;
66) (*S*)-4-(5,5-difluoro-4-hydroxyl-3-(trifluoromethyl)-4,5,6,7-tetrahydro-1*H*-indol-1-yl)-3-fluorobenzonitrile;
67) (4*S*)-1-(3-(difluoromethyl)-4-fluorophenyl)-5,5-difluoro-3-((fluoromethyl)sulfinyl)-4,5,6,7-tetrahydro-1*H*-indol-4-ol;
68) (*S*)-1-((R)-6,8-difluoro-1,2,3,4-tetrahydronaphthalen-1-yl-yl)-5,5-difluoro-3-(trifluoromethyl)-4,5,6,7-tetrahydro-1*H*-indol-4-ol;
69) (*S*)-1-((*S*)-6,8-difluoro-1,2,3,4-tetrahydronaphthalen-1-yl-yl)-5,5-difluoro-3-(trifluoromethyl)-4,5,6,7-tetrahydro-1*H*-indol-4-ol;
70) (*S*)-1-(2,4-difluorobenzyl)-5,5-difluoro-3-(trifluoromethyl)-4,5,6,7-tetrahydro-1*H*-indol-4-ol;
71) (*S*)-2-((5,5-difluoro-4-hydroxyl-3-(trifluoromethyl)-4,5,6,7-tetrahydro-1*H*-indol-1-yl)methyl)-5-fluorobenzonitrile;
72) (*S*)-1-(2,4-difluorophenyl)-5,5-difluoro-3-(trifluoromethyl)-4,5,6,7-tetrahydro-1*H*-indol-4-ol;
73) *N*-(((S)-1-(3-(difluoromethyl)-4-fluorophenyl)-5,5-difluoro-4-hydroxyl-4,5,6,7-tetrahydro-1*H-*indol-3-yl)(oxy)(trifluoromethyl)-16-sulfino)cyanamide;
74) *N*-((difluoromethyl)((*S*)-1-(3-(difluoromethyl)-4-fluorophenyl)-5,5-difluoro-4-hydroxyl-4,5,6,7-tetrahydro-1*H*-indol-3-yl)(oxo)-16-sulfino)cyanamide;
75) (*S*)-2-(5-(5,5-difluoro-4-hydroxyl-3-(trifluoromethyl)-4,5,6,7-tetrahydro-1*H*-indol-1-yl)-2-fluorophenyl) acetonitrile;
76) (*S*)-4-(5,5-difluoro-4-hydroxyl-3-(trifluoromethyl)-4,5,6,7-tetrahydro-1*H*-indol-1-yl)-2-(fluoromethyl)benzonitrile;
77) (*S*)-1-(3-(difluoromethyl)-4-fluorophenyl)-5,5-difluoro-3-((fluoromethyl)sulfonyl)-1,4,5,6-tetrahydrocyclopentadiene[*b*]pyrrol-4-ol;
78) (*S*)-1-(3-(difluoromethyl)-4-fluorophenyl)-5,5-difluoro-3-(methylsulfonyl)-1,4,5,6-tetrahydrocyclopentadiene[*b*] pyrrol-4-ol;
79) (*S*)-1-(3-(difluoromethyl)-4-fluorophenyl)-3-((difluoromethyl)sulfonyl)-5,5-difluoro-1,4,5,6-tetrahydrocyclopentadiene[*b*] pyrrol-4-ol;
80) (*S*)-1-(3-(difluoromethyl)-4-fluorophenyl)-5,5-difluoro-3-((trifluoromethyl)sulfonyl)-1,4,5,6-tetrahydrocyclopentadiene[*b*]pyrrol-4-ol;
81) (*S*)-5-(5,5-difluoro-4-hydroxyl-3-((trifluoromethyl)sulfonyl)-5,6-dihydrocyclopentadiene[b]pyrrol-1(4*H*)-yl)-yl2-fluorobenzonitrile;
82) (*S*)-3-(3-(difluoromethyl)-4-fluorophenyl)-7,7-difluoro-1-((trifluoromethyl)sulfonyl)-5,6,7,8-tetrahydroindol-8-ol;
83) (*S*)-3-(3-(difluoromethyl)-4-fluorophenyl)-7,7-difluoro-1-((trifluoromethyl)sulfonyl)-5,6,7,8-tetrahydroimidazo[1,5-*a*]pyridin-8-ol;
84) (7*R*,8*S*)-3-(3-(difluoromethyl)-4-fluorophenyl)-7-fluoro-1-((trifluoromethyl)sulfonyl)-5,6,7,8-tetrahydroindolizin-8-ol;
85) 1-(3-(difluoromethyl)-4-fluorophenyl)-5,5-difluoro-3-((trifluoromethyl)sulfonyl)-1,4,5,7-tetrahydropyran[3,4-*b*]pyrrol-4-ol;
86) 2-(difluoromethyl)-4-((4*S*,5*R*)-3-((difluoromethyl)sulfonyl)-5-fluoro-4-hydroxyl-4,5,6,7-tetrahydro-1*H*-indol-1-yl)benzonitrile;
87) 2-(difluoromethyl)-4-((4*S*,5*R*)-5-fluoro-4-hydroxyl-3-((trifluoromethyl)sulfonyl)-4,5,6,7-tetrahydro-1*H*-indol-1-yl)benzonitrile;
88) (*S*)-5,5-difluoro-1-(imidazo[1,2-a]pyridin-8-yl)-3-(trifluoromethyl)-4,5,6,7-tetrahydro-1*H*-indol-4-ol;
89) (4*S*,5*R*)-1-(3-(difluoromethyl)-4-fluorophenyl)-5-fluoro-3-((trifluoromethyl)sulfonyl)-4,5,6,7-tetrahydro-1*H*-indol-4-ol;
90) (*S*)-8-(5,5-difluoro-3-((fluoromethyl)sulfonyl)-4-hydroxyl-4,5,6,7-tetrahydro-1*H*-indol-1-yl)- 5-fluoro-1-naphthonitrile;
91) (*S*)-3-((difluoromethyl)sulfonyl)-5,5-difluoro-1-(4-fluoro-5,6,7,8-tetrahydronaphthalen-1-yl)-4,5,6,7-tetrahydro-1*H*-indol-4-ol;
92) (*S*)-5,5-difluoro-1-(quinolin-8-yl)-3-((trifluoromethyl)sulfonyl)-4,5,6,7-tetrahydro-1*H*-indol-4-ol;
93) (4*S*)-1-(6,8-difluoro-1,2,3,4-tetrahydronaphthalen-1-yl)-5,5-difluoro-3-(trifluoromethyl)-4,5,6,7-tetrahydro-1*H*-indol-4-ol;
94) (4*S*)-1-(6,8-difluoro-1,2,3,4-tetrahydronaphthalen-1-yl)-5,5-difluoro-3-((trifluoromethyl)sulfonyl)-4,5,6,7-tetrahydro-1*H*-indol-4-ol;
95) (4*S*)-1-(6,8-difluoro-1,2,3,4-tetrahydronaphthalen-1-yl)-3-((difluoromethyl)sulfonyl)-5,5-difluoro-4,5,6,7-tetrahydro-1*H*-indol-4-ol;
96) 1-(6,8-difluoro-1,2,3,4-tetrahydronaphthalen-1-yl)-5,5-difluoro-3-((fluoromethyl)sulfonyl)-4,5,6,7-tetrahydro-1*H*-indol-4-ol;
97) (4*S*)-1-(5,7difluoro-2,3-dihydro-1H-inden-1-yl)-5,5-difluoro-3-(trifluoromethyl)-4,5,6,7-tetrahydro-1*H*-indol-4-ol;
98) (4*S*)-5,5-difluoro-1-(5,6,7,8-tetrahydroquinolin-8-yl)-3-(trifluoromethyl)- 4,5,6,7-tetrahydro-1*H-*indol-4-ol;
99) (4*S*)-5,5-difluoro-3-(methylsulfonyl)-1-(5,6,7,8-tetrahydroquinolin-8-yl)- 4,5,6,7-tetrahydro-1*H-*indol-4-ol;
100) 5,5-difluoro-3-((fluoromethyl)sulfonyl)-1-(5,6,7,8-tetrahydroquinolin-8-yl)- 4,5,6,7-tetrahydro-1*H*-indol-4-ol;
101) (4*S*)-3-((difluoromethyl)sulfonyl)-5,5-difluoro-1-(5,6,7,8-tetrahydroquinolin-8-yl)- 4,5,6,7-tetrahydro-1*H*-indol-4-ol;
102) (4*S*)-5,5-difluoro-1-(5,6,7,8-tetrahydroquinolin-8-yl)-3-((trifluoromethyl)sulfonyl)- 4,5,6,7-tetrahydro-1*H*-indol-4-ol;
103) 2-(difluoromethyl)-4-(5-fluoro-4-hydroxyl-3-((trifluoromethyl)thio)- 4,5,6,7-tetrahydro-1*H*-indol-1-yl)benzonitrile;
104) 2-(difluoromethyl)-4-(5-fluoro-4-hydroxyl-3-((trifluoromethyl)sulfonyl)- 4,5,6,7-tetrahydro-1*H-*indol-1 -yl)benzonitrile;
105) 2-fluoro-5-(5-fluoro-4-hydroxyl-3-(trifluoromethyl)-4,7-dihydropyran[3,4-*b*]pyrrol-1(5*H*)-yl)benzonitrile;
106) 2-fluoro-5-(2,5,5-trifluoro-4-hydroxyl-3-(thien-2-yl)-4,5,6,7-tetrahydro-1*H*-indol-1-yl)benzonitrile; or
107) 2-(difluoromethyl)-4-(5-fluoro-4-hydroxyl-3-(trifluoromethyl)-4,5,6,7-tetrahydro-1*H*-indol-1-yl)benzonitrile.

30. A pharmaceutical composition, comprising a therapeutically effective amount of at least one of the compounds according to any one of claims 1 to 29 and at least one pharmaceutically acceptable excipient.

31. Use of the compound according to any one of claims 1 to 29 or the pharmaceutical composition according to claim 30 in the preparation of a medicament for treating a HIF-2α-mediated disease.

32. The use according to claim 31, wherein the disease is VHL syndrome, an autoimmune disease, an inflammatory disease and/or cancer.

33. The use according to claim 32, wherein the cancer is selected from bone cancer, pancreatic cancer, skin cancer, head and neck cancer, malignant melanoma, uterine cancer, ovarian cancer, rectal cancer, gastric cancer, cervical cancer, endometrial cancer, lymphoma, esophageal cancer, colon cancer, thyroid cancer, prostate cancer, sarcoma, leukemia, bladder cancer, kidney cancer, glioma, epidermal cystadenoma, squamous cell carcinoma, pheochromocytoma, lung cancer, liver cancer, breast cancer, exothelioma, neurocytoma, paraganglioma, blastoma, endocrine tumor, meningioma and medulloblastoma.

34. A method for treating and/or preventing a HIF-2α-mediated disease, comprising administering a therapeutically effective amount of the compound according to any one of claims 1 to 29 or the pharmaceutical composition according to claim 30 to a subject to be treated.

35. The method according to claim 34, wherein the HIF-2α-mediated disease is VHL syndrome, an autoimmune disease, an inflammatory disease and/or cancer.

36. The method according to claim 35, wherein the cancer is selected from bone cancer, pancreatic cancer, skin cancer, head and neck cancer, malignant melanoma, ovarian cancer, rectal cancer, gastric cancer, uterine cancer, cervical cancer, endometrial cancer, lymphoma, esophageal cancer, colon cancer, thyroid cancer, prostate cancer, sarcoma, leukemia, bladder cancer, kidney cancer, glioma, epidermal cystadenoma, squamous cell carcinoma, pheochromocytoma, lung cancer, liver cancer, breast cancer, exothelioma, neurocytoma, paraganglioma, blastoma, endocrine tumor, meningioma and medulloblastoma.

37. A method for preparing the compound according to claim 28, comprising subjecting an intermediate compound as shown in formula (A-1) or a compound as shown in formula (A-2) to an oxidation reaction to form a compound as shown in formula (IX-1) or formula (IX-2), 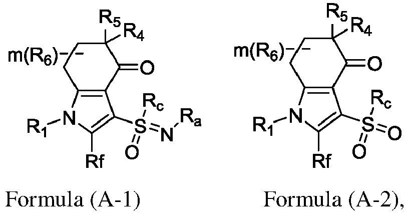
wherein R₁, R₄, R₅, R₆, R_{f}, R_{c}, Rₐ and m are as defined in any one of claims 1 to 27;
further preferably, comprising preparing the compound as shown in formula (A-1) by the method of scheme 1 or scheme 2, 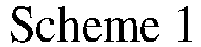 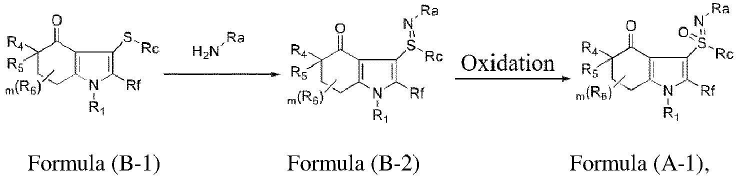
wherein R₁, R₄, R₅, R₆, R_{f}, R_{c}, Rₐ and m are as defined in any one of claims 1 to 27; 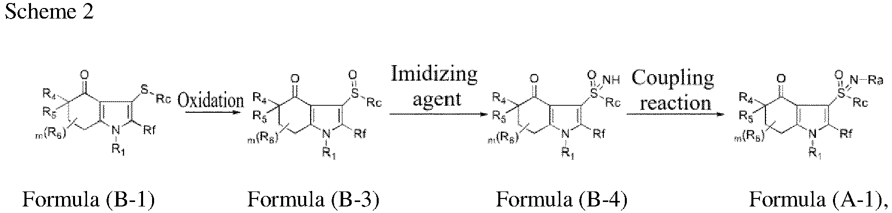
wherein R₁, R₄, R₅, R₆, R_{f}, R_{c}, Rₐ and m are as defined in any one of claims 1 to 27;
further preferably, comprising preparing the compound as shown in formula (A-2) by the method of scheme 3, scheme 4, scheme 5 or scheme 6, 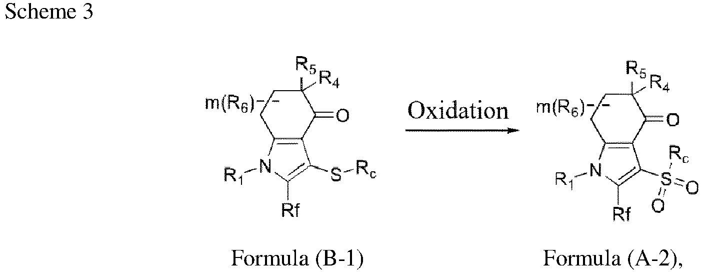
wherein R₁, R₄, R₅, R₆, R_{f}, R_{c} and m are as defined in any one of claims 1 to 27; 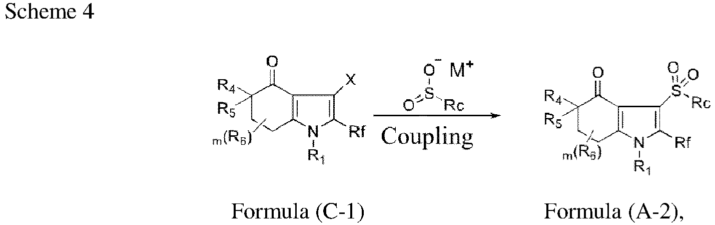
wherein R₁, R₄, R₅, R₆, R_{f} and m are as defined in any one of claims 1 to 27, and X is halogen; 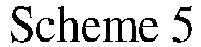 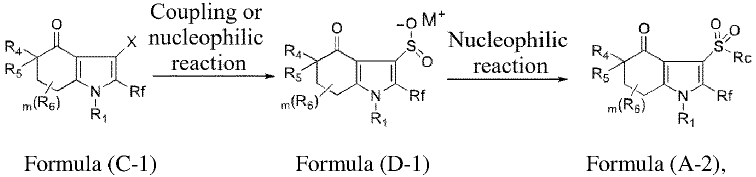
wherein R₁, R₄, R₅, R₆, R_{f}, R_{c} and m are as defined in any one of claims 1 to 27, and X is halogen; 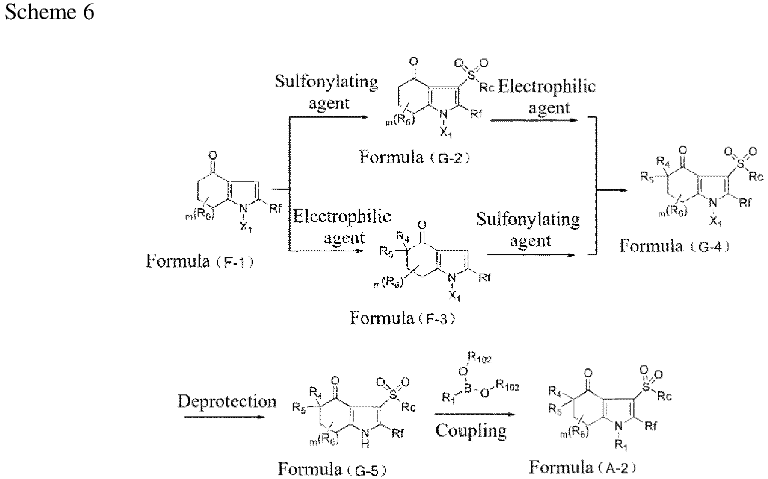
wherein R₁, R₄, R₅, R₆, R_{f}, R_{c} and m are as defined in any one of claims 1 to 27, X₁ is -TIPS, -Trt or - TBDPS, and R₁₀₂ is H or C₁₋₃ alkyl, or two R₁₀₂ together with the O atoms to which they are attached form a 5-membered heterocycle containing two oxygen atoms and one boron atom, and the 5-membered heterocycle can be optionally substituted with one or more C₁₋₃ alkyl;
even further preferably, comprising preparing the compound as shown in formula (C-1) by the method of scheme 7, 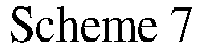 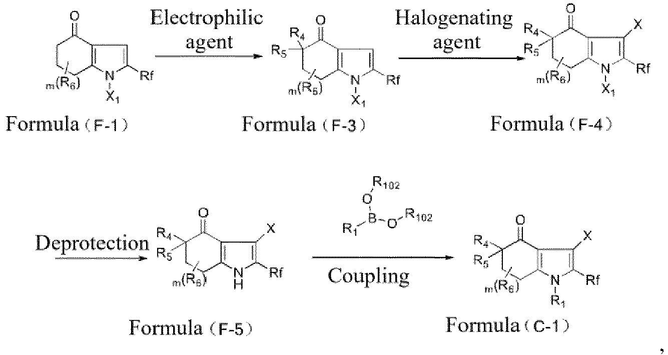
wherein R₁, R₄, R₅, R₆, R_{f} and m are as defined in any one of claims 1 to 27, X is halogen, X₁ is -TIPS, - Trt or -TBDPS, and R₁₀₂ is H or C₁₋₃ alkyl, or two R₁₀₂ together with the O atoms to which they are attached form a 5-membered heterocycle containing two oxygen atoms and one boron atom, and the 5-membered heterocycle can be optionally substituted with one or more C₁₋₃ alkyl;
even further preferably, comprising preparing the compound as shown in formula (B-1) by the method of scheme 8, scheme 9 or scheme 10, 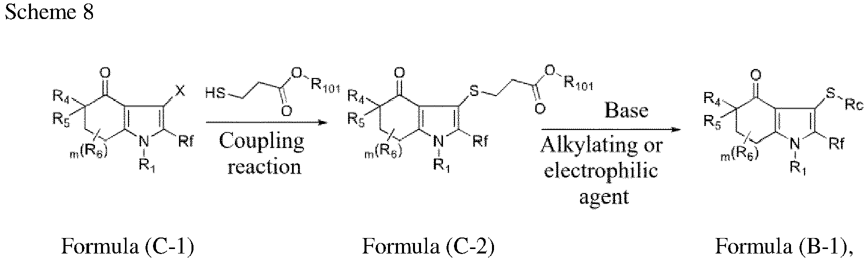
wherein R₁, R₄, R₅, R₆, R_{f}, R_{c} and m are as defined in any one of claims 1 to 27, X is halogen, and R₁₀₁ is C₁₋₁₀ alkyl; 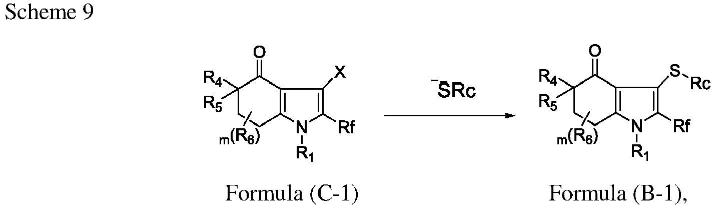
wherein R₁, R₄, R₅, R₆, R_{f}, R_{c} and m are as defined in any one of claims 1 to 27, and X is halogen; 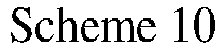 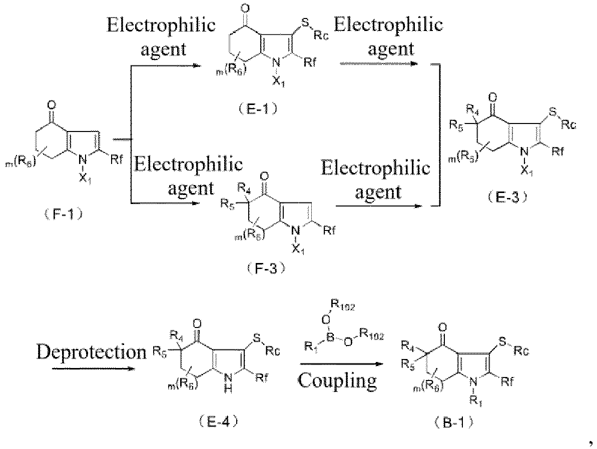
wherein R₁, R₄, R₅, R₆, R_{f}, R_{c} and m are as defined in any one of claims 1 to 27, and R₁₀₂ is H or C₁₋₃ alkyl, or two R₁₀₂ together with the O atoms to which they are attached form a 5-membered heterocycle containing two oxygen atoms and one boron atom, and the 5-membered heterocycle can be optionally substituted with one or more C₁₋₃ alkyl;
even further preferably, comprising preparing a compound as shown in formula (G-5) by the method of scheme 11, 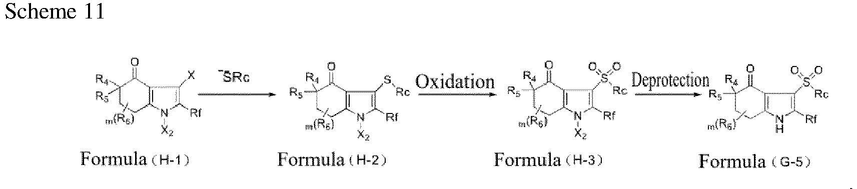
wherein R₄, R₅, R₆, R_{f}, R_{c} and m are as defined in any one of claims 1 to 27, X is halogen, and X₂ is -Boc or benzenesulfonyl.

38. A compound, or a stereoisomer, tautomer, pharmaceutically acceptable salt, prodrug, chelate, non-covalent complex or solvate thereof, wherein the compound is selected from the following structure: 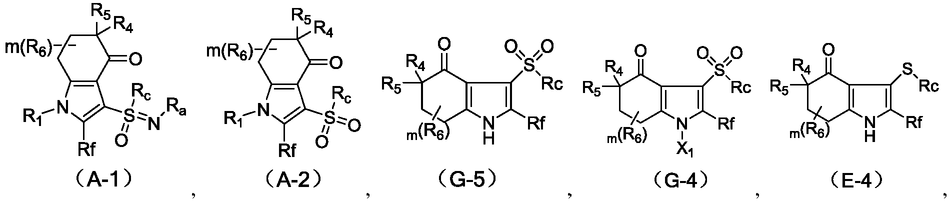 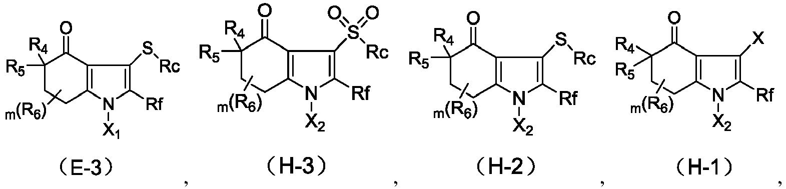 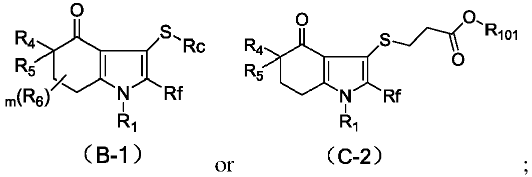 wherein R₁, R₄, R₅, R₆, R_{f}, R_{c}, Rₐ and m are as defined in any one of claims 1 to 27, X is halogen, X₁ is - TIPS, -Trt or -TBDPS, X₂ is -Boc or benzenesulfonyl, R₁₀₁ is C₁₋₁₀ alkyl, and R₁₀₂ is H or C₁₋₃ alkyl, or two R₁₀₂ together with the O atoms to which they are attached form a 5-membered heterocycle containing two oxygen atoms and one boron atom, and the 5-membered heterocycle can be optionally substituted with one or more C₁₋₃ alkyl.
